# EUROPEAN PATENT APPLICATION

(11) **EP 1 541 139 A1**
(43) Date of publication of application: **15.06.2005**
(21) Application number: 03736274.6
(22) Date of filing: 26.06.2003
(51) Int. Cl.: A61K 31/167, A61K 45/00, A61K 31/336, A61P 9/10, A61P 35/00, A61P 35/04, A61P 43/00

(54) **DRUG COMPOSITION CONTAINING NF-KAPPA B INHIBITOR**

(30) Priority: 26.06.2002 JP 2002185866; 14.02.2003 JP 2003037167
(71) Applicant: Signal Creation Inc., Yokohama-shi, Kanagawa 223-0061 (JP)
(72) Inventor: UMEZAWA, Kazuo, Keio Uni. Fac. of Science & Tech., Yokohama-shi, Kanagawa 223-8522 (JP); KAWAI, Yohko, Keio University School of Medicine, Tokyo 160-8582 (JP); HORIE, Ryouichi, Setagaya-ku, Tokyo 157-0066 (JP); WATANABE, Toshiki, Yokohama-shi, Kanagawa 227-0043 (JP); TOI, Masakazu, Tokyo Metropolitan Komagome Hosp., Tokyo 113-8677 (JP); MATSUMOTO, Gaku, Tokyo Metropolitan Komagome Hosp., Tokyo 113-8677 (JP); HORIGUCHI, Yutaka, Keio Uni. School of Medicine, Tokyo 160-8582 (JP); NAKASHIMA, Jun, Keio University School of Medicine, Tokyo 160-8582 (JP)
(74) Representative: Horner, Martin Grenville
(86) International application number: PCT/JP2003/008134
(87) International publication number: WO 2004/002465

(57) **Abstract**

Pharmaceutical compositions for improving at least one symptom resulting from tumor cells, which contains a compound represented by the following general formula (1) or a pharmacologically acceptable salt thereof as an active ingredient. 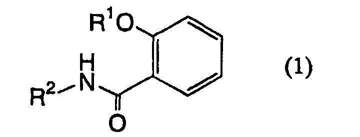 wherein R¹ represents a hydrogen atom or a C2-4 alkanoyl group and R² represents a group represented by the following formulae (A), (B), (C), (D), (E), (F) or (G): 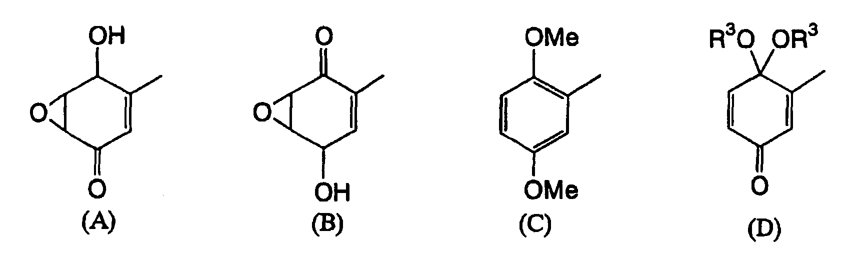 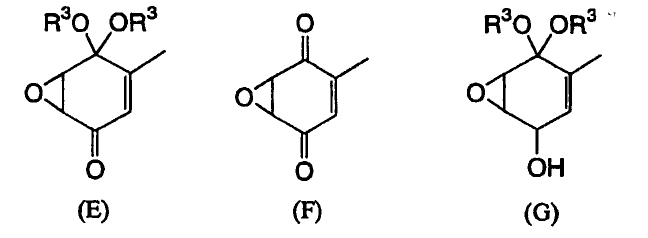 wherein R³ represents a C1-4 alkyl group.

## Description

### CROSS REFERENCE TO RELATED APPLICATIONS

This application claims the benefit of priority to Japan Patent Application No. 2002-185866, filed on June 26, 2002, and Japan Patent Application No. 2003-37167, filed on February 14, 2003, which are incorporated herein by reference.

### TECHNICAL FIELD

The present invention relates to pharmaceutical compositions, tumor cell proliferation inhibitors, adhesion molecule expression inhibitors, apoptosis inducers, preventive and therapeutic agents for arteriosclerosis or cancer, and therapeutic agents for cachexia, together with therapeutic methods for such diseases.

### BACKGROUND ART

In recent years, constant activation of NF-κB in various tumors has been reported one after another. NF-κB is often activated, for example, in tumors such as bladder cancer (Hum. Gene Ther. 10: 37-47, 1999), breast cancer (Cancer Res. 9: 3810-3818, 2001), and melanoma (Cancer Res. 61: 4901-4949, 2001). It is considered that such activation of NF-κB is likely to inactivate the function of inducing apoptosis and to promote tumor advancement. This is also suggested by the fact that NF-κB is inhibited by highly expressing IκB, thereby inducing cell death specific to neoplastic cells having high NF-κB activity (Hum. Gene Ther. 10: 37-47, 1999).

Cachexia is a disease that exhibits systemic defect with cardinal symptoms of anorexia, progressive loss of body weight, anemia, dry skin, edema, etc. in chronic diseases such as malignant tumors, tuberculosis, diabetes, hemopathy, and disorders of metabolism and internal secretion. Cachexia is often seen especially in terminally ill patients of malignant tumors etc. Patients with cachexia show loss of body weight, anemia, and other symptoms of deteriorations in systemic functions. Development of cachexia in cancer patients causes a high risk of complications and poor responses to chemotherapy. Moreover, weakness in the whole body produces strong side effects by chemotherapy or radiotherapy of cancer; cachexia can lead to death.

The detailed mechanism by which cachexia develops has not completely been elucidated yet. Only recently, however, clues to the mechanism have been emerging, including the involvement of cytokines such as interleukin-6 (IL-6) and tumor necrosis factor-α(TNF-α) (Saishin Igaku Vol. 54, No.10, 1999, 2502-2507). For example, it is considered that the expression mechanism of various symptoms in cancer cachexia is the action of cytokines overexpressed due to induction of expression by cachexia on the central nervous system, resulting in symptoms such as decreased food intake, fever, low blood pressure, and the state of inertia, also leading to the enhancement state of sugar, protein, and lipid catabolism.

Administration of steroid is effective in suppressing such symptoms in cachexia. When steroid is administered to cachexia patients, the suppressive effect on immunological reaction, the resulting antiinflammatory effect, and, further, the suppressive effect on the production of cachexia-inducing cytokines are exerted by steroid, whereby metabolic errors of cancer are corrected. As a result, cachexia symptoms such as loss of body weight, anorexia, inertia, dysgeusia, and anemia are alleviated and/or improved. However, long-term intake of steroid causes a problem of serious side effects. Since steroid is a hormone intrinsically present in the individual bodies, steroid taken in exhibits an similar effect to that of an excess hormone, sometimes causing edema or high blood pressure as side effects by the involvement in the reabsorption of salt in the kidney.

Meanwhile, omega-3 unsaturated fatty acid suppresses the production of inflammatory cytokines such as IL-6 and influences the synthesis of acute phase reaction proteins. By taking advantage of these mechanisms, administration of eicosapentaenoic acid (EPA) has produced a certain effect in improving cachexia. However, since the action of such a nutrition formula is indirect, it is difficult to expect a marked and reliable effect from that.

Therefore, there has been a growing demand for development of cachexia-specific drugs having a marked effect on cachexia, which is different from steroids having extensive effects. Under such a request, for example, having the inhibitory effect on TNF-α, thalidomide has been expected to improve the cachexia symptoms, and has been used as a therapeutic agent for cancer cachexia. However, TNF-α has another action--angiogenesis--in the living body; administration of thalidomide causes the side effect of inhibiting angiogenesis as well. Thus, a drug with comparatively high specificity inevitably produces a side effect. Considering uses under various situations, development of a wide variety of drugs having different mechanisms of action has been desired.

Meanwhile, arteriosclerosis is treated using mevalotin having an indirect therapeutic effect in such a way as to decreases cholesterol, but its effect is insufficient. No drugs have found clinical use as cancer cell metastasis inhibitors. Currently, a metal protease inhibitor etc. is under development, but it does not seem promising. It is well known that popular anticancer agents have strong side effects and their use is severely limited.

Accordingly, an object of the present invention is to provide pharmaceutical compositions capable of improving symptoms accompanied by activation of NF-κB.

### DISCLOSURE OF THE INVENTION

NF-κB is a transcription factor that functions in nuclei, but in the presence of IκB, the endogenous repressor thereof, they form a complex to be present as an inactive form in the cytoplasm. When the cell is stimulated by TNF-α etc., degradation of IκB is induced and NF-κB is activated. Activated NF-κB enters the nucleus and binds to the NF-κB binding site on DNA, where it regulates expression of genes encoding cytokines involved in immunological reactions or inflammatory reactions (e.g., IL-1, IL-2, IL-8, TNF-α, etc.) and cell adhesion molecules (e.g., ICAM-1, VCAM-1, etc.) (Ghoshi, S., et al., Annu.Rev.Immunol. 16:225-260 (1998)). It is thus thought that one of the intracellular target molecules for expression of TNF-α functions is NF-κB.

In recent years, the compounds represented by the following general formula (1) has been developed as substances having the inhibitory effect on activation of NF-κB(WO 01/12588; Matsumoto et al., Bioorg.Med.Chem.Lett. 10, 865 (2000)). 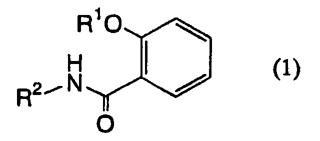 wherein R¹ represents a hydrogen atom or a C2-4 alkanoyl group and R² represents a group represented by the following formulae (A), (B), (C), (D), (E), (F) or (G): 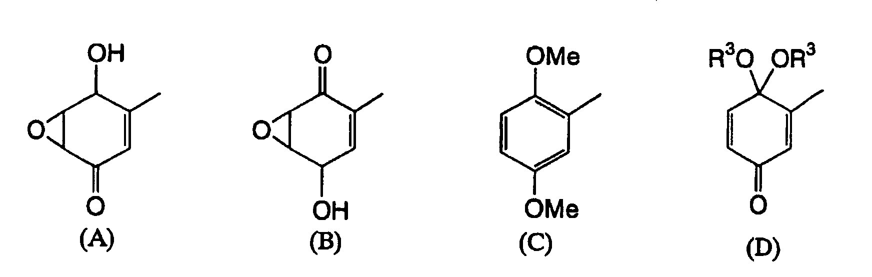 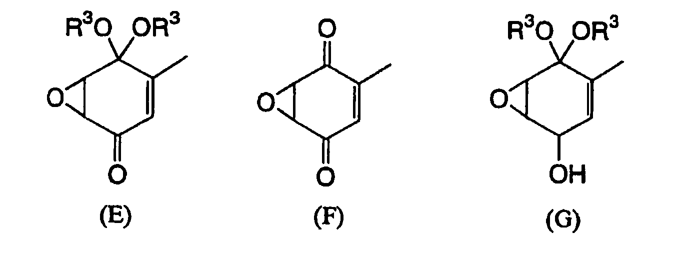 wherein R³ represents a C1-4 alkyl group.

Since both arteriosclerosis and cancer cell metastasis require expression of adhesion molecules in vascular endothelial cells, the inventors solved the problem by using the drugs that do not cause adhesion molecules to be expressed. The inventors thought that, similarly, supposing IL-6 or TNF-α are involved in the mechanism of cachexia development, inhibition of the functions of NF-κ B, an intracellular target molecule thereof, might be effective in prevention/improvement of the cachexia-associated symptoms.

Thus, the above-mentioned compounds were administered to model mice in which cachexia symptoms had been induced and the symptoms were observed. It was found that the compounds are useful in prevention/improvement of the cachexia symptoms, and, accordingly, the present invention has been accomplished. "Symptoms" as used herein refers to a wide spectrum of phenomena that occur accompanying the fact of having suffered from a disease; they do not necessarily refer only to apparent anomalies that a patient pointed out.

Thus, the pharmaceutical composition according to the present invention contains a compound for improving at least one symptom resulting from tumor cells represented by the following general formula (1) or a pharmacologically acceptable salt thereof as an active ingredient. 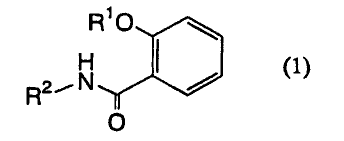 wherein R¹ represents a hydrogen atom or a C2-4 alkanoyl group. An alkanoyl group includes, acetyl, propionyl, and butanoyl groups, together with isomer groups thereof, and particularly preferred among these is an acetyl group.

R² is a group represented by the following formulae (A), (B), (C), (D), (E), (F), or (G). 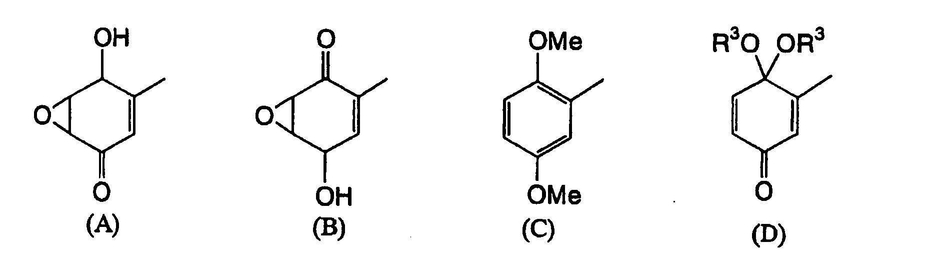 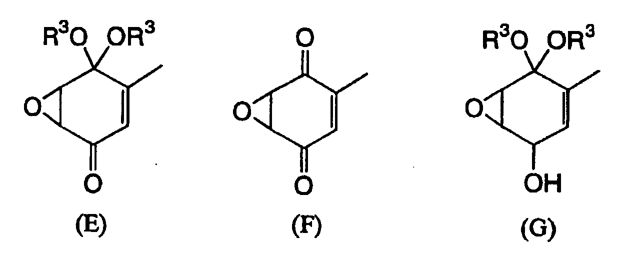 wherein R³ represents a C1-4 alkyl group. Examples of the alkyl group include a methyl group, an ethyl group, a propyl group, and butyl group, together with isomer groups thereof. Particularly preferred among these are a methyl group and an ethyl group.

At least one symptom may be improved by apoptosis of the aforementioned tumor cells, or at least one symptom resulting from the aforementioned tumor cells may be improved without the contribution of apoptosis of the tumor cells.

At least one symptom resulting from the aforementioned tumor cells may be improved by inhibiting activation of NF-κB.

The aforementioned symptom is, for example, tumor metastasis. Tumor metastasis may be improved by inhibiting adhesion to vascular endothelial cells.

At least one symptom resulting from the aforementioned tumor cells may be improved by inhibiting proliferation of the tumor cells.

The aforementioned symptom is one selected from the group consisting of Hodgkin's disease, cancer cachexia, and leukemia. The aforementioned tumor cells are, for example, breast cancer cells etc.

The aforementioned compound may be the following formula (1a) or (1b).

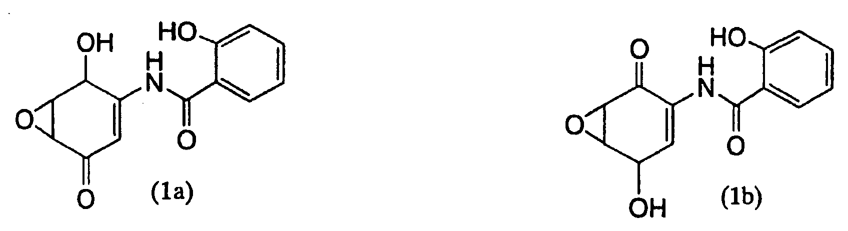

At least one symptom among loss of body weight, a decrease in hematocrit, a decrease in fat, and a decrease in muscle, which are the symptoms of the cancer cachexia, may be prevented or improved. However, symptoms accompanying cachexia are not limited to these; dry skin and an edema fall within the scope of the present invention.

Further, the pharmaceutical composition according to the present invention may improve at least one symptom resulting from the aforementioned tumor cells by inhibiting intratumoral angiogenesis formed by the tumor cells.

The pharmaceutical composition according to the present invention contains as an active ingredient a compound, represented by the following general formula (1), which is capable of enhancing the effect of a therapy by inhibiting activation of NF-κB caused by the therapy that causes the activation of NF-κB, or a pharmacologically acceptable salt thereof. 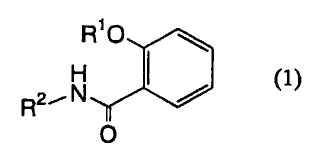 wherein R¹ represents a hydrogen atom or a C2-4 alkanoyl group. Examples of the alkanoyl group includes, acetyl, propionyl, and butanoyl groups, together with isomer groups thereof, and particularly preferred among these is an acetyl group.

R² is a group represented the following formulae (A), (B), (C), (D), (E), (F), and (G). 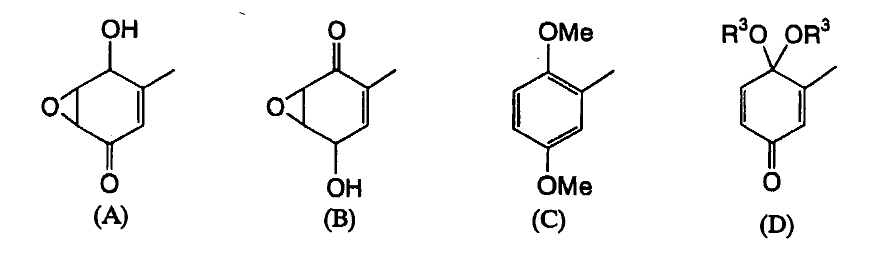 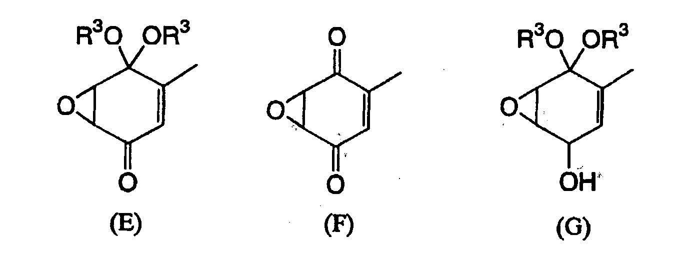 wherein R³ represents a C1-4 alkyl group. Examples of the alkyl group includes a methyl group, an ethyl group, a propyl group, and butyl group, together with isomer groups thereof. Particularly preferred among these are a methyl group and an ethyl group.

The therapy that activates NF-κB may be a therapy using an antitumor agent or radiotherapy for tumor cells. The aforementioned pharmaceutical composition may contain the aforementioned antitumor agent as an active ingredient. The antitumor agent is illustratively camptothecin or daunorubicin.

The aforementioned compound may be the following formula (1a) or (1b).

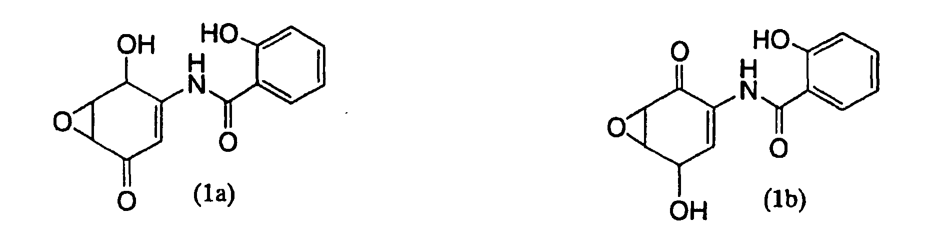

The tumor cell proliferation inhibitor for inhibiting proliferation of tumor cells according to the present invention contains a compound represented by the following general formula (1) or a pharmacologically acceptable salt thereof as an active ingredient. 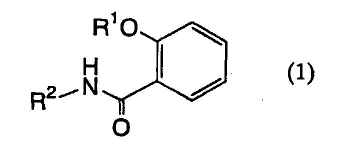 wherein R¹ represents a hydrogen atom or a C2-4 alkanoyl group. Examples of the alkanoyl group includes, acetyl, propionyl, and butanoyl groups, together with isomer groups thereof and particularly preferred among these is an acetyl group.

R² is a group represented the following formulae (A), (B), (C), (D), (E), (F), and (G). 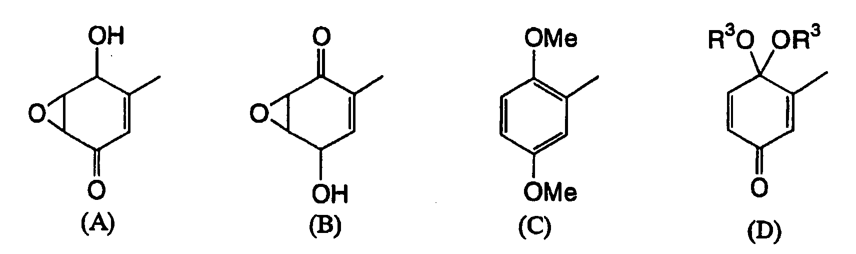 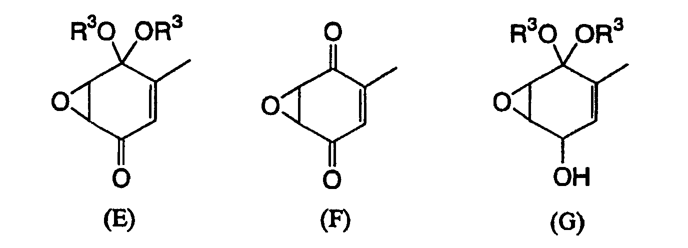 wherein R³ represents a C1-4 alkyl group. Examples of the alkyl group include a methyl group, an ethyl group, a propyl group, and butyl group, together with isomer groups thereof. Particularly preferred among these are a methyl group and an ethyl group.

The aforementioned compound may be the following formula (1a) or (1b).

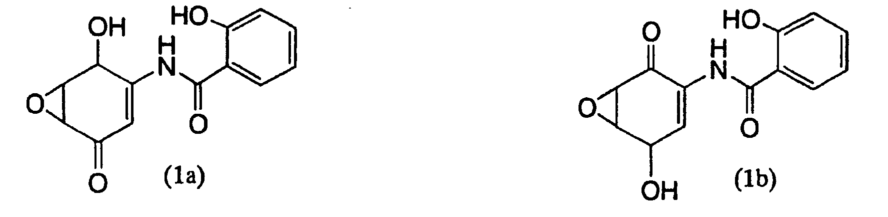

The adhesion molecule expression inhibitor for suppressing the expression of adhesion molecules in vascular endothelial cells contains a compound represented by the following general formula (1) or a pharmacologically acceptable salt thereof as an active ingredient. 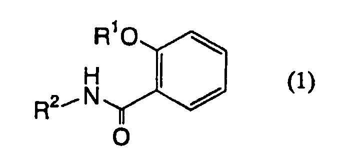 wherein R¹ represents a hydrogen atom or a C2-4 alkanoyl group. Examples of the alkanoyl group include acetyl, propionyl, and butanoyl groups, together with isomer groups thereof and particularly preferred among these is an acetyl group.

R² is a group represented the following formulae (A), (B), (C), (D), (E), (F), and (G). 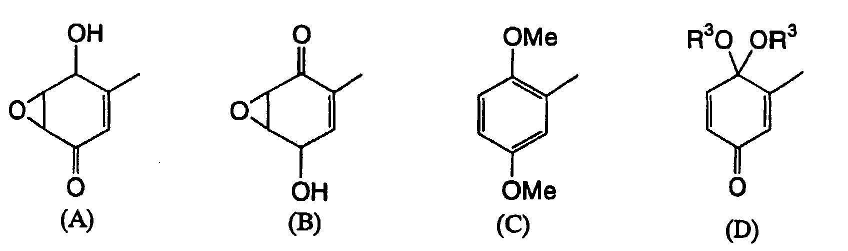 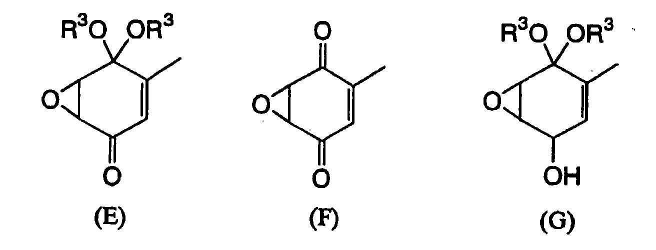 wherein R³ represents a C1-4 alkyl group. Examples of the alkyl group include a methyl group, an ethyl group, a propyl group, and butyl group, together with isomer groups thereof. Particularly preferred among these are a methyl group and an ethyl group.

The aforementioned compound may be the following formula (1a) or (1b).

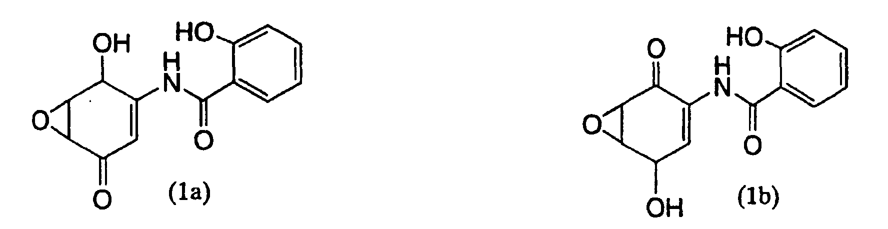

The apoptosis inducer for inducing apoptosis of tumor cells contains a compound represented by the following general formula (1) or a pharmacologically acceptable salt thereof as an active ingredient. 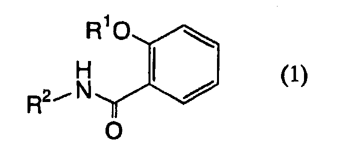 wherein R¹ represents a hydrogen atom or a C2-4 alkanoyl group. Examples of the alkanoyl group includes, acetyl, propionyl, and butanoyl groups, together with isomer groups thereof, and particularly preferred among these is an acetyl group.

R² is a group represented the following formulae (A), (B), (C), (D), (E), (F), and (G). 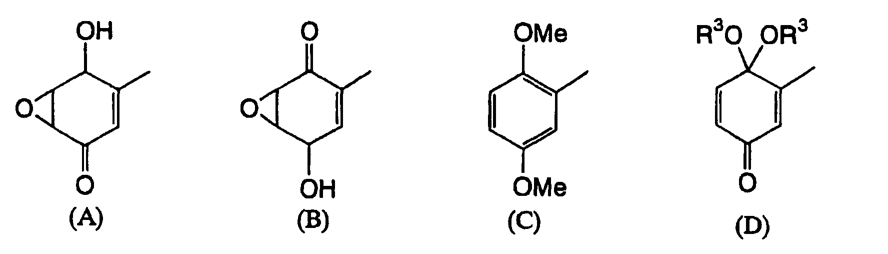 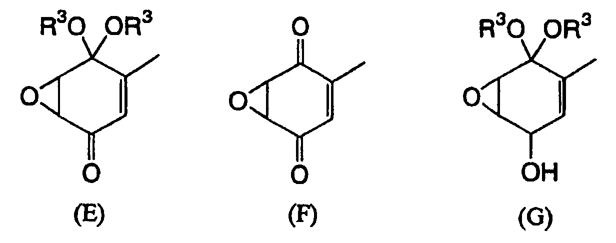 wherein R³ represents a C1-4 alkyl group. Examples of the alkyl group include a methyl group, an ethyl group, a propyl group, and butyl group, together with isomer groups thereof. Particularly preferred among these are a methyl group and an ethyl group.

The aforementioned compound may be the following formula (1a) or (1b).

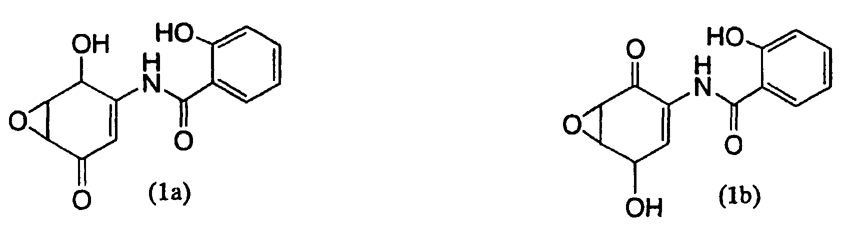

The preventive and therapeutic agent for arteriosclerosis according to the present invention contains a compound having NF-κB-inhibitory effect as an active ingredient. The compound having NF-κB-inhibitory effect may be a compound represented by the following general formula (1) or a pharmacologically acceptable salt thereof. 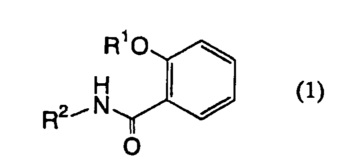 wherein R¹ represents a hydrogen atom or a C2-4 alkanoyl group. Examples of the alkanoyl group include acetyl, propionyl, and butanoyl groups, together with isomer groups thereof and particularly preferred among these is an acetyl group.

R² is a group represented the following formulae (A), (B), (C), (D), (E), (F), and (G). 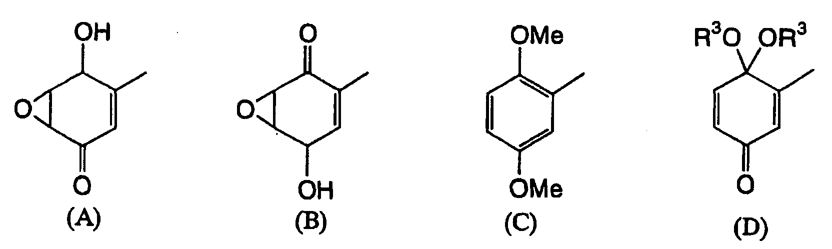 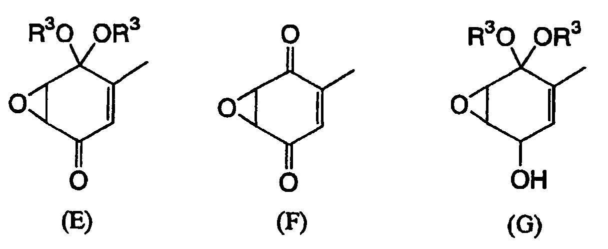 wherein R³ represents a C1-4 alkyl group. Examples of the alkyl group include a methyl group, an ethyl group, a propyl group, and butyl group, together with isomer groups thereof. Particularly preferred among these are a methyl group and an ethyl group.

The preventive and therapeutic agent for cancer according to the present contains a compound having NF-κB-inhibitory effect as an active ingredient. The compound having NF-κB-inhibitory effect may be a compound represented by the following general formula (1) or a pharmacologically acceptable salt thereof. 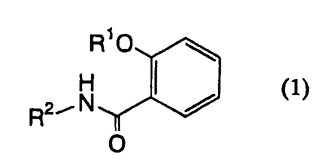 wherein R¹ represents a hydrogen atom or a C2-4 alkanoyl group. Examples of the alkanoyl group include, acetyl, propionyl, and butanoyl groups, together with isomer groups thereof, and particularly preferred among these is an acetyl group.

R² is a group represented the following formulae (A), (B), (C), (D), (E), (F), and (G). 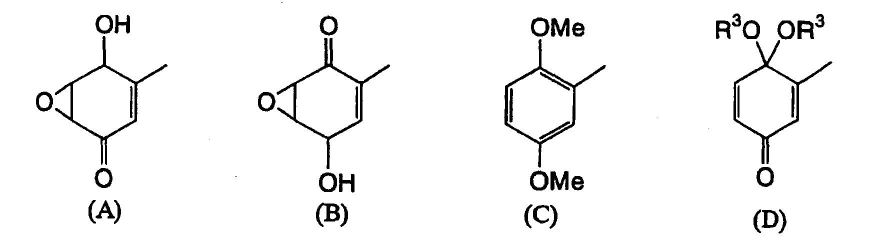 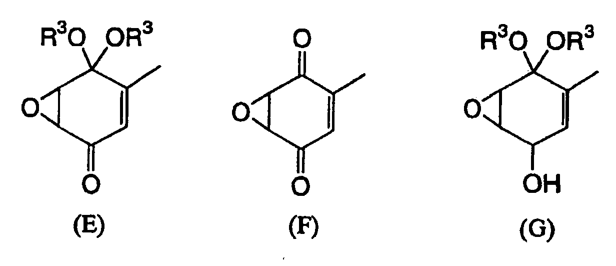 wherein R³ represents a C1-4 alkyl group. Examples of the alkyl group include a methyl group, an ethyl group, a propyl group, and butyl group, together with isomer groups thereof. Particularly preferred among these are a methyl group and an ethyl group.

The aforementioned preventive and therapeutic agent may be used for repressing cancer metastasis.

The therapeutic agent for cachexia according to the present invention contains a compound represented by the following general formula (1) or a pharmacologically acceptable salt thereof as an active ingredient. 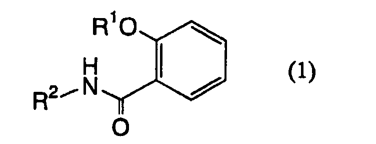 wherein R¹ represents a hydrogen atom or a C2-4 alkanoyl group. Examples of the alkanoyl group include, acetyl, propionyl, and butanoyl groups, together with isomer groups thereof, and particularly preferred among these is an acetyl group.

R² is a group represented the following formulae (A), (B), (C), (D), (E), (F), and (G). 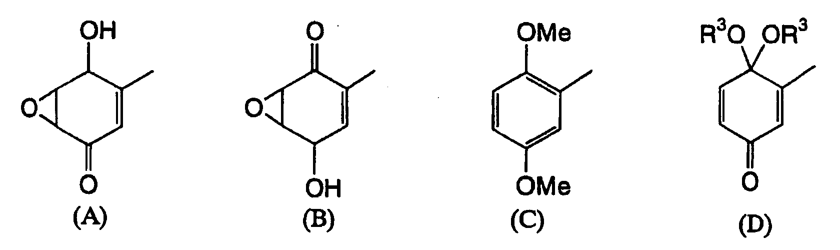 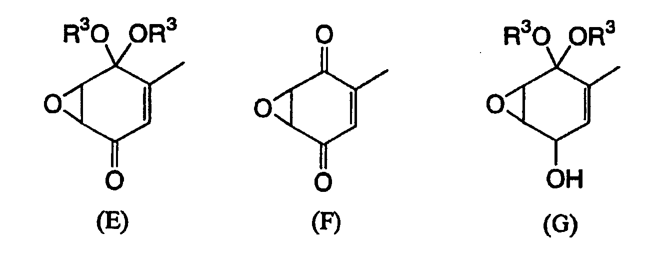 wherein R³ represents a C1-4 alkyl group. Examples of the alkyl group include a methyl group, an ethyl group, a propyl group, and butyl group, together with isomer groups thereof. Particularly preferred among these are a methyl group and an ethyl group.

The aforementioned compound may be the following formula (1a) or (1b).

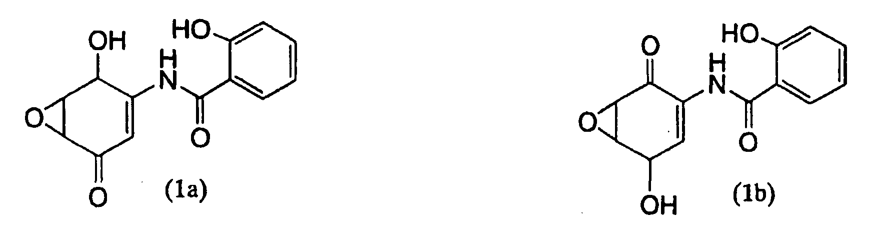

These compounds may be therapeutic agents for cancer cachexia in tumor patients (e.g., cancer-bearing patients). However, as long as the patients are the ones who have cachexia, the cause may not necessarily be a cancer.

At least one symptom among loss of body weight, a decrease in hematocrit, a decrease in fat, and a decrease in muscle, which are the symptoms of the cancer cachexia, may be prevented or improved. However, symptoms accompanying cachexia are not limited thereto; dry skin, an edema, etc. fall within the scope of the present invention.

Further, the therapeutic agent for cachexia according to the present invention may contain a compound having NF-κB-inhibitory effect as an active ingredient.

The therapeutic method according to the present invention uses a compound for improving at least one symptom resulting from tumor cells, represented by the following general formula (1), or a pharmacologically acceptable salt thereof. 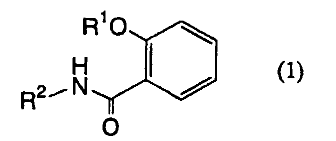 wherein R¹ represents a hydrogen atom or a C2-4 alkanoyl group. Examples of the alkanoyl group includes , acetyl, propionyl, and butanoyl groups, together with isomer groups thereof and particularly preferred among these is an acetyl group.

R² is a group represented the following formulae (A), (B), (C), (D), (E), (F), and (G). 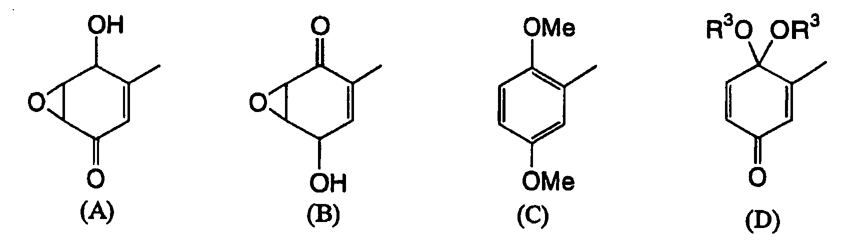 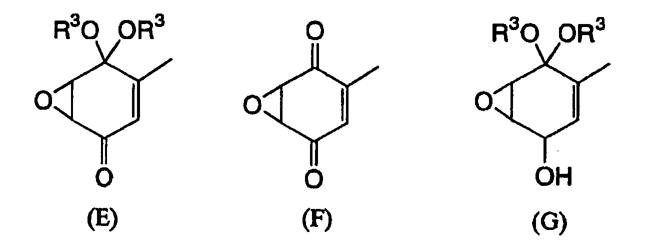 wherein R³ represents a C1-4 alkyl group. Examples of the alkyl group include a methyl group, an ethyl group, a propyl group, and butyl group, together with isomer groups thereof. Particularly preferred among these are a methyl group and an ethyl group. The therapeutic method includes a preventive method and a method for suppressing progression, etc.

At least one symptom may be improved through apoptosis of the aforementioned tumor cells, or at least one symptom resulting from the aforementioned tumor cells may be improved without the contribution of apoptosis of the tumor cells. Further, at least one symptom resulting from the aforementioned tumor cells may be improved by inhibiting activation of NF-κB.

The aforementioned symptom is one selected from the group consisting of tumor metastasis, symptoms resulting from proliferation of the aforementioned tumor cells, Hodgkin's disease, and cancer cachexia.

The aforementioned compound may be the following formula (1a) or (1b).

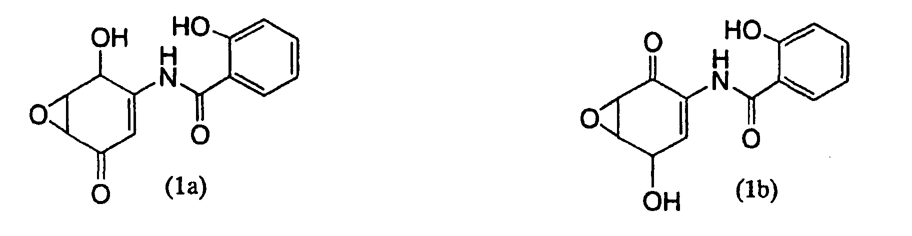

The therapeutic method according to the present invention may use a compound for improving arteriosclerosis by inhibiting adhesion of vascular endothelial cells to leucocytes, represented by the following general formula (1), or a pharmacologically acceptable salt thereof. 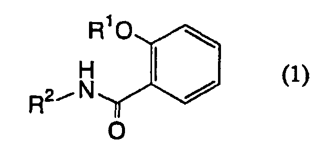 wherein R¹ represents a hydrogen atom or a C2-4 alkanoyl group. Examples of the alkanoyl group includes , acetyl, propionyl, and butanoyl groups, together with isomer groups thereof and particularly preferred among these is an acetyl group.

R² is a group represented the following formulae (A), (B), (C), (D), (E), (F), and (G). 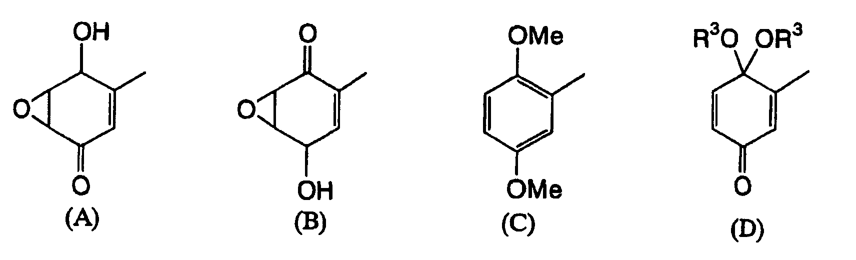 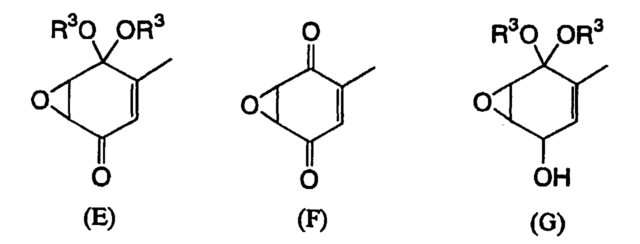 wherein R³ represents a C1-4 alkyl group. An alkyl group includes a methyl group, an ethyl group, a propyl group, and butyl group, together with isomer groups thereof. Particularly preferred among these are a methyl group and an ethyl group. The therapeutic method includes a preventive method for arteriosclerosis and a method for suppressing progression of arteriosclerosis, etc.

The aforementioned compound may be the following formula (1a) or (1b).

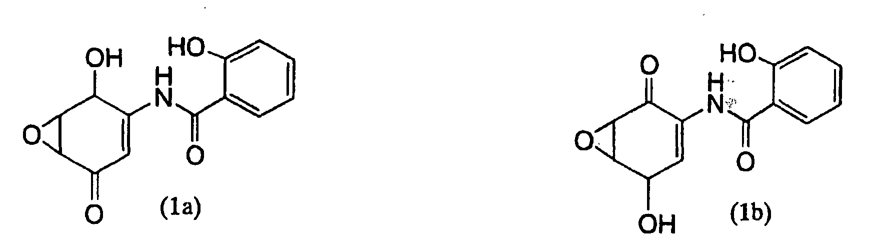

The therapeutic method according to the present invention may include the steps of performing a therapy for activating NF-κB and administering a pharmaceutical composition, containing a compound represented by the following general formula (1) or a pharmacologically acceptable salt thereof as an active ingredient. 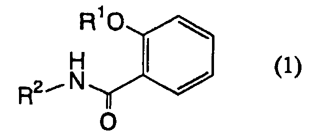 wherein R¹ represents a hydrogen atom or a C2-4 alkanoyl group. Examples of the alkanoyl group include acetyl, propionyl, and butanoyl groups, together with isomer groups thereof and particularly preferred among these is an acetyl group.

R² is a group represented by any of the following formulae (A), (B), (C), (D), (E), (F), and (G). 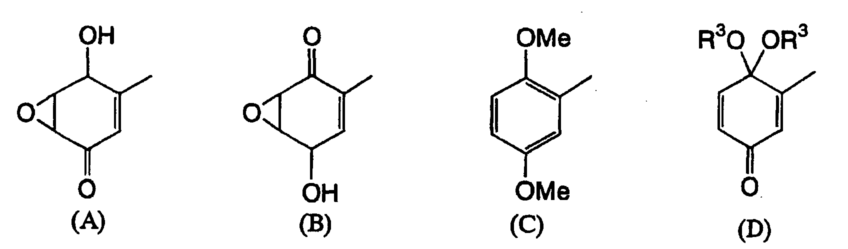 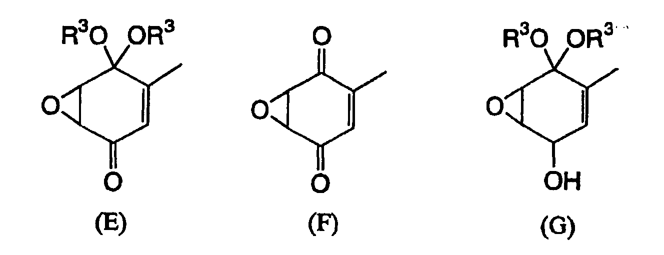 wherein R³ represents a C1-4 alkyl group. Examples of the alkyl group include a methyl group, an ethyl group, a propyl group, and butyl group, together with isomer groups thereof. Particularly preferred among these are a methyl group and an ethyl group. The therapeutic method includes a preventive method and a method for suppressing progression, etc.

The aforementioned therapy that activates NF-κB may be a therapy using an antitumor agent or irradiation to tumor cells.

The aforementioned compound may be the following formula (1a) or (1b).

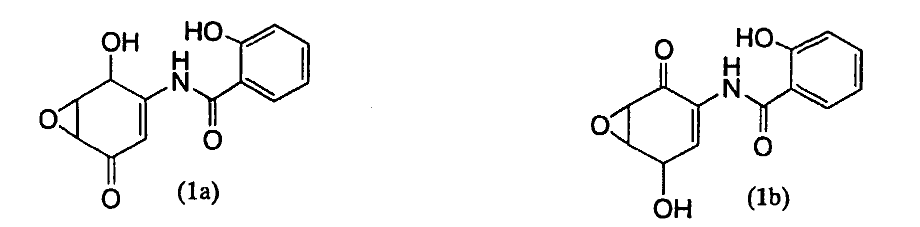

### BRIEF DESCRIPTION OF THE DRAWINGS

FIG. 1 schematically explains the mechanism of action by which a compound having a NF-κB-inhibitory effect suppresses arteriosclerosis and cancer metastasis.
FIG. 2 shows the inhibitory effect when TNF-stimulation is applied.

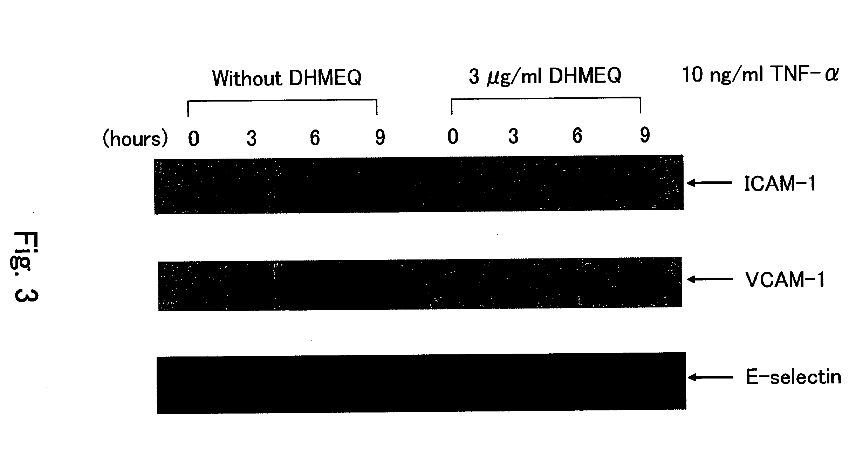

FIG. 3 shows the suppressive effect of DHMEQ on expression of ICAM-1, VCAM-1, and E-selectin when TNF-α stimulation is applied.

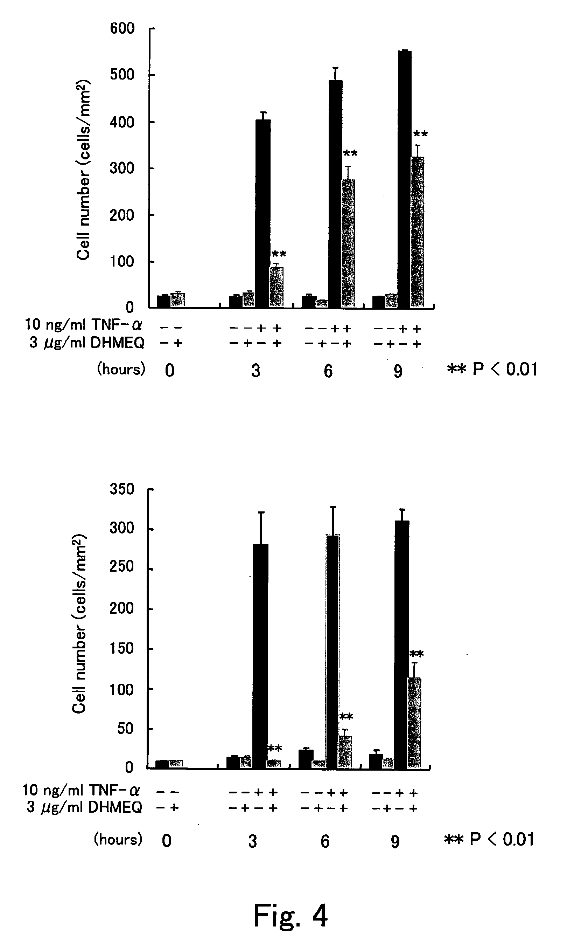

FIG. 4 shows the suppressive effect of DHMEQ on adhesion of HUVECs to leukocytes (upper) or that of HUVECs to HL-60 (lower).
FIG. 5 shows the results of analysis by the gel shift assay regarding the inhibitory effect of DHM2EQ on the constitutive activation of NF-κB in ATL cells.
FIG. 6 shows the results of analysis by the reporter gene assay regarding the inhibitory effect of DHM2EQ on the constitutive activation of NF-κB in ATL cells.
FIG. 7 shows the results of analysis by a confocal microscopy regarding the inhibitory effect of DHM2EQ on the constitutive activation of NF-κB in ATL cells.
FIG. 8 shows the results of a concentration-dependent analysis of the inhibitory effect of DHM2EQ on proliferation of ATL cells.
FIG. 9 shows the results of a time-course analysis of the inhibitory effect of DHM2EQ on proliferation of ATL cells.
FIG. 10 shows the results of an analysis of the inhibitory effect of DHM2EQ on proliferation of peripheral blood cells of ATL patients.
FIG. 11 shows the results of an analysis of the inhibitory effect of DHM2EQ on proliferation of normal peripheral blood mononuclear cells.
FIG. 12 shows the results of an analysis of the apoptosis-inducing effect of DHM2EQ on ATL cells.
FIG. 13 shows the results of an analysis of the inhibitory effect of DHM2EQ on proliferation of Hodgkin's lymphoma cells.
FIG. 14 shows the results of analysis of the inhibitory effect of DHM2EQ on proliferation of multiple myeloma cells.
FIG. 15 shows the effect in which activation of NF-κB induced by TNF-α is inhibited by DHMEQ.
FIG. 16 shows the suppressive effect of DHMEQ on proliferation of MCF-7.
FIG. 17 shows the suppressive effect of DHMEQ on proliferation of the human breast cancer cell line MCF-7 transplanted into SCID mice.
FIG. 18 shows the suppressive effect of the COX-2 inhibitor celecoxib on proliferation of Lewis lung tumors and HT-29.
FIG. 19 is a graph showing a luciferase activity observed when, after transfection of p6 kb-Luc into JCA-1 cells, DHMEQ with various concentrations was administered in one example of the present invention.
FIG. 20 is a graph showing the time-course changes in body weight of mice when DHMEQ was administered to cancer-bearing mice inoculated with JCA-1 cells in one example of the present invention.
FIG. 21 is a graph showing the time-course changes in tumor weight calculated from the diameters of tumors when DHMEQ was administered to cancer-bearing mice inoculated with JCA-1 cells in one example of the present invention.
FIG. 22 is a graph showing the tumor weights on day 26 after the start of administration of DHMEQ to cancer-bearing mice inoculated with JCA-1 cells in one example of the present invention.
FIG. 23 is a graph showing the weights of the fat around the testis on day 26 after the start of administration of DHMEQ to cancer-bearing mice inoculated with JCA-1 cells in one example of the present invention.
FIG. 24 is a graph showing the weights of the gastrocnemius on day 26 after the start of administration of DHMEQ to cancer-bearing mice inoculated with JCA-1 in one example of the present invention.
FIG. 25 is a graph showing the hematocrits on day 26 after the start of administration of DHMEQ to cancer-bearing mice inoculated with JCA-1 cells in one example of the present invention.
FIG. 26 is a table showing the weights of each organ <dissected> and then measured on day 26 after the start of administration of DHMEQ to cancer-bearing mice inoculated with JCA-1 cells in one example of the present invention.
FIG. 27 shows the results of the inhibitory effect of DHMEQ on constitutive activation of NF-κB in the multiple myeloma (MM) cell lines in one example of the present invention.
FIG. 28 shows the results of the inhibitory effect of DHMEQ on constitutive activation of NF-κB in the multiple myeloma (MM) cell lines in one example of the present invention.
FIG. 29 shows the results of the inhibitory effect of DHMEQ on proliferation of cells of multiple myeloma (MM) patients in one example of the present invention.
FIG. 30 shows the results of the inhibitory effect of DHMEQ on constitutive activation of NF-κB in Hodgkin' s lymphoma (HL) cell lines in one example of the present invention.
FIG. 31 shows the results of the effect of enhancing the action of antitumor agents exerted by DHMEQ in one example of the present invention.
FIG. 32 shows that the effect of enhancing the action of antitumor agents exerted by DHMEQ results from inhibition of activation of NF-κB caused by the antitumor agents in one example of the present invention.
FIG. 33 shows the results of investigation into an *in vivo* effect of DHMEQ using SCID mice that had their abdominal cavity inoculated with ATL cell lines in one example of the present invention.
FIG. 34 shows the results of investigation into the apoptosis-enhancing effect of DHMEQ on irradiated tumor cells in one example of the present invention.
FIG. 35 shows the results of investigation into an *in vivo* suppressive effect of DHMEQ on proliferation of tumor cells in human pancreatic cancer in one example of the present invention.
FIG. 36 shows the results of investigation into an *in vitro* suppressive effect of DHMEQ in combination with irradiation on proliferation of tumor cells in human pancreatic cancer cell lines in one example of the present invention in combination with irradiation.

### BEST MODE FOR CARRYING OUT THE INVENTION

The objective, characteristics, and advantages of the present invention as well as the idea thereof will be apparent to those skilled in the art from the descriptions given herein. It is to be understood that the embodiments and specific examples of the invention described hereinbelow are to be taken as preferred examples of the present invention. These descriptions are for illustrative and explanatory purposes only and are not intended to limit the invention to these embodiments or examples. It is further apparent to those skilled in the art that various changes and modifications may be made based on the descriptions given herein within the intent and scope of the present invention disclosed herein.

The pharmaceutical composition according to the present invention can improve at least one symptom resulting from tumor cells. To improve symptoms resulting from tumor cells, apoptosis of tumor cells may be caused. Examples of the symptom to be the subjects of this method include, but not limited to, Hodgkin's disease, leukemia, etc. Further, to improve symptoms resulting from tumor cells, apoptosis of tumor cells does not need to make contribution; examples of the symptoms in this case include, but not limited to, tumor metastasis, cancer cachexia, etc. "Not to make contribution" as used herein means that even if the pharmaceutical composition according to the present invention is administered to the affected part, the effect does not depend on apoptosis of the tumor cells in the affected part. However, apoptosis of tumor cells may take place, apart from the effect on which apoptosis does not depend. Incidentally, "tumor" has the same meaning as cancer in a broad sense and refers to, for example, malignant tumors of the lymphoid system, breast cancer, cancer-bearing, pancreatic cancer, etc.

It has been reported that activation of NF-κB is involved in various aspects of tumorigenesis including regulation of apoptosis, cell proliferation, and cell differentiation (Albert S.Baldwin, J.Clin.Invest. 107: 241-246 (2001)).
It has also been reported that activation of NF-κB in cancer cells by chemotherapy or radiotherapy decreases the effect of cancer therapy (Albert S.Baldwin, as mentioned above). The inventors therefore thought that DHMEQ, represented by the aforementioned formula (1), designed and synthesized base on the structure of the antibiotic epoxyquinomicin C, has an anticancer effect in cancer as well, as described above.

The inventors investigated the influence of DHMEQ on activation of NF-κB of adult T cell leukemia/lymphoma (ATL) cells and clarified that DHMEQ inhibits activation of NF-κB in ATL cells. Upon further investigation of the influence of DHMEQ on proliferation of ATL cells, the inventors clarified that DHMEQ does not inhibit proliferation of normal cells but does inhibit proliferation of ATL cells. Yet another investigation of the influence of DHMEQ on induction of apoptosis of ATL cells revealed that DHMEQ induces apoptosis of ATL cells but does not induce apoptosis of normal cells. Compounds having NF-κB-inhibitory effect are illustratively salicylamide derivatives (WO01/12588 A1), panepoxydone (Biochem.Biophys.Res.Commun.226, 214-221, 1996), cycloepoxydon (J.Antibiot.51, 455-463, 1998), and SN-50 (J.Biol.Chem.270, 14255-14258). The methods for producing manufacturing these other compounds having NF-κB-inhibitory effect are described in the following literatures: Panepoxydone, cycloepoxydon, and SN50 are described in Biochem.Biophys.Res.Commun.226, 214-221, 1996; J.Antibiot. 51, 455-463, 1998; and J. Biol. Chem. 270, 14255-14258, and 1995, respectively.

Moreover, the inventors investigated the influence of DHMEQ on proliferation of Hodgkin's lymphoma cells and clarified that DHMEQ inhibits proliferation of Hodgkin's lymphoma cells in which NF-κB is activated but does not inhibit proliferation of myelocytic leukemia cells in which NF-κB is not activated. Further, upon investigation of DHMEQ on proliferation of a multiple myeloma cells, the inventors clarified that DHMEQ inhibits proliferation of multiple myeloma cells as well.

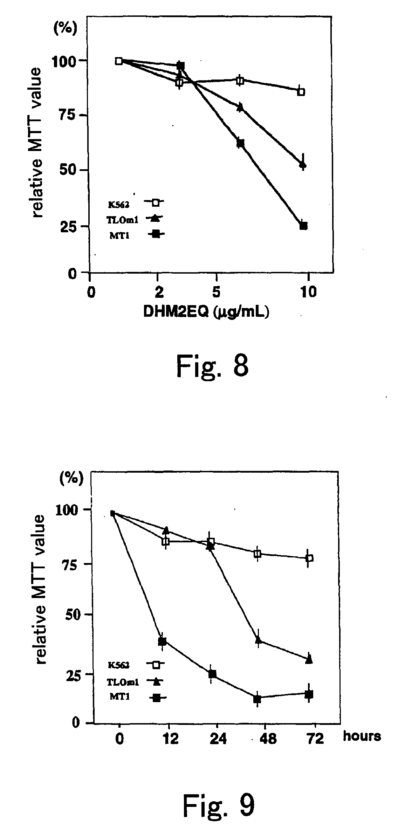

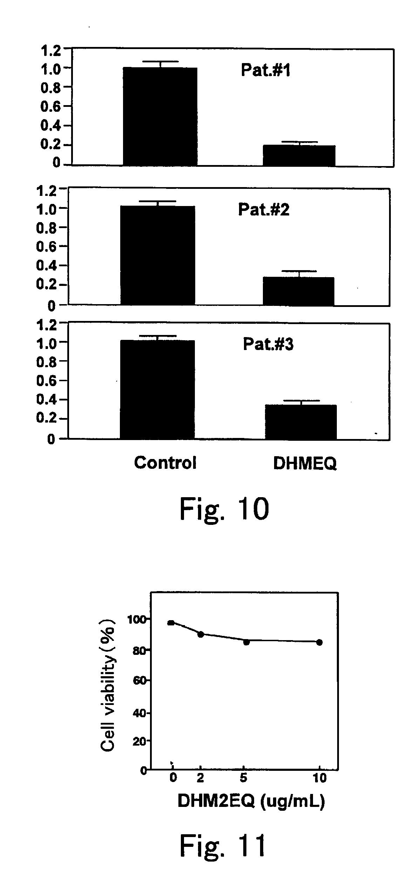

Conventional chemotherapy widely has targeted proliferation mechanisms universal among cells; it had a significant impact not only on tumor cells but also on normal cells and thus it was not necessarily effective therapeutic means. The NF-κB inhibitor DHMEQ, however, represented by the aforementioned general formula (1) induces apoptosis of leukemia cells in which NF-κB is activated but does not in the least induce apoptosis of human normal leukocytes at the same concentration, as revealed in the experiments (FIGS 8 and 11), indicating DHMEQ has a high specificity to tumor cells. Consequently, therapy with DHMEQ has a fewer side effects than conventional chemotherapy; DHMEQ is more useful as a pharmaceutical composition to tumors including malignant tumors.

DHMEQ is also useful as an apoptosis inducer of tumor cells. For example, the pharmaceutical composition according to the present invention can prevent or treat malignant tumors of the lymphoid system by the inhibitory effect on proliferation of malignant tumor cells of the lymphoid system, together with the apoptosis-inducing effect on malignant tumor cells of the lymphoid system. Examples of the types of malignant tumors of the lymphoid system to be prevented or treated preferably include, but not limited to, malignant lymphoma, leukemia, or myeloma. Malignant lymphoma includes non-Hodgkin's lymphoma, Hodgkin's lymphoma, etc. Myeloma includes plasma cell tumors etc., such as multiple myeloma. Leukemia includes acute lymphatic leukemia, adult T-cell leukemia/lymphoma, chronic lymphocytic leukemia, etc.

The tumor cell proliferation inhibitor according to the present invention can suppress proliferation of malignant tumor cells of the lymphoid system by an effect of inhibiting proliferation of malignant tumor cells of the lymphoid system, thereby capable of preventing or treating malignant tumors of the lymphoid system. Examples of the types of malignant tumor cells of the lymphoid system to be inhibited from proliferating preferably include, but not limited to malignant lymphoma cells, leukemia cells, or myeloma cells. Malignant lymphoma, leukemia, or myeloma includes various malignant tumors of the lymphoid systems illustrated above.

The apoptosis inducer according to the present invention can induce apoptosis of malignant tumor cells of the lymphoid system by the apoptosis-inducing effect on malignant tumor cells of the lymphoid system, thereby capable of preventing or treating malignant tumors of the lymphoid system. Examples of the types of malignant tumor cells of the lymphoid system in which apoptosis is to be induced preferably include, but not limited to, malignant lymphoma cells, leukemia cells , or myeloma cells. Malignant lymphoma, leukemia, or myeloma includes various malignant tumors of the lymphoid systems illustrated above.

Since the pharmaceutical composition, tumor cell proliferation inhibitor, and apoptosis inducer according to the present invention act specifically on malignant-tumor cells of the lymphoid system and have practically no bad influence on normal cells, they are promising for exerting excellent preventive and therapeutic effects on malignant tumors of the lymphoid system.

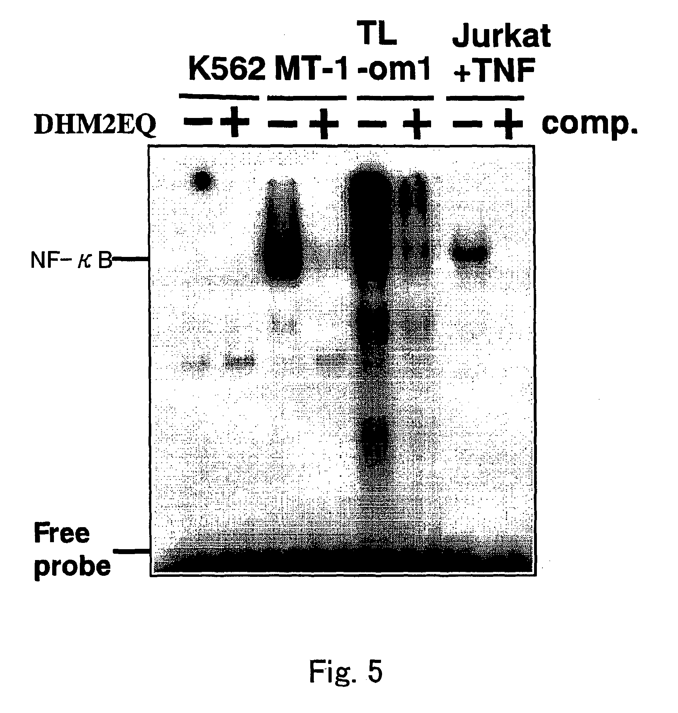

The compounds (particularly DHMEQ) represented by the general formula (1) and pharmacologically acceptable salts thereof act on cancer cells as well on interstitial (the part composed of normal cells in cancer tissue) cells in cancer tissue. Particularly, in vascular endothelial cells in cancer tissue, DHMEQ does not cause apoptosis but suppresses expression of an adhesion molecule etc. (FIG. 5), thereby exerting a suppressive effect on cancer progression.

If inflammatory, physical, and other stimuli are applied to vascular endothelial cells, expression of adhesion molecules is enhanced, and leukocytes adhere to the surface of the vascular endothelial cells to migrate out of blood vessels. This is because expression of adhesion molecules, such as ICAM-1, VCAM-1, and E-selectin, is activated by activation of NF-κB, a transcription factor in vascular endothelial cells. Further, it has been reported that sialyl Lewis X, an E-selectin ligand, is highly expressed in high-metastasizing colon cancer, suggesting that activation of NF-κB, a transcription factor in vascular endothelial cells, facilitates adhesion of colon cancer cells to the surface of vascular endothelial cells, thereby facilitating exudation of the cells out of blood vessels and, accordingly, promoting metastasis. Thus, it is thought that suppressing expression of the above-mentioned adhesion molecules on the surface of vascular endothelial cells will lead to suppression of arteriosclerosis or tumor metastasis, and that an NF-κB inhibitor is useful in suppressing arteriosclerosis or metastasis of cancer cells.

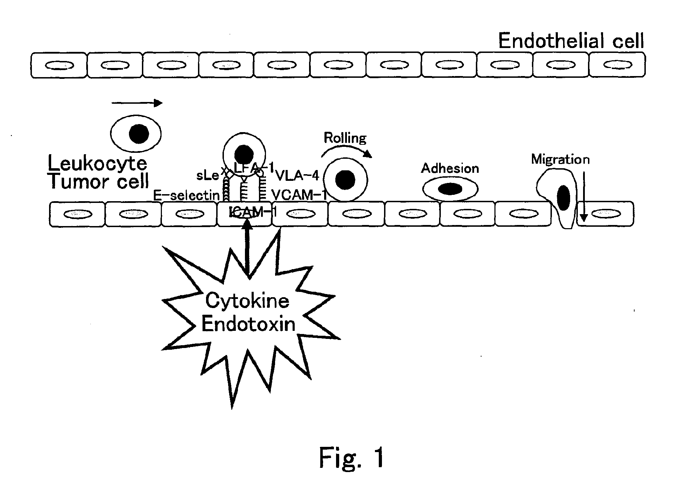

Accordingly, the inventors investigated the influence of DHMEQ on expression of adhesion molecules in human umbilical vein endothelial cells (HUVECs). Activation of NF-κB by TNF-α was evaluated by the gel shift assay. Activation of NF-κB was suppressed without suppressing degradation of IκB-α. Further, the influence of DHMEQ on expression of ICAM-1, VCAM-1, and E-selectin, induced by TNF-α, was investigated by the Western blot technique. It was found that DHMEQ suppresses expression of these adhesion molecules and inhibits both adhesion of leukocytes to HUVECs and that of leukemia cells to HUVECs as well. While adhesion of leukocytes to the blood vessel wall induces arteriosclerosis via accumulation of lipid etc., adhesion of cancer cells causes their exudation and metastasis from blood vessels. Inhibiting these adhesions, accordingly, leads to an anti-arteriosclerosis agent in the former and a cancer metastasis inhibitor in the latter. The aforementioned compound having the NF-κB-inhibitory effect is therefore in suppressing arteriosclerosis or metastasis cancer cells (FIG. 1). DHMEQ is useful as an expression inhibitor of adhesion molecules in vascular endothelial cells, as will be shown in the Examples given later.

Another example of the effect that does not depend on apoptosis is suppression of cell proliferation. The inventors investigated the in vitro and in vivo influences of DHMEQ on proliferation of human breast cancer cells, and clarified that DHMEQ suppresses proliferation of human breast cancer cells. The compounds represented by the general formula (1) and pharmacologically acceptable salts thereof have the suppressive effect on proliferation of breast cancer cells. These compounds are therefore useful as preventive and therapeutic agents for breast cancer.

Conventionally, breast cancer therapy is divided roughly into two known methods: chemotherapy (the therapy based on anticancer agents) and hormone therapy. Both of these methods are known to have the effects of regressing cancer and preventing its recurrence, but they have had problems. That is, anticancer agents have the biggest problem of having toxicity (side effects), together with a significant problem of drug tolerance. A major problem with hormone therapy is that it is effective only to cancers having hormone sensitivity. Hormone-sensitive cancers account for 60% of the total, and patients with cancers of the remaining 40% cannot receive hormone therapy from the beginning. Drug tolerance has developed in hormone therapy as well, causing a serious problem. DHMEQ is therefore extremely useful for breast cancer.

IL-6 and TNF-α are involved in the mechanism of cachexia development commonly observed in terminally ill patients etc. of chronic disease, particularly malignant tumors. Thus, on the assumption that inhibition of the functions of NF-κB, an intracellular target molecule, is effective in prevention/improvement of symptoms resulting from cachexia, the inventors investigated whether or not DHMEQ is useful in the case of cachexia as well. That is, DHMEQ was administered to model mice with induced cachexia symptoms and the symptoms were observed. It was found that DHMEQ is effective in prevention/improvement of cachexia symptoms. This revealed that DHMEQ is also useful for cachexia.

Further, since the pharmaceutical composition according to the present invention can inhibit activation of NF-κB, it follows that the composition can suppress gene expression of cyclooxygenase 2 (COX-2) that occurs by activation of NF-κB. Moreover, being capable of suppressing gene expression of cyclooxygenase 2 makes it possible to suppress synthesis of prostaglandin as well, which will probably enable inhibition or suppression of tumor angiogenesis promoted by prostaglandin. It is therefore expected that the pharmaceutical composition according to the present invention is also useful as a therapeutic agent that exerts an antitumor effect by inhibiting intratumoral angiogenesis and blocking provision of supply of oxygen and nutritive substance to tumors.

Further, on the assumption that the effect of oncotherapy could be enhanced by inhibiting activation of NF-κB in tumor cells resulting from therapies that activate NF-κB, such as chemotherapy and radiotherapy, the inventors first investigated the enhancing effect of DHMEQ on the actions of antitumor agents. Antitumor agents such as amptothecin (CPT) and daunomycin (DNR) were used and the enhancing effect of DHMEQ on the actions of such antitumor agents was examined. It was found that DHMEQ enhances the effect of any antitumor agent tested.

The inventors also investigated activation of NF-κB by therapies with various antitumor agents. It was found that therapy with any antitumor agent--camptothecin (CPT), daunomycin (DNR), or etoposide (ETP) -- transiently enhanced the NF-κB activity in tumor cells 3 to 20-fold, as compared with that in tumor cells before therapy. This revealed that the enhancing effect of DHMEQ on the actions of antitumor agents results from inhibition of activation of NF-κB caused by antitumor agents.

Next, to investigate the DHMEQ-associate inhibition of activation of NF-κB caused by antitumor agents, activation of NF-κB in tumor cells treated by various antitumor agents (camptothecin (CPT) and daunomycin (DNR)) in combination with DHMEQ was investigated. It was found that, in therapy with either antitumor agent, activation of NF-κB is strongly suppressed by the concurrent use of DHMEQ.

Meanwhile, since radiotherapy generates active hydrogen, it is likely to cause another cancer by damaging DNA in normal tissue as well or to aggravate the remaining cancer cells. It is known that, when activation of NF-κB has been induced by oxidization stress by irradiation, it becomes difficult for cancer cells to undergo apoptosis, thereby exhibiting resistance to radiotherapy. Thus, on the assumption that the combined use of DHMEQ and irradiation would produce a synergistic effect, the present inventors investigated an in vitro suppressive effect of DHMEQ in combination with irradiation on proliferation of tumor cells. As a result, it was found that DHMEQ exhibits a synergistic effect of suppressing proliferation in combination with irradiation.

Thus, the pharmaceutical composition according to the present invention is useful also as an inhibitor of activation of NF-κB caused by therapy using antitumor agents, therapy by tumor cell irradiation, etc. The pharmaceutical composition according to the present invention is useful also in combination with an antiviral agent. The therapy using antitumor agents using an antitumor agent is not limited as long as it is an antitumor agent that activates NF-κB. Examples of such an antitumor agent include camptothecin, daunorubicin, etc.

The pharmaceutical composition according to the present invention can produce an enhanced therapeutic effect by using in combination with therapies for neoplastic, allergic, immunological, inflammatory, and other diseases. The pharmaceutical composition according to the present invention may be used simultaneously with the therapy that activates NF-κ B, but it may be used to inhibit activation of NF-κB after the therapy that activates NF-κB. To inhibit activation of NF-κ B, the pharmaceutical composition according to the present invention may be administered in advance to mammals including humans and animals other than humans afflicted with the above-mentioned diseases.

One big problem with the current anticancer drug therapy is that tolerance, having the mechanism of drug excretion, often develops in cancer cells, whereas, in general, it is considered difficult for normal cells to develop resistance. This point is therefore the first advantage of the preventive and therapeutic agents for breast cancer according to the present invention. A second advantage is that a low toxicity is expected, which is an advantage over chemotherapy.
Further, in terms of the action mechanism, hormone-insensitive tumors that hormone therapy does not target are also targeted for therapy. This point is the great advantage over hormone therapy. In summary, a low toxicity, a different target from that of hormone therapy, a low tolerance in vascular endothelial cells etc. will be great clinical advantages.
These points also suggest the possibility of a combined use with conventional drugs and application to cancer prevention.

In conclusion, the pharmaceutical composition according to the present invention is useful for tumor cell proliferation inhibitors, adhesion molecule expression inhibitors, apoptosis inducers, preventive and therapeutic agents for arteriosclerosis or cancer, preventive and therapeutic agents for malignant tumors of the lymphoid system, prevention and the therapeutic agents for breast cancer, and therapeutic agents for cachexia, etc.

The pharmaceutical composition containing the compound represented by general formula (1) as an active ingredient according to the present invention is therefore useful for therapies (including preventive agents, progression suppressors, etc.) of tumors, cachexia, arteriosclerosis, etc.

The methods for producing the compounds according to the present invention and usage form and examples are hereinafter described in detail.

### === Process for producing the compounds of the present invention ===

The compounds represented by the general formula (1) can be produced according to the synthetic process by Wipf et al. (Synthesis, No. 12, p. 1549-1561, 1995).

One example of the processes for producing compounds represented by the general formula (1) will be illustrated hereinbelow, based on the following reaction schemes.

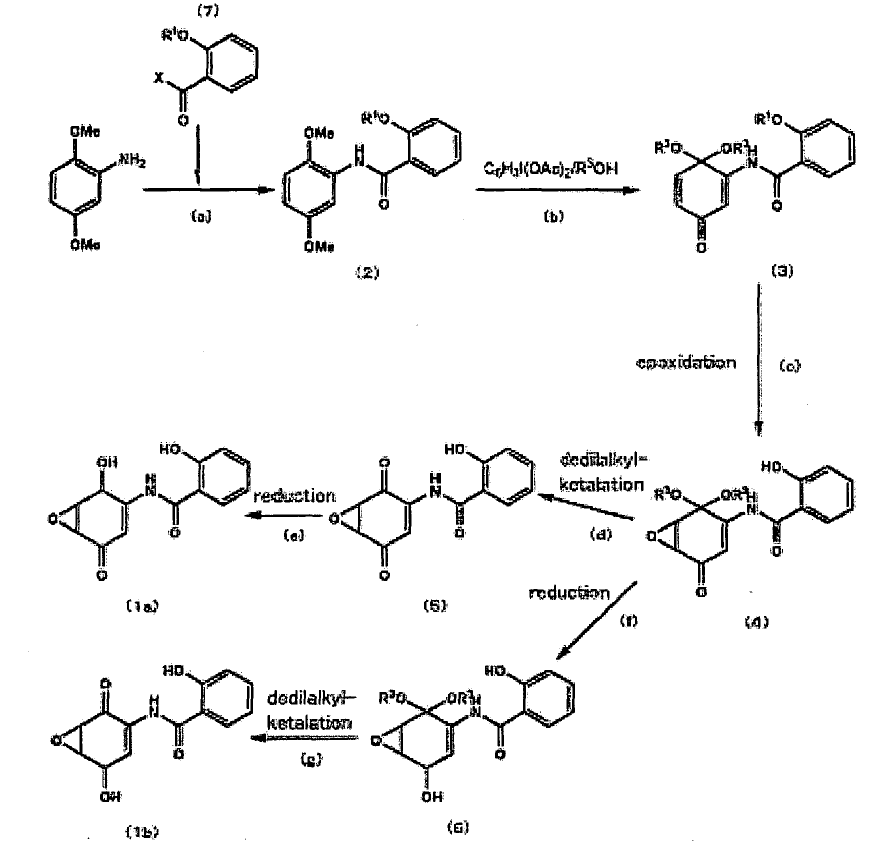

### Step a: Preparation of N-(2-alkanoylbenzoyl)-2,5-dimethoxyaniline

2, 5-Dimethoxyaniline is dissolved in a solvent (pyridine, etc.), and ethyl acetate solution of O-alkanoylsalicyloyl halide is added thereto at -78°C to 50°C, preferably under ice cooling, and the mixture is reacted while stirring. After stopping the reaction by addition of water, ethyl acetate is added to the reaction mixture, which then is sequentially washed with hydrochloric acid, water, a sodium hydrogencarbonate solution and water. After drying, the organic layer is concentrated under reduced pressure and dried under vacuum to obtain an N-(2-alkanoylbenzoyl)-2,5-dimethoxyaniline compound represented by formula (2). The compound can be used in the next step without purification.

### Step b: Preparation of 3-(O-alkanoylsalicyloyl) amino-4,4-dialkoxy-2,5-cyclohexadienone

The compound of formula (2) obtained as described above is dissolved in a solvent such as methanol, diacetoxyiodobenzene is added thereto at -20°C to 50°C, preferably under ice cooling and the mixture is reacted at room temperature while stirring. After concentration under reduced pressure, ethyl acetate is added and the reaction mixture is washed with sodium hydrogencarbonate solution and saline. Then, the solvent is concentrated under reduced pressure and the obtained residue is purified by column chromatography to obtain 3-(O-alkanoylsalicyloyl) amino-4,4-dialkoxy-2,5-cyclohexadienone.

### Step c: Preparation of 5,6-epxoy-4,4-dialkoxy-3-salicyloylamino-2-cyclohexenone compound

3-(O-Alkanoylsalicyloyl)amino-4,4-dialkoxy-2,5-cycloh exadienone represented by formula (3) is dissolved in a solvent (tetrahydrofuran, methanol, etc.), hydrogen peroxide water and sodiumhydroxide are added thereto at -20°C to 50°C, preferably under ice cooling, and the mixture is reacted while stirring. Ethyl acetate is added to the reaction mixture, which is sequentially washed with hydrochloric solution, aqueous sodium thiosulfate solution, and saline. After drying in the air, the reaction mixture is dried under vacuum. In order to remove the residual starting compound, the residue is dissolved in acetone; p-toluenesulfonic acid is added thereto and stirred at room temperature to decompose the starting compound. Ethyl acetate is added to the residue obtained by distilling off methanol under reduced pressure, and the solution is washed with water. The residue obtained by drying the ethyl acetate layer is purified by column chromatography to obtain 5,6-epxoy-4,4-dialkoxy-3-salicyloylamino-2-cyclohexenone compound represented by formula (4).

### Step d: Preparation of 5,6-epoxy-2-salicyloylamino-2cyclohexen-1,4dione

5,6-Epxoy-4,4-dialkoxy-3-salicyloylamino-2-cyclohexen one compound represented by formula (4) is dissolved in methylene chloride, an inorganic acid or organic acid (trifluoroboron diethyl ether complex, etc.) is added under ice cooling, and the mixture is reacted while stirring. A solvent (ethyl acetate, etc.) is added to the reaction mixture, which is washed with water. After concentrating the ethyl acetate layer, the obtained residue is washed with methanol to obtain 5,6-epoxy-2-salicyloylamino-2-cyclohexen-1,4-dione represented by formula (5).

### Step e: Preparation of 5,6-epoxy-4-hydroxy-3-salicyloylamino-2-cyclohexenone (1a, DHM2EQ)

5,6-Epoxy-2-salicyloyalamino-2-cyclohexen-1,4-dione represented by formula (5) is suspended in a solvent (methanol, ethanol, THF, etc.) and a reducing agent (sodium borohydride, etc.) is added thereto at -78°C to 50° C, preferably under ice cooling. A solvent (ethyl acetate, methylene chloride, etc.) is added to the reaction mixture, which is sequentially washed with hydrochloric acid and water. After drying, the solvent layer is concentrated under reduced pressure, suspended, stirred and washed with methanol to obtain 5,6-epoxy-4-hydroxy-3-salicyloylamino-2-cyclohexenone (DHM2EQ) represented by formula (1a).

### Step f: Preparation of 3,3-dialkoxy-4,5-epoxy-6-hydroxy-2-salicyloylamino-cyclohexene

5,6-Epxoy-4,4-dialkoxy-3-salicyloylamino-2-cyclohexeno ne compound represented by formula (4) is dissolved in a mixed solution of a solvent such as methanol and sodium hydrogen carbonate solution, a reducing agent (sodium borohydride, etc.) is added at -78° C to 50°C, preferably under ice cooling, and the mixture is reacted while stirring. A solvent (ethyl acetate, etc.) is added to the reaction mixture, which is sequentially washed with hydrochloric acid and water. After drying, the solvent layer is concentrated under reduced pressure, dried under vacuum and purified by column chromatography to obtain 3,3-dialkoxy-4,5-epoxy-6-hydroxy-2-salicyloylamide-cyclohex ene represented by formula (6)

### Step g: Preparation of 5,6-epoxy-4-hydroxy-2-salicyloylamino-2-cyclohexenone (1b, DHM3EQ)

3,3-Dialkoxy-4,5-epoxy-6-hydroxy-2-salicyloylamino-cy clohexene represented by formula (6) is dissolved in a solvent (acetone, etc), p-toluenesulfonic acid is added to the solution, which is then reacted at room temperature while stirring. A solvent (ethyl acetate, etc.) is added to the reaction mixture, which is washed with water. The solvent layer is dried, concentrated under reduced pressure and purified to obtain 5,6-epoxy-4-hydroxy-2-salicyloylamino-2-cyclohexenone (DHM3EQ) represented by formula (1b).

The compounds represented by the general formula (1) are weak acidic substances, and salts thereof include organic bases such as quaternary ammonium salts, or salts with various metals--with alkali metals such as sodium, also available in the form of salts thereof. These salts can be produced by known methods.

### === Usage forms of the compounds according to the present invention ===

The compounds presented by the general formula (1) or pharmacologically acceptable salts thereof may be used alone or in combination with other drugs (e.g., other anticancer agents and hormone therapy agents).

When administered to humans, the compounds represented by the general formula (1) or a pharmacologically acceptable salt thereof may be administered orally or intravenously. The dosage range in adults is, for example, 1 to 100 mg/kg bw daily, preferably 4 to 12 mg/kg bw, either as a single dose or divided into multiple doses. However, the amount and number of doses can be suitably changed depending on the type of disease, symptoms, age, body weight, duration of therapy, therapeutic effects, dosage regimen, etc.

The compounds presented by the general formula (1) or a pharmacologically acceptable salts thereof may be administered orally in preparations, such as emulsions, tablets, capsules, granule, powder, syrups, etc. by blending with pharmacologically accepted carriers. Alternatively, they may be administered parenterally in such a way that they are injected subcutaneously, intramuscularly, intraperitoneally, or intravenously in preparations such as injectable formulations; injected intrarectally in preparation such as suppositories; or sprayed into the oral cavity or respiratory tract membrane in forms such as sprays or applied/attached to the affected parts (e.g., the skin or membrane) in preparations such as ointments or tapes.

Liquid preparations such as emulsions or syrups can be produced using the following as additive: water; saccharides such as sucrose, sorbitol, and fruit sugar; glycols such as polyethylene glycol and propylene glycol; oils such as sesame oil, olive oil, and soybean oil; preservatives such as p-hydroxy benzoate ester; flavors such as a strawberry flavor and peppermint; etc. Capsules, tablets, powders, granules, etc. can be produced using as additives excipients such as milk sugar, grape sugar, sucrose, and mannitol; disintegrators such as starch and sodium arginine; lubricants such as magnesium stearate and talc; binders such as a polyvinyl alcohol, hydroxypropylcellulose and gelatin; detergents such as fatty acid ester; plasticizers such as glycerin; etc.

Injectable formations can be produced by using, for example, a salt solution, a grape sugar solution, or a mixture thereof as a carrier. Suppositories can be produced by using, for example, cacao oil, hydrogenation fat, or carboxylic acid as a carrier. Sprays can be produced by using milk sugar, glycerin, etc. as a carrier that disperses active ingredients as fine particles to facilitate their absorption without stimulating a recipient's oral cavity or respiratory tract membrane, and can be formulated into aerosol, dry powders, etc.

As pharmacologically acceptable carriers, one or more kinds of various conventional organic or inorganic carrier substances may be used as materials for preparation, and formulated. Illustratively, such substances include, water, pharmacologically acceptable organic solvents, collagen, polyvinyl alcohol, polyvinylpyrrolidone, carboxyvinyl polymer, sodium arginine, water-soluble dextran, carboxymethyl starch sodium, pectin, xanthane gum, gum arabic, casein, gelatin, agar, glycerin, propylene glycol, polyethylene glycol, vaseline, paraffin, stearyl alcohol, stearic acid, human serum albumin, mannitol, sorbitol, lactose, etc. As additives used in formulation, for example, an excipient in a solid preparation, a lubricant, a binder, a disintegrator, a solvent in a liquid preparation, a solubilizing agent, a suspending agent, a tonicity adjusting agent, a buffer, a soothing agent, etc. may be employed. In addition, additives for formulation such as a preservative, an antioxidant, a colorant, a sweetening agent, a filler, an extender, a humidifying agent, a surfactant, a stabilizing agent, a germicide, a chelating agent, pH adjustor, and a detergent can also be used, as necessary. These additives are appropriately selected according to the administration unit form etc. of preparations. Of these additives, components used for usual preparations such as a stabilizing agent, a germicide, a buffer, a tonicity adjusting agent, a chelating agent, pH adjustor, a detergent, etc. are preferably selected.

Additives are illustratively listed below.

Stabilizing agents: human serum albumin; L-amino acids such as glycine, cystine, and glutamic acid; saccharides such as monosaccharides (e.g., glucose, mannose, galactose, and fruit sugar), sugar alcohols (e.g., mannitol, inositol, and xylitol), disaccharides (e.g., sucrose, maltose, and milk sugar), and polysaccharides (e.g., dextran, hydroxypropyl starch, chondroitin sulfuric acid, and hyaluronic acid), together with derivatives thereof; cellulose derivatives such as methylcellulose, ethyl cellulose, hydroxyethyl cellulose, hydroxypropyl cellulose, hydroxypropyl methylcellulose, and carboxymethylcellulose sodium etc.

Detergents: polyoxyethyleneglycol sorbitan alkyl ester and detergents based on polyoxyethylene alkyl ether, sorbitan monoacyl ester, fatty acid glyceride, etc.

Buffers: boric acid, phosphoric acid, acetic acid, citric acid, ε-aminocaproic acid, glutamic acid, and salts thereof (e.g., alkali metal salts such as sodium salt, potassium salt, calcium salt, and magnesium salt; and alkaline earth metal salts,)

Tonicity adjusting agents: sodium chloride, potassium chloride, saccharides, glycerin, etc.

Chelating agents: edetate sodium, citric acid, etc.

The contents of a compound represented by the general formula (1) or a pharmacologically acceptable salt thereof (active ingredient) in a preparation can vary between 1 to 90% by weight. For example, when the preparation is in the form of a tablet, a capsule, a granule, a powder, etc. the content of an active ingredient is preferably 5 to 80% by weight. In the case of a liquid preparation such as syrup, the content of an active ingredient is preferably 1 to 30% by weight.
In addition, in the case of an injectable preparation the content of an active ingredient is preferably 1 to 10% by weight.

The compounds represented by the general formula (1) or pharmacologically acceptable salts thereof are formulated by known methods using the following: excipients (saccharides such as milk sugar, sucrose, grape sugar, and mannitol; starches such as potato, wheat, and corn; inorganic substances such as calcium carbonate, calcium sulfate, and sodium bicarbonate; crystalline cellulose; etc.), binders (starch-paste liquid, gum arabic, gelatin, sodium arginine, methylcellulose, ethyl cellulose, polyvinylpyrrolidone, polyvinyl alcohol, hydroxypropylcellulose, carmellose, etc.), lubricants (magnesium stearate, talc, hydrogenerated vegetable oil, macrogol, and silicone oil), disintegrators (starch, agar, gelatin powder, crystalline cellulose, carboxymethylcellulose sodium, carboxymethylcellulose sodium calcium, calcium carbonate, sodium bicarbonate, sodium arginine, etc.), correctives (milk sugar, sucrose, grape sugar, mannitol, fragrant essential oils, etc.), solvents (water for injection, sterile purified water, sesame oil, soybean oil, corn oil, olive oil, cottonseed oil, etc.), stabilizers (inert gases such as nitrogen and carbon dioxide; chelating agents such as EDTA and thioglycolic acid; reducing substances such as sodium bisulfite, sodium thiosulfate, L-ascorbic acid, and rongalite; etc.), preservatives (paraoxybenzoic acid, chlorobutanol, benzyl alcohol, phenol, benzalkonium chloride, etc.),detergents (hydrogenated castor oil, polysorbates 80 and 20, etc.), buffers (sodium salts of citric acid, acetic acid, and phosphoric acid; boric acid; etc.), diluents, etc.

The Examples according to the present invention are hereinafter described in detail. Unless otherwise explained, methods described in standard sets of protocols such as J.Sambrook and E.F.Fritsch & T.Maniatis (Ed.),"Molecular Cloning, a Laboratory Manual (3rd edition), Cold Spring HarborPress and Cold Spring Harbor, New York (2001); and F.M.Ausubel, R.Brent, R.E.Kingston, D.D.Moore, J.G.Seidman, J.A.Smith, and K.Struhl (Ed.),"Current Protocols in Molecular Biology," John Wiley & Sons Ltd., or alternatively, modified/changed methods from these are used. When using commercial reagent kits and measuring apparatus, unless otherwise explained, attached protocols to them are used.

The compound (DHMEQ) represented by the formula (1a) used in the following Examples was produced using the methods described in Examples 1-5 of WO01/12588 A1. In some cases, the compound represented by the formula (1a) is referred to as "DHM2EQ," and the compound represented by the formula (1b) "DHM3EQ" hereinbelow.

### EXAMPLE 1

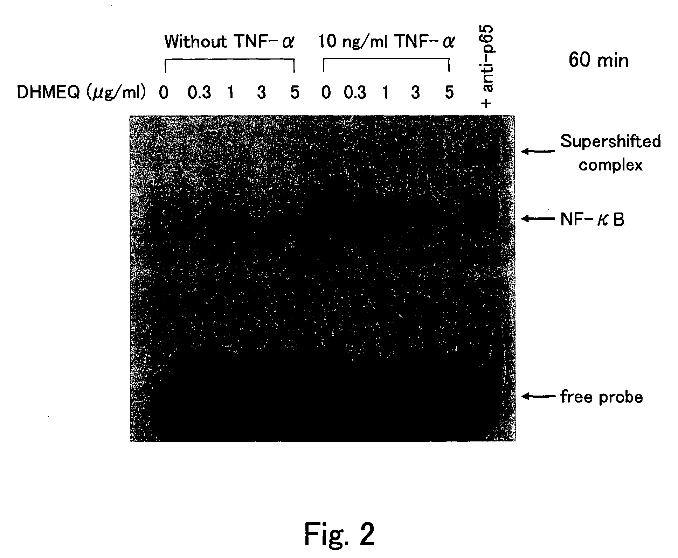

Activation of NF-κB was investigated by the gel shift assay, or electrophoretic mobility shift assay (EMSA). DHMEQ completely suppressed activation of NF-κB in HUVECs (prepared from the umbilical cords at Keio University Hospital) caused by TNF- α(Techne), IL-1β(PEPRO TECH EC LTD), and LPS (Sigma) at 3µg/ml. FIG. 2 shows the inhibitory effect of DHMEQ on activation of NF-κB when HUVECs are stimulated with TNF-α. The experimental method is as follows:
HUVECs were pretreated with DHMEQ as follows. That is, DHMEQ diluted with methanol was incubated for 2 hours in 5% CO₂ at 37°C in the culture medium in which HUVECs was being cultured after adding 1% volume of the liquid medium at the concentration indicated in the data.

Two-hour DHMEQ-pretreated HUVECs and untreated HUVECs were stimulated with 10ng(s)/ml TNF-k, 10ng/ml IL-1β, and 10 µg/ml LPS (i.e., after addition to each liquid medium, incubated in 5% CO₂ at 37°C for the period of time indicated in the data--e.g., 0, 5, 10, or 30 min) ; and cells were sampled. The collected cells were suspended in 400 µl of buffer A (10 mM HEPES (Sigma): pH 7.9, 1.5 mM DTT (Merck), 0.1 mM PMSF (Sigma)) and allowed to stand still for 15 min. Subsequently, the cells were centrifuged at 15,000 x g for 5 min and the supernatant removed. Another 400 µl of buffer A was added, followed by centrifugation at 15,000 x g for 5 min and removal of the supernatant. Next, 40µl of buffer C (20mM HEPES : pH7.9, 25% glycerol (Kanto Kagaku), 420 mM NaCl (Kanto Kagaku), 1.5 mM MgCl₂ (Kanto Kagaku), 0.2 mM DTT, 0.2 mM PMSF) was added. The cells were suspended by finger tapping and allowed to stand still for 20 min, followed by centrifugation at 15, 000 x g for 5 min and recovery of the supernatant. Nuclear extract was thus obtained. Four microliter of the following synthetic oligonucleotides (Promega), 2 µl of [γ-³²P]-ATP (Amersham), and 2 µl of 10X T4 PNK buffer (0.5 M Tris-HCl(Sigma): pH 7.6, 0.1 mM MgCl2, 50 mM DTT, 1 mM spermidine. HCl (Sigma), 1 mM EDTA (Kanto Kagaku) were added. Distilled water was added to a total volume of 18 µl. Further, 2µl of T4 polynucleotide kinase (Takara) (10 units/µl was added and reacted for 10 min at 37° C. Subsequently, the reaction was stopped by adding 80 µl of TE buffer (10 mM Tris-HCl: pH 7.5, 1 mM EDTA). The reaction mixture was purified on a nick column (Amersham) to obtain 32p-labeled NF-κB probes

Synthetic oligonucleotides:
  5′-AGT TGA GGG GAC TTT CCC AGG C-3′ (SEQ ID NO: 1)
  3′-TCA ACT CCC CTG AAA GGG TCC G-5′ (SEQ ID NO: 2)

Next, 4 µl of 5 x binding buffer (375 mM NaCl, 75 mM Tris-HCl: pH 7.0, 7.5 mM EDTA, 7.5m MDTT, 37.5% glycerol, 1.5% NP-40 (Nacalai Tesque), 0.5 µg/ml BSA (Sigma)),1 µl of poly dI-dC (Amersham) prepared at a concentration of 1µg/µl, and 0.5 µg of the nuclear extract were added. Distilled water was added to this mixture to yield a total volume of 17 µl. The samples for a super-shift assay were further supplemented with 0.5 µg of anti-p65 antibody (Santa Cruz). To this, 3 µl of the 32p-labeled NF-κB probe was added, followed by incubation for 30 min at 25°C. Subsequently, the 20 µl sample was applied to wells of 4% polyacrylamide gel (6.7 mM acrylamide (30/2; Wako), 1.25 ml 10 x TBE buffer (0.9 M Tris-boric acid (Kanto Kagaku) : pH8.3, 20mM EDTA), 42 ml H2O, 500 µl ammonium peroxodisulfate (Kanto Kagaku), 50 µl TEMED (Kanto Kagaku)), through which a current had been passing for 1 hour in advance, and electrophoresed at 150 V. After the electrophoresis, the gel was transferred to a filter paper, dried with a gel dryer, which was exposed to a film.

### EXAMPLE 2

The influence of DHMEQ on expression of ICAM-1, VCAM-1, and E-selectin was investigated when TNF-α stimulation was applied, using the Western-blotting method. In treatment with TNF-α alone, expression of ICAM-1 and VCAM-1 reached a peak 9 hours after stimulation and expression of E-selectin reached a peak 6 hours after stimulation. In HUVECs pretreated with DHMEQ for 2 hours, however, expression of any of the three adhesion molecules was markedly suppressed. The experimental method was as follows:
Two-hour DHMEQ-pretreated HUVECs and untreated HUVECs were each stimulated with 10 ng/ml TNF-α and cells were sampled. To this, a lysis buffer (20 mM Tris-HCl: pH 8.0, 150mM NaCl, 2mM EDTA, 100mM NaF (Kanto Kagaku), 400 µM Na₃VO₄ (Kanto Kagaku), 1% NP-40, 1 µg/ml leupeptin (Microbial Research Chemistry Foundation), 1 µg/ml antipain (Microbial Research Chemistry Foundation), 1mM PMSF) was added, and the cells were solubilized for 30 min by stirring on ice every 5 min, followed by centrifugation at 15,000 x g for 10 min and recovery of the supernatant. The protein concentration of this supernatant was determined by a liquid of Coomassie Brilliant Blue (Bio-Rad), and concentration was adjusted. Subsequently, 3 x SDS loading buffer (150 mM Tris-HCl: pH 6.8, 30% glycerol, 3% SDS (Kanto Kagaku), 0.03 mg/ml bromophenol blue, 150µl/ml 2-mercaptoethanol (Kanto Kagaku)) was added at a volume equal to half the volume of the lysis buffer that had been added, followed by boiling for 5 min. Using this as a sample, electrophoresis was performed in a 12.5% polyacrylamide gel. After the electrophoresis, the proteins in the gel were transferred to a PVDF membrane (Amersham) and blocked with TBS buffer (20 mM Tris-HCl: pH7.6, 137 mM NaCl) containing 5% skim milk (Snow Brand). Subsequently, the proteins were reacted with the PVDF membrane using antibodies to ICAM-1 (Santa Cruz), VCAM-1 (Santa Cruz), and E-selectin (Santa Cruz), followed by reactions with secondary antibodies (the secondary antibodies to ICAM-1, VCAM-1, and E-selectin (Amersham and Santa Cruz)) suitable to each primary antibody. Further, color was developed by the ECL method, followed by exposure to a film.

### EXAMPLE 3

Leukocytes (prepared from the experimenters' blood), and HL-60 cells (purchased from a cell bank) were both stimulated with TNF-α. The number of cells adhered increased time-dependently after 3, 6, and 9 hours. However, treatment with DHMEQ at 3 µg/ml markedly suppressed the adhesion. DHMEQ at this concentration neither exhibited toxicity to HUVECs nor suppressed their proliferation. The experimental method was as follows:
HUVECs were plated 24 well plates and confluently cultured. The culture medium was composed of 9.8 g/l medium 199 (provided from Nissui), 1.8 g/l NaHCO₃ (Wako), 10ml/l 1M Hepes buffer (Sigma), 30mg/l EGGS (Becton Dickinson), 6 ml/l heparin sodium injection (Takeda Pharmaceutical), and 10% heat-inactivated FBS (JRH). Subsequently, the following experiments were conducted. Two-hour DHMEQ-pretreated HUVECs and untreated HUVECs were each stimulated with 10 ng/ml TNF-α and adhesion of leukocytes to HUVECs as well as that of the human acute promyelocytic leukemia cell line HL-60 cells to HUVECs 0, 3, 6, and 9 hours after stimulation were evaluated. In this experiment, mononuclear cells isolated by the specific gravity centrifugation method were used as leukocytes.

After stimulation with TNF-α, the wells were washed twice with HBSS+ (provided from Nissui) and the culture medium was changed to 500 µl of a fresh medium composed of 9.8 g/l medium 199 (provided from Nissui), 1.8 g/l NaHCO₃ (Wako), 10ml/l 1M Hepes buffer (Sigma), 30mg/l ECGS (Becton Dickinson), 6 ml/l heparin sodium injection (Takeda Pharmaceutical), and 10% heat-inactivated FBS (JRH). Leukocytes and HL-60 cells were placed in wells at 2 x 10⁵ cells/well and 7 x 10⁴ cells/well, respectively. The samples were incubated for 1 hour in 5% CO₂ at 37°C and then gently washed three times with HBSS+. That state was photographed and the number of cells adhered to HUVECs was counted.

In summary, this DHMEQ suppressed activation of NF-κB as well as adhesion of HUVECs to leukocytes and to leukemia cells without exhibiting toxicity or suppression of proliferation. Accordingly, it is concluded that DHMEQ can stand long-term experimental or clinical use and is therefore useful.

### EXAMPLE 4

### (INHIBITORY EFFECT OF DHM2EQ ON CONSTITUTIVE ACTIVATION OF NF-κ B IN ADULT T CELL LEUKEMIA/LYMPHOMA (ATL) CELLS)

### (1) Gel shift assay

It was examined by the following method whether or not transcription factors present in the nucleus of a cell bind to the region in a promoter that contains specific sequences.

The ATL cell lines (MT-1 and TL-Om1) and the control cell lines (K562 and Jurkat+TNF), 2 x 10⁶ cells each, were treated with 10µg/ml DHM2EQ for 12 hours, and nuclear extract was prepared from each cell line. Likewise, nuclear extract was prepared also from the cell lines not treated with DHM2EQ. MT-1 and TL-Om1 are the cell lines transformed with HTLV-1; NF-κB is activated in these cell lines. K562 (myelocytic leukemia cell line) is a cell line in which constitutive NF-κB is not confirmed (a negative control), and Jurkat+TNF is a µNF-κB consensus oligomers (Promega) were end-labeled with [γ-³²P]-ATP and polynucleotide kinase (PNK) to prepare ³²P-labeled NF-κB probes.

The equivalent of 2 µg of nuclear extract protein was mixed with NF-κB probes (the equivalent of 10,000 cpm) in a volume of 20 µl and reacted at room temperature for 30 min. The solution after the reaction was subjected to 7.5% polyacrylamide gel electrophoresis. The gel was then dried and exposed to an X-ray film.

The results are shown in FIG. 5. In the figure, in the left six lanes, "-" and "+" indicate the results of DHM2EQ untreatment and DHM2EQ treatment, respectively. "-" and "+" at the far right 2 lanes, the positive controls, indicate the results of competitive inhibition ("+" means the experiment in the presence of competitor molecules) with non-labeled probe, indicating that signals are specific to a NF-κB-binding sequences.

As indicated in FIG. 5, the signal of NF-κB in the ATL cell lines (MT-1 and TL-Om1) was almost lost by DHM2EQ treatment. On the other hand, activation of NF-κB was not observed in K562.

### (2) Reporter gene assay (also called Luciferase assay)

To examine the transcriptional activity of NF-κB, by using a luciferase construct (6 kB-Luc plasmid), containing six tandem copies of the NF-κB binding sequence, driven with an artificial promoter as a reporter, this plasmid DNA was transiently introduced into the ATL cell lines (MT-1 and TL-Om1) as well as into the control cell line (K562) (transfected on a scale of 2 x 10⁵ cells/transfection using DMRIE-C (Invitrogen)). After 12 hours, treatment with 5 µg/ml DHM2EQ was started. Cells were recovered after 48 hours and the transcriptional activity of NF-κB was evaluated as the enzyme activity of the luciferase. The cell lines not treated with DHM2EQ were also evaluated in the same manner. All experiments were performed three times to obtain average values as well as standard deviations.

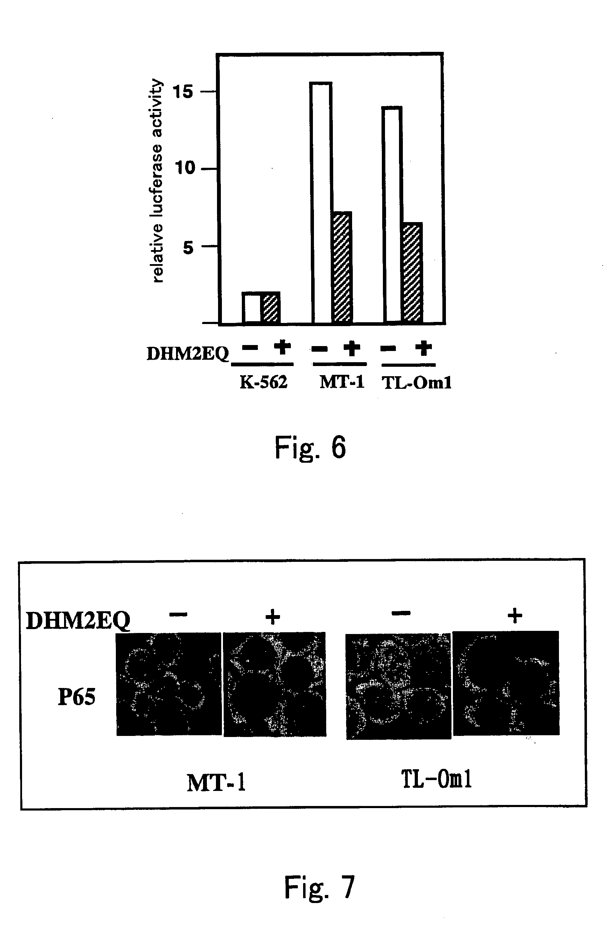

The results are shown in FIG. 6. In the figure,"-" and "+" indicate the results of DHM2EQ untreatment and DHM2EQ treatment, respectively. As indicated in FIG. 6, in the ATL cell lines (MT-1 and TL-Om1), luciferase activity was suppressed by DHM2EQ treatment to about 50%, indicating a suppressive effect of DHM2EQ on the NF-κB transcriptional activity in the ATL cell line. On the other hand, luciferase activity was not observed in the negative control cell line (K562).

### (3) Analysis with a confocal microscope

It is thought that DHM2EQ inhibits nuclear translocation of the p65 subunit of NF-κB. The ATL cell lines (MT-1 and TL-Om1) were treated with 10 µg/ml DHM2EQ for 24 hours and distribution of p65 was examined with a confocal microscope, using fluorescence-labeled anti-p65 antibody. The cell lines not treated with DHM2EQ were also examined in the same manner. The results are shown in FIG. 7. In the figure,"-" and "+" indicate the results of DHM2EQ untreatment and DHM2EQ treatment, respectively.

As indicated in FIG. in the ATL cell lines (MT-1 and TL-Om1), hollow nuclei were observed, indicating nuclear translocation of p65 has been inhibited by DHM2EQ treatment.

The results of the aforementioned (1) to (3) confirmed that DHM2EQ inhibits constitutive activation of NF-κB in ATL cells.

### EXAMPLE 5

### INHIBITORY EFFECT OF DHM2EQ ON PROLIFERATION OF ATL CELLS

### (1) Concentration dependence analysis of the proliferation-inhibitory effect of DHM2EQ

Cells from the ATL cell lines (MT-1 and TL-Om1) and a control cell line (K562) were plated in 96-well plates at 1 x 10⁵ cells/well and DHM2EQ was added at the desired final concentrations (2, 5, and 10 µg/ml). Cells to which the solvent DMSO had been added in equal volume (0 µg/ml) were used as <controls>. After incubation for 72 hours, cell viability was judged by the MTT assay. Relative MTT values were obtained as the ratio of the MMT values of DHM2EQ-treated cells to those of DHM2EQ-untreated cells, i.e., (MTT values of DHM2EQ-treated cells/MTT values of DHM2EQ-untreated cells) x 100(%).

The results are shown in FIG. 8. In the figure, the white square, black triangle, and black square indicate results of K562, TL-Om1, and MT-1, respectively.

As indicated in FIG. 8, in the ATL cell lines (MT-1 and TL-Om1), cell proliferation was inhibited in proportion to the DHM2EQ concentrations added, whereas, in a control cell line (K562), cell proliferation was hardly inhibited.

### (2) Time-course analysis of the proliferation-inhibitory effect of DHM2EQ

DHM2EQ was added to the ATL cell lines (MT-1 and TL-Om1) and a control cell line (K562) at a final concentration of 10 µg/ml and the cells were incubated for 12, 24, 48, and 72 hours. The proliferation-inhibitory effect was examined by the MTT assay. Cells to which the solvent DMSO had been added in equal volume were used as controls. Relative MTT values were obtained in the same manner as the aforementioned (1).

The results are shown in FIG. 9. In the figure, the white square, black triangle, and black square indicate results of K562, TL-Om1, and MT-1, respectively.

As indicated in FIG. 9, in the ATL cell lines (MT-1 and TL-Om1), cell proliferation was inhibited in proportion to the DHM2EQ treatment time, whereas, in a control cell line (K562), cell proliferation was hardly inhibited.

### (3) Inhibitory effect of DHM2EQ on proliferation of peripheral blood cells of ATL patients

Mononuclear cells were isolated from peripheral blood cells of ATL patients and ATL cells were isolated. DHM2EQ was added to the isolated ATL cells of three cases at a final concentration of 10 µg/ml and the cells were incubated for 24 hours. The proliferation-inhibitory effect was examined by the MTT assay. Using the cells to which solvent DMSO had been added in equal volume as controls, the proliferation-inhibitory effect was evaluated by the ratio of the MMT values of DHM2EQ-treated cells to those of DHM2EQ-untreated cells, i.e., (MTT values of DHM2EQ-treated cells/MTT values of DHM2EQ-untreated cells).

The results are shown in FIG.10.

As indicated in FIG. 10, it was confirmed that DHM2EQ exhibits an inhibitory effect on proliferation of ATL cells obtained from all patients examined.

### (4) Inhibitory effect of DHM2EQ on proliferation of normal peripheral blood mononuclear cells

Mononuclear cells were isolated from normal peripheral blood and DHM2EQ was added at final concentrations of 2, 5, and 10 µg/ml, followed by 72-hour incubation. The proliferation-inhibitory effect was examined by the MTT assay. Cells to which the solvent DMSO had been added in equal volume (0 µg/ml) were used as controls. The cell viability was represented as the ratio to the MTT value of the controls (DHM2EQ-untreated) by letting it = 100%.

The results are shown in FIG. 11.

As indicated in FIG. 11, DHM2EQ exhibited almost no inhibitory effect on proliferation of normal peripheral blood mononuclear cells.

The results of aforementioned (1) to (4) thus confirmed that DHM2EQ inhibits proliferation of ATL cells but not that of normal cells.

### EXAMPLE 6

### THE APOPTOSIS-INDUCING EFFECT OF DHM2EQ ON ATL CELLS

DHM2EQ was added to the ATL cell lines (MT-1 and TL-Om1) and a control cell line (K562) at a final concentration of 10 µg/ml, followed by incubation for 72 hours. Subsequently, apoptosis was examined by observing nuclear concentration or fragmentation on Hoechst staining with a fluorescence microscope. Cells to which the solvent DMSO had been added in equal volume were also examined in the same manner.

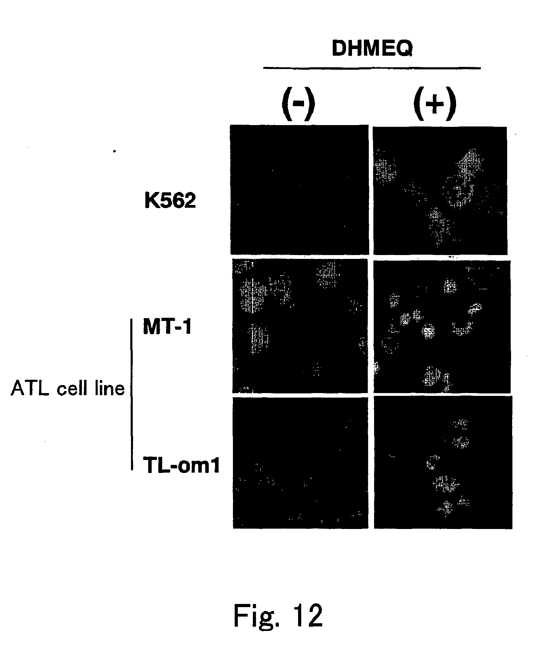

The results are shown in FIG. 12. In the figure,"-" and "+" indicate the results of DHM2EQ untreatment (DMSO only) and DHM2EQ treatment, respectively.

As indicated in FIG. 12, in the ATL cell lines (MT-1 and TL-Om1), apoptosis was induced by DHM2EQ treatment, and nuclear fragmentation was observed, whereas, in DHM2EQ-untreated cells and a control cell line (K562), apoptosis was not observed.

These results confirmed that DHM2EQ induces apoptosis of ATL cells but not that in normal cells.

### EXAMPLE 7

### INHIBITORY EFFECT OF DHM2EQ ON PROLIFERATION OF HODGKIN'S LYMPHOMA CELLS

Time-course analysis and concentration dependence analysis of the inhibitory effect of DHM2EQ on proliferation of Hodgkin's lymphoma cells were performed in the same manner as in Example 2. As Hodgkin's lymphoma cells, the Hodgkin's lymphoma cell lines KMH-2 and L-540 were used.

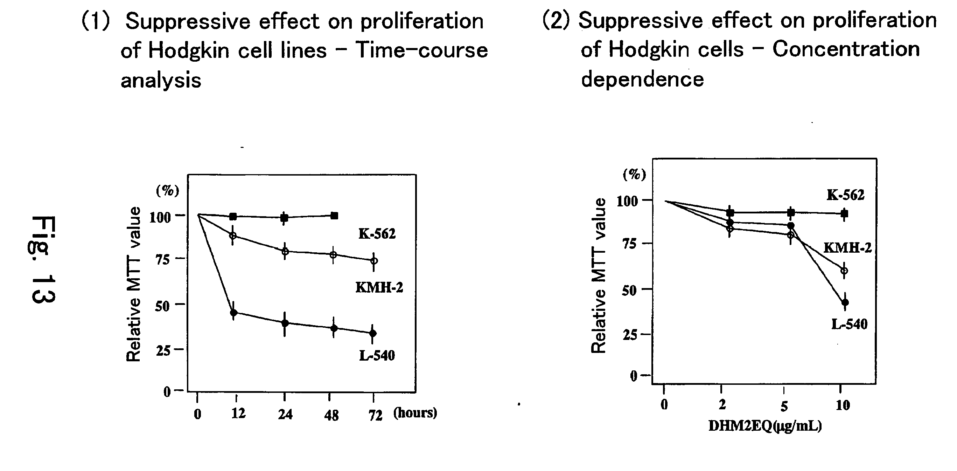

The results are shown in FIG. 13. In the figure, the black squares, white circles, and black circles show the result of K562, KMH-2, and L-540, respectively.

As indicated in FIG. 13, in the Hodgkin's lymphoma cell lines (KMH-2 and L-540), cell proliferation was inhibited in proportion to the DHM2EQ concentrations added, whereas in a control cell line (K562), cell proliferation was hardly inhibited. Further, in the Hodgkin's lymphoma cell lines (KMH-2 and L-540), cell proliferation was inhibited in proportion to the DHM2EQ treatment time, whereas in a control cell line (K562), cell proliferation was hardly inhibited even after time had elapsed.

These results confirmed that DHM2EQ inhibits proliferation of Hodgkin's lymphoma cells, in which NF-κB is constitutively activate, but not proliferation of control cells, in which NF-κB is not activated.

### EXAMPLE 8

### INHIBITORY EFFECT OF DHM2EQ ON PROLIFERATION OF MULTIPLE MYELOMA CELLS

Concentration dependence analysis of the inhibitory effect of DHM2EQ on proliferation of multiple myeloma cells was performed in the same manner as in Example 2. As multiple myeloma cells, the multiple myeloma cell line 196TIB was used.

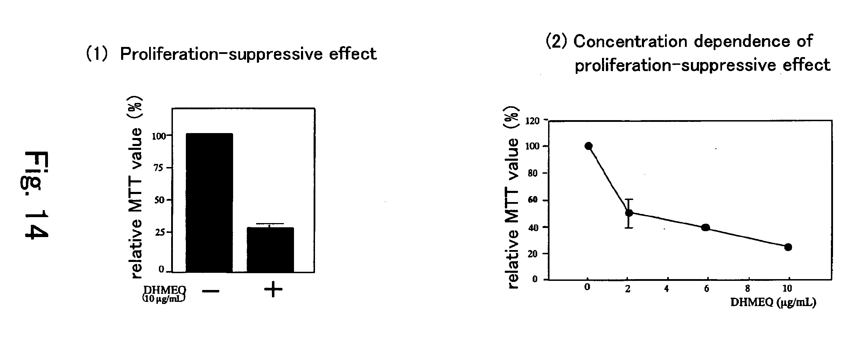

The results are shown in FIG. 14. In the figure,"-" and "+" indicate the results of DHM2EQ untreatment and DHM2EQ treatment, respectively.

As indicated in FIG. 14, in the multiple myeloma cell line (196TIB), cell proliferation was inhibited in proportion to the DHM2EQ concentrations added.

These results confirmed that DHM2EQ inhibits proliferation of multiple myeloma cells.

### EXAMPLE 9

### EFFECT IN WHICH DHMEQ INHIBITS BINDING BETWEEN NF-κB AND DNA IN MCF-7 CELLS

### 1. Methods

### 1-1 Preparation of a nuclear extract

MCF-7 cells (endowed by Professor Adrian L. Harriswere, Oxford University) were plated in portions of 4 ml at 1 x 10⁵ cells/ml in 60 ml dishes (2 dishes per condition). On the next day, the culture medium in the 60 mm dishes was adjusted to 2 ml, treated with DHMEQ prepared at 1, 3, and 10 µg/ml for 2 hours, and subsequently, with TNF-α at 20 ng/ml. The culture medium in 60 mm dishes was extracted with a sucker 30 min after TNF-α treatment, cells were washed twice with PBS- for external application, 1 ml of cold PBS- was added, the cells were scraped with a rubber policeman (twice), and transferred to a 15 ml centrifuge tube. Cells were collected from two 60 mm dishes into a 15 ml centrifuge tube, centrifuged at 1,000 rpm for 5 min, and the supernatant was removed. To this, 700 µl of cold PBS- was added. The cells were pipetted and then collected into 1.5 ml Eppendorf tubes (twice), followed by centrifugation at 3, 500 rpm for 5 min and removal of the supernatant. The following operations were performed in ice. The cells were suspended in 400 µl of buffer A (10 mM HEPES: pH 7. 9, 1.5 mM DTT, 0.2 mM PMSF), vortexed, and subsequently allowed to stand for 15 min, followed by centrifugation at 13,000 rpm for 5 min and removal of the supernatant. Once again, 400 µl of buffer A was added to each tube, followed by another centrifugation at 13,000 rpm for 5 min and removal of the supernatant. Next, collected nuclei were suspended in 40 µl buffer C (20 mM HEPES-KOH: pH7.9 or 25% glycerol, 420 mM NaCl, 1.5mM MgCl₂, 0.2 mM EDTA, 0.5 mM DTT, 0.2 mM PMSF) and allowed to stand for 20 min. Subsequently, the nuclei were centrifuged at 13,000 rpm for 5 min and the supernatants were recovered into Eppendorf tubes to obtain nuclear extract.

### 1-2 Preparation of probes

Four microliter of 1.75 pmol/µl oligonucleotide (Promega: Madison, MA), 2 µl of 10 x T4 PNK buffer (500 mM Tris-HCl: pH 8.0, 100 mM MgCl2, 50mM DTT), and 10 µl of distilled water were mixed. Two microliter of [γ-³²P]-ATP and another 2 µl of T4 PNK were added. This mixture was incubated for 10 min at 37°C, and subsequently the reaction was stopped by adding 80 µl of TE buffer (10 mM Tris-HCl: pH 8.0, 1mM EDTA).

Next, a Nick column was installed in the stand and a waste-liquid bottle was placed under the column. The top and bottom caps were removed to collect the TE buffer in the column into the bottle. About 3 ml of distilled water was added to the column by allowing it to run down the wall and recovered into the waste liquid bottle. Here, 100 µl of labeled DNA solution was placed in the column without allowing it to run down the wall. Under the column were prepared 1.5 ml Eppendorf tubes and 400 µl of distilled water was added to the column. The drops of the solution were recovered into the Eppendorf tubes (fraction 1). New Eppendorf tubes were prepared under the column, another 400 µl of distilled water was placed in the column, and drops of the solution were recovered into the Eppendorf tubes (fraction 2 = labeled oligonucleotide). When titration was finished, the column was capped. The column, fraction 1, and fraction 2 were measured individually with a Geiger counter and the readings confirmed fraction 2 > the column > fraction 1.

Purified labeled refined DNA probes used were diluted with distilled water at about 3 x 10⁴ cpm/µl.

The DNA probe sequence was as follows:

5′-ATGTGAGGGGACTTTCCCAGGC-3′ (SEQ ID NO: 3, J.Biol Chem. 277,
24625-24630, 2002)

### 1-3 Binding reaction and gel electrophoresis

Four milliliter of 5 x binding buffer (375 mM NaCl, 75 mM Tris-HCl: pH 7.0, 7.5 mM EDTA, 7.5mM DTT, 37.5% glycerol, 1.5% NP-40, 1.25 mg/ml BSA), 1 µl of 1 µg/ml poly dI-dC (Amersham Pharmacia Biotech, Inc), and 5 µg of nuclear extract protein were mixed, and water was added to a total volume of 17 µl. To this, 3 µl of DNA probe was added and mixed, followed by incubation for 20 min at 25°C. Subsequently, 20 µl of the reaction mixture was transferred to the well of 4% polyacrylamide gel, followed by elecrophoresis at 150 V in 0. 25 x TBE buffer. After the electrophoresis, the gel was dried and exposed to a film

### 2. Results

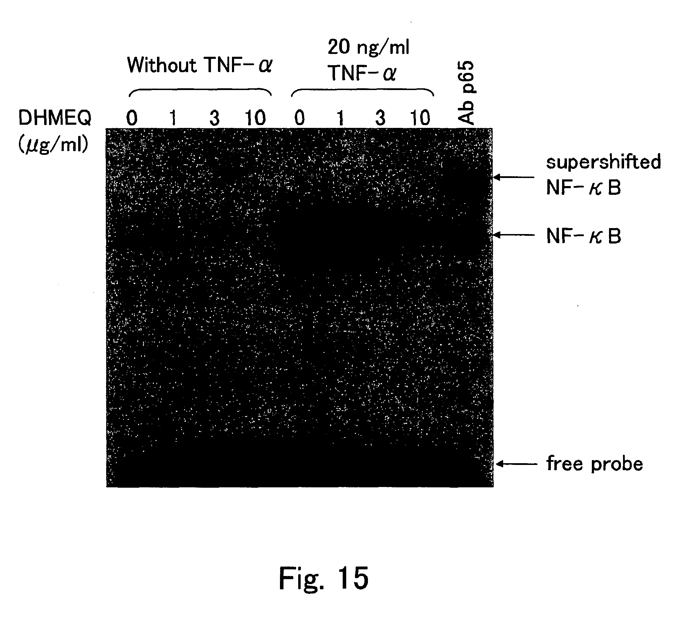

In the control cells, not treated with DHMEQ, a band indicating binding between NF-κB and DNA was observed 30 min after the TNF-α treatment. In the DHMEQ-treated cells, however, binding between NF-κB and DNA was concentration-dependently inhibited, and completely inhibited at the concentration of 10 µg/ml (FIG. 15).

It was therefore indicated that DHMEQ inhibits activation of NF-κB induced by TNF-α.

### EXAMPLE 10

### IN VITRO ANTITUMOR EFFECT OF DHMEQ ON HUMAN BREAST CANCER CELLS

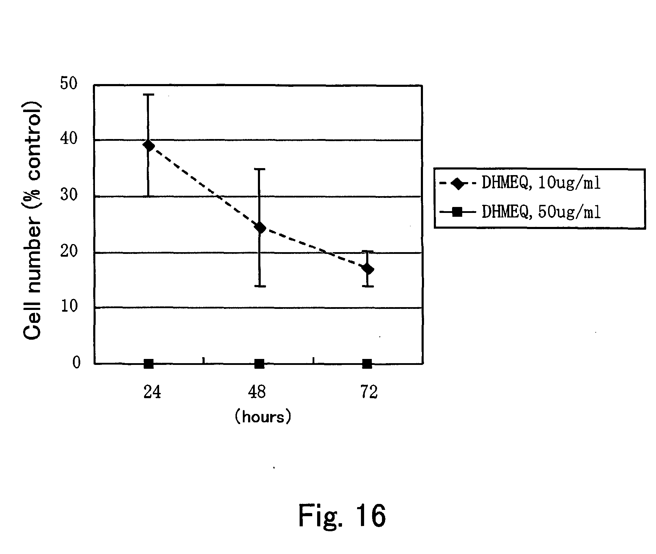

The human breast cancer cell line MCF-7 (NF-κB non-constitutively activated tumor) was incubated in 24, 48, and 72 hours in the presence of 10 and 50 µg/ml DHMEQ, and the proliferation-suppressive effect was examined. Compared with the control, at 10 µg/ml, 39%, 25%, and 17% of the cells survived in 24, 48, and 72 hours, respectively; and at 50 µ/ml no cells were observed to survive in and after 24 hours (FIG. 16). Cell proliferation was suppressed concentration- and time-dependently.

These experimental results indicate that although DHMEQ suppresses cancer cell proliferation time-dependently, its apoptosis-inducing effect is small even at comparatively high concentrations in short time, having a mechanism different from that of anticancer agents. This suggests that DHMEQ can be used as a drug with few side effects in the living body.

Details of the experimental method are as follows.

MCF-7 was plated in 6 well plates at 1 x 10⁵ /well. The next day, the culture media were changed into those (5ml) containing DHMEQ at 10 and 50 µg/ml and into the control medium (5ml) containing only DMSO, which is used for dissolution of DHMEQ, in equal amount to those containing each concentration ofDHMEQ. The assay was performed in three wells per each group. Immediately after the change, cells were incubated for 24, 48, and 72 hours. Subsequently, the cells were scraped with trypsin + EDTA. The number of cells was counted with trypan blue and averages as well as standard deviations were calculated. Data for each group were obtained and plotted in comparison with the control cells treated with DMSO only.

### EXAMPLE 11

### IN VIVO ANTITUMOR EFFECT OF DHMEQ ON HUMAN BREAST CANCER CELLS

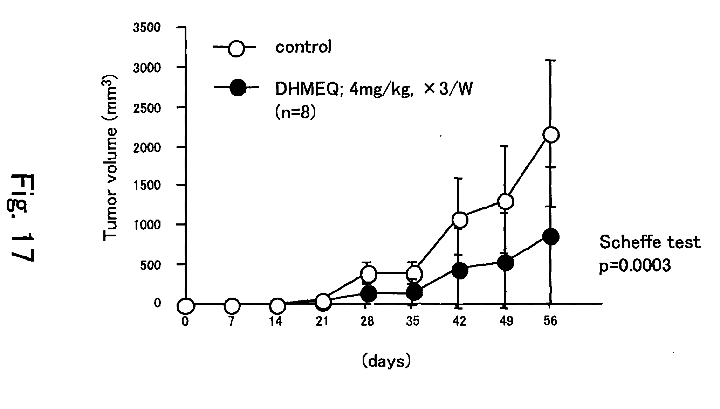

Subcutaneous tumor model SCID mice (CLEA Japan) were given DHMEQ (4 mg/kg) three days a week and the volume of the tumors were periodically measured for examination of the suppressive effect of DHMEQ on tumor growth. The body weights of the mice were also measured simultaneously. As a result, a significant suppressive effect on tumor growth was observed (FIG. 17). No mortality or weight loss was noted in the mice.

The details of the experimental methods were as follows.

MCF-7 (1 x 10⁶ cells) suspended in 100 µl of PBS was inoculated subcutaneously into the back of six-week-old SCID mice. DHMEQ was suspended in 0.5% methylcellulose (Nacalai Tesque) solution at 4 mg/kg in 200 µl. The drug-containing solution or 0.5% methylcellulose solution (200 µl) was intraperitoneally administered three times per week (n=8/group), starting on the next day after inoculation. The major axis and minor axis of the tumors were measured with calipers every seven days. The tumor volume was calculated as follows: (major axis) x (minor axis) ²/2. The body weights were also measured simultaneously.

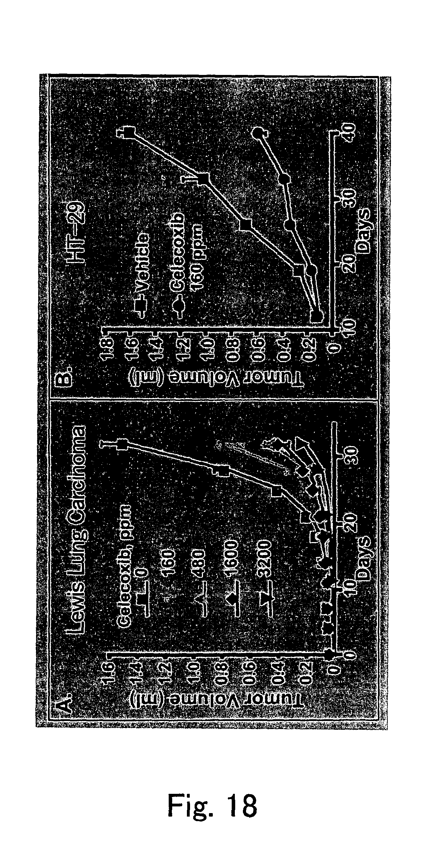

As far as antitumor agents are concerned, there have been numerous data on the suppressive effect on tumor growth in animal experiments with existing therapeutic agents for breast cancer. However, when comparing the efficacy of DHMEQ with another drug having a similar mechanism, the anti-inflammatory agent COX-2 inhibitors, whose efficacy on tumor growth suppression has been clarified in recent years, are appropriate. Efficacy of COX-2 inhibitors on MCF-7 has not yet been reported. Nevertheless, comparison with the suppressive effect on proliferation of other caner cells used as commonly as MCF-7 leads to the conclusion that the effect on MCF-7 is at least equal or greater. (The efficacy of the COX-2 inhibitor celecoxib (Pharmacia) on Lewis lung tumors and HT-29 is shown in FIG. 18.) The experimental methods used were as follows (Cancer Res., 60, 1306-1311, March 1, 2000): Lewis lung tumor (10⁶ cells) was inoculated into the posterior limbs of C57/B16 mice. The tumor volumes of the groups (n=20/group) fed diets containing 160, 480, 1600, and 3200 ppm celecoxib (offered by G.D.Searle/Monsanto Co.) each were measured twice a week with a plethysmometer, starting on the day of inoculation. The HT-29 human colon cancer cell line (10⁶ cells) was inoculated into the posterior limbs of nude mice. When the tumor volume reached 100 mm³, oral administration at 160 ppm was started and the tumor volume was measured once a week. The data were denoted as mean ± SD.

These results revealed that DHMEQ has a strong antitumor effect that does not depend on apoptosis, also suggesting that DHMEQ has a low side effect profile. It was therefore concluded that DHMEQ is effective not only in suppressing the primary lesion of breast cancer and cancer metastasis from the primary lesion to other tissues but also in suppressing metastasis of prognostic breast cancer or preventing breast cancer.

Currently, the only preventive agent used for breast cancer is the anti-estrogen tomoxifen (AstraZeneca), which is approved in North America but not in Japan, where its usefulness has not been confirmed. The mechanism of action of tomoxifen is to bind to the estrogen receptor, thereby competitively inhibiting binding of the female hormone estrogen. Accordingly, the preventive effect of tamoxifen on cancer development is exerted to hormone-sensitive breast cancer only; development of non-hormone sensitive breast cancer cannot be prevented. Moreover, tamoxifen has a side effect of causing endometrial cancer. No drugs other than hormone therapy agents have exhibited the inhibitory effect on breast cancer development. Having a different mechanism from that of conventional drugs that has strong cytotoxicity, DHMEQ is likely to be used for cancer prevention in the future. Further, inactivation of NF-κB by DHMEQ inhibits production of COX-2 as well, the central mediator of inflammation, like NF-κB, in the upstream of the cascade.

### Summary:

The drugs according to the present invention has an extremely high novelty from the clinical viewpoint in the following two points:
1) They have an extremely low toxicity, thereby having completely different advantages from those of anticancer agents, etc.
2) They are greatly different in spectrum from hormone therapy that targets hormone-sensitive cancers only.
   Further,
3) They are promising as drugs with a novel mechanism also from the viewpoint of chemoprophylaxis of breast cancer, which has recently become a focus of attention.

### EXAMPLE 12

### TOXICITY TEST

An acute toxicity test of DHMEQ was performed as follows: DHMEQ was dissolved in one drop of 10% DMSO saline + Tween, administered to the abdominal cavity of ICR mice, and the mortality/survival and status of the mice after 24 hours were investigated. As a result, mice receiving 0.156, 0.313, 0.625, 1.25, or 2.5 mg/mouse survived, and mice receiving 5 mg/mouse were dead on the day of administration. The anatomical findings on those dead on the day of administration revealed deposition of organs and small amount of ascites. As 5 mg/mouse is equivalent to about 250mg/kg, the acute toxicity LD 50 was calculated to be 187.5 mg/kg.

### EXAMPLE 13

To assay the effect of the compounds represented by the general formula (1) or pharmacologically acceptable salts thereof on cachexia, cachexia-induced BALB/c nude mice were used as model mice of human patients with cachexia. Here, cachexia symptoms were induced using the androgen-insensitive human prostatic cancer cell-line JCA-1.

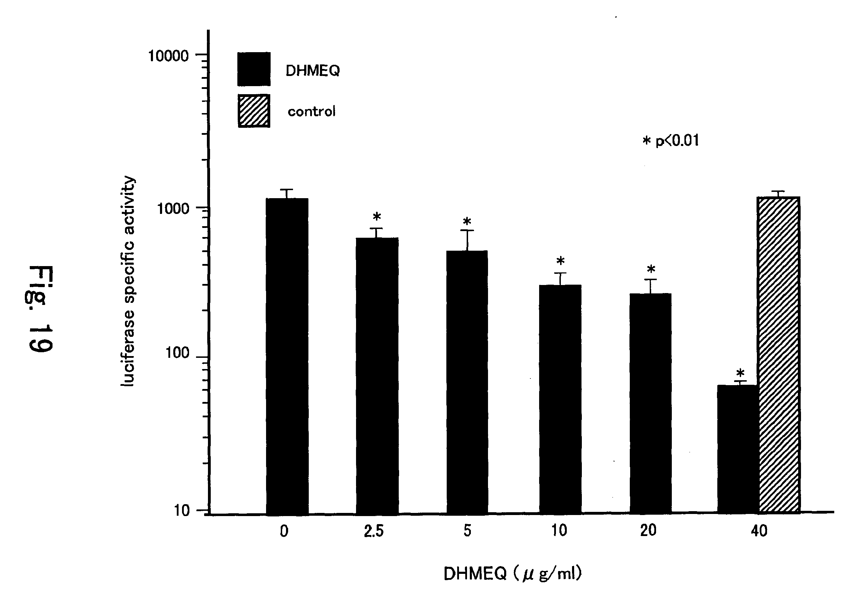

To measure the inhibitory effect of DHMEQ on activity of NF-κB, the activity of NF-κB on DHMEQ at various concentrations was measured using transcriptional activity of NF-κB as an index. As the reporter, the vector construct (p6kb-Luc), containing a luciferase gene as the reporter in the downstream of the promoter containing six tandem copies of the NF-κB binding sequence, was used. This reporter plasmid was transfected into JCA-1 cells using GenePOETER™ (Gene Therapy Systems). Fourteen hours after the transfection, 2.5, 5, 10, 20, and 40 µg/ml DHMEQ was added to the cell medium, followed by further incubation for 8 hours. Subsequently, cells were recovered and luciferase activity was measured. As controls, cells treated with nothing and those treated only with a solvent (DMSO in this case) that does not contain DHMEQ were isolated and simultaneously subjected to the experiment. Each luciferase activity was measured and the absolute value was presented by standardization. All experiments were conducted independently three times, producing averages and standard deviations. The results are shown in FIG. 19.

As indicated in FIG 19, the higher the concentration of DHMEQ administered, the more significantly great the inhibitory effect on intracellular activity of NF-κB, revealing that TDHMEQ concentration-dependently inhibits NF-κB activity in JCA-1 cells.

### EXAMPLE 14

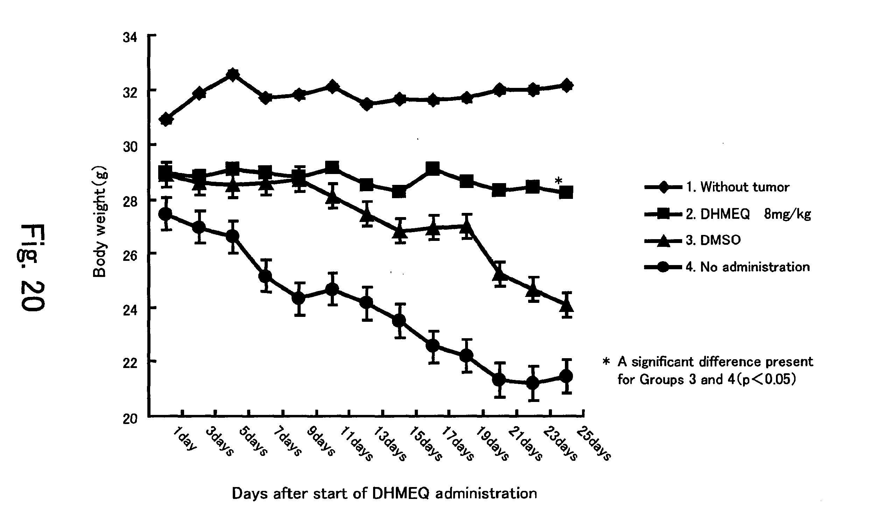

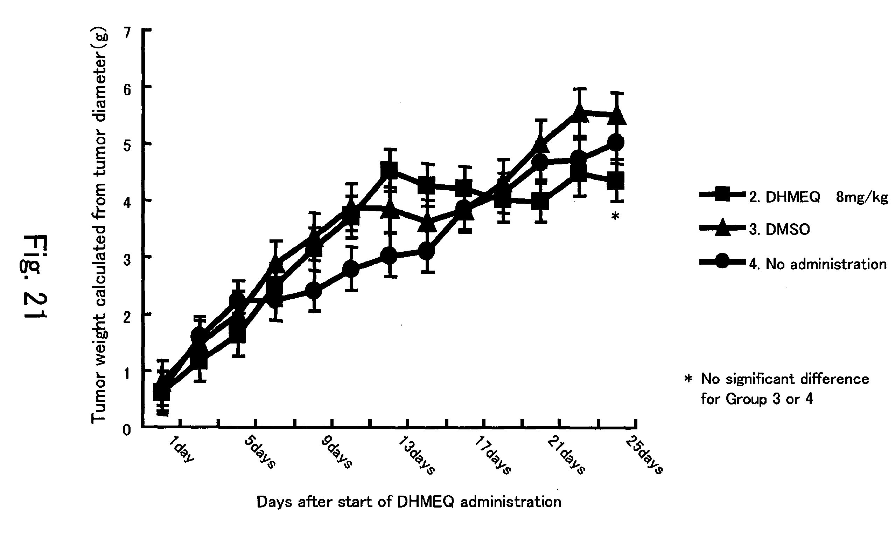

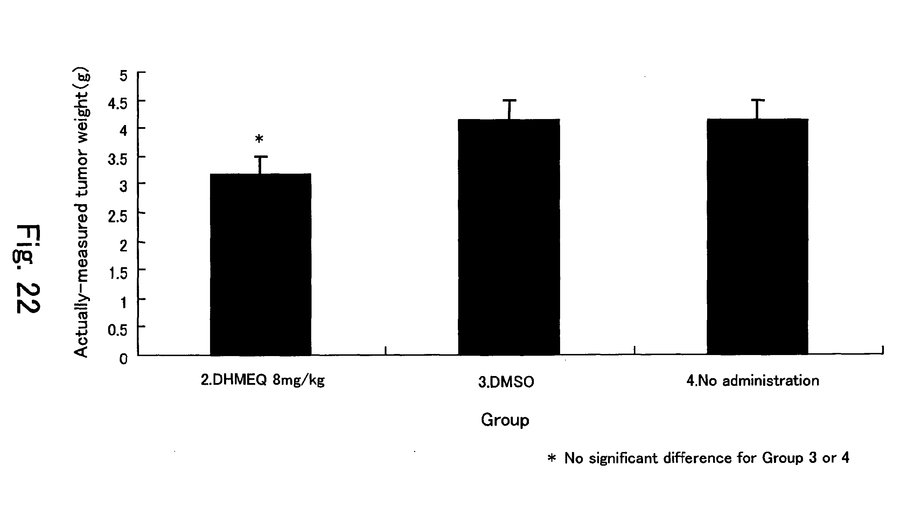

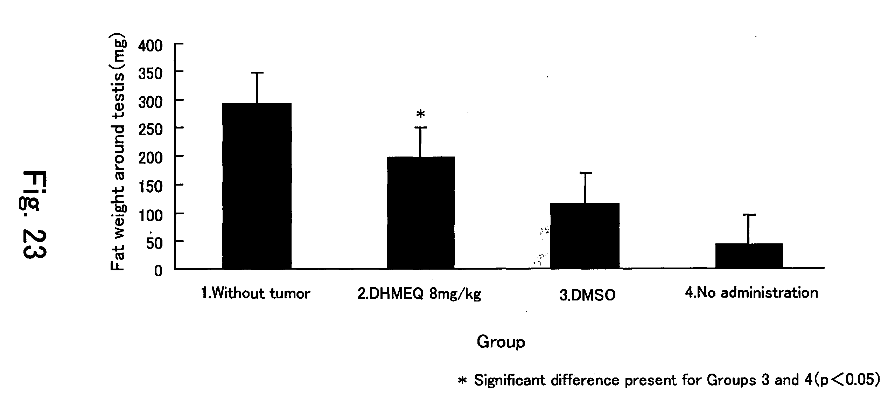

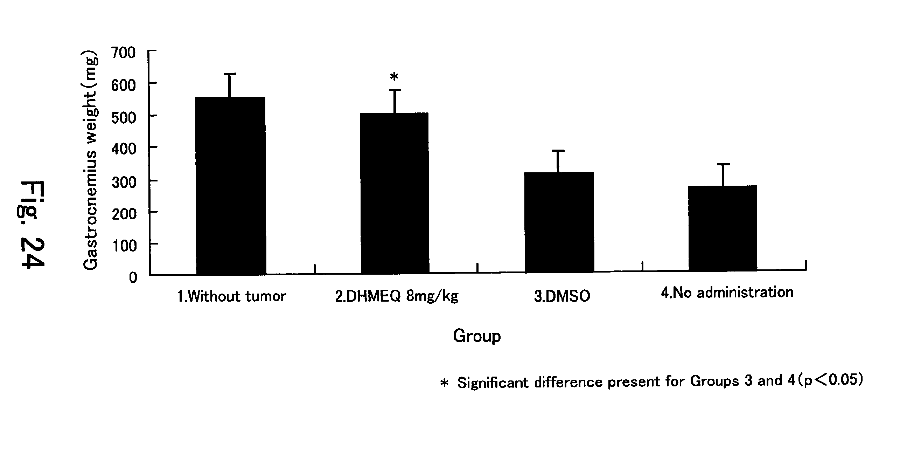

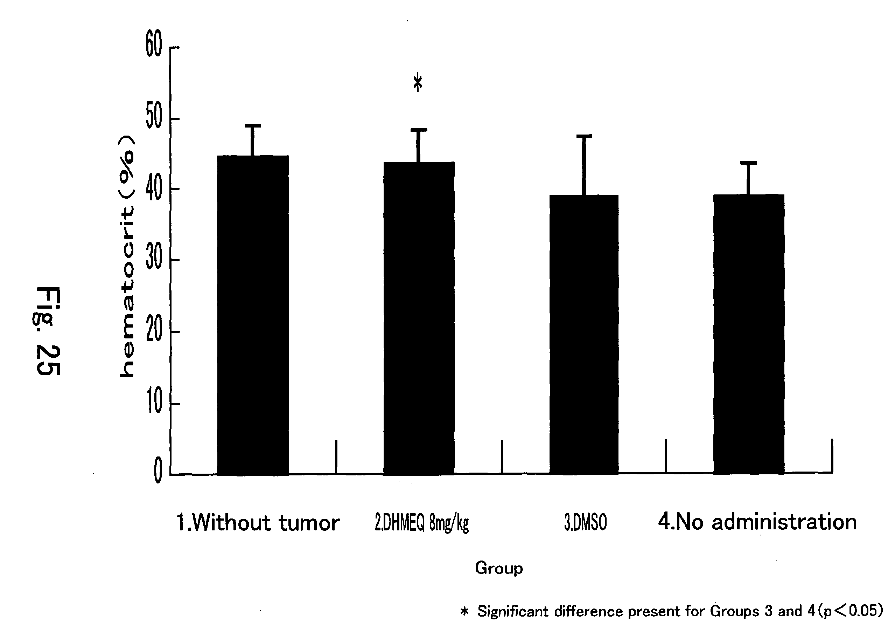

To measure the effect of DHMEQ on cachexia symptoms, the cachexia model mice were prepared using the aforementioned nude mice. JCA-1 cells (1 x 10⁷ cells) suspended in 100 µl of PBS were inoculated subcutaneously into the flank of six-old -week nude mice (hereafter called tumor-bearing mice). Fourteen days after the inoculation, when the tumors had reached palpable sizes, the inoculated mice were randomly assigned to three groups: group 2 (Gr2; n=13), given DHMEQ (8 mg/kg bw) every day; group 3 (Gr3; n=13), given DMSO every day; and group 4 (Gr4; n=11), given nothing. Group 1 (Gr1; n=14), consisting of normal nude mice not inoculated with JCA-1 cells (hereinafter called "normal mice"), was also included. From the next day of administration of DHMEQ, the body weights (FIG. 20) and the tumor weights (FIG. 21) calculated from the tumor diameters were measured every other day. On day 26 after the start of administration of DHMEQ, all mice were dissected and the tumor weights (FIG. 22), the weights of fat around the testis (FIG. 23) the gastrocnemius weights (FIG. 24), and the hematocrits (FIG. 25; the ratio of the volume of blood cell components to that of whole blood, which can be measured by blood centrifugation) were measured. The measured values were summed up for every group.

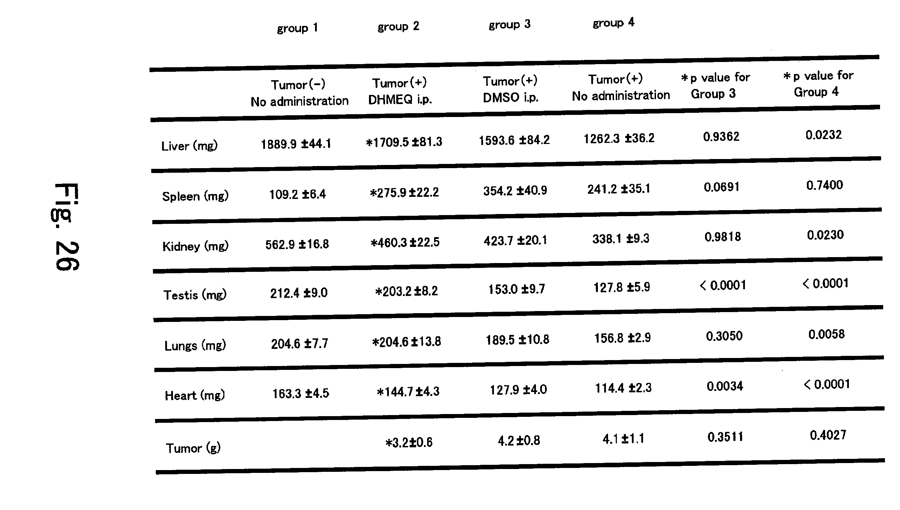

The experimental results are described below. First, in the 8 mg/kg bw DHMEQ-administered (Gr2), body weight reduction was significantly suppressed, as compared with the non-DHMEQ-administered groups (Gr3 and Gr4) (FIG. 20). However, no regressive effect on tumors was observed at the same DHMEQ concentration (FIGS. 21 and 22). In the measurement when the experiment was completed (day 26 after <the start of administration of DHMEQ>), as for the weights of fat around the testis (FIG. 23) and the gastrocnemius weights (FIG. 24), in the DHMEQ- administered group (Gr2), weight reduction was significantly suppressed , as compared with non-DHMEQ-administered groups (Gr3 and Gr4). As for the hematocrit, in the DHMEQ-administered group (Gr2) a significant tendency of recovery was found, as compared with non-DHMEQ-administered groups (Gr3 and Gr4). When the experiment was completed, the weights of each internal organ was measured and comparatively examined. No unfavorable influence of DHMEQ administration on each internal organ was observed (FIG. 26).

In conclusion, alleviation and suppression of cachexia symptoms were observed as a result of administering DHMEQ to the mice that had developed cancer and resulting cachexia symptoms by inoculation with JCA-1 cells, even though in the DHMEQ concentrations so as not to influence the sizes or weights of the cancer developed.

### EXAMPLE 15

### INHIBITORY EFFECT OF DHMEQ ON CONSTITUTIVE NF-κB ACTIVATION IN MULTIPLE MYELOMA (MM) CELL LINES

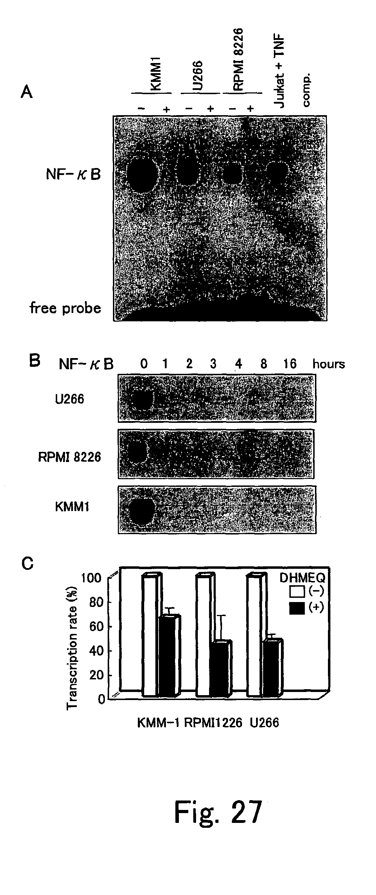

The MM cell lines, KMM1, RPMI8226, and U266 (2 x 10⁶ cells each) were treated with 10 µg/ml of DHMEQ for 12 hours, and nuclear extract was prepared from each cell line. The gel shift assay was performed in the same method as in Example 4 and the inhibitory effect of DHMEQ on NF-κB was examined. As a result, it was found that signals by NF-κB are lost by DHMEQ treatment in the MM cell lines (FIG. 27A). The TNF-treated Jurkat (Jurkat+TNF) was used as the positive control. "Comp" in the FIG. represents the result of a competitive inhibition experiment using an unlabeled probe, indicating that the signals are specific to the NF-κB binding sequence.

Next, the MM cell lines, KMM1, RPMI8226, and U266 were treated with 10 µg/ml DHMEQ for 12 hours, and nuclear extract was prepared from each cell line. The gel shift assay was performed in the same method as in Example 4 (1) and the time-course inhibitory effect on NF-κB was examined.
The results are shown in FIG. 27B. Accordingly, it was confirmed that, in 1 hour after the DHMEQ treatment, activation of NF-κB was almost inhibited and that the inhibition of NF-κ B activation was sustained even after a lapse of 16 hours.

Next, the inhibitory effect on activation of NF-κB was investigated using the reporter assay. First, 6 kB-Luc plasmid was introduced by transfection into the MM cell lines, KMM1, RPMI8226, and U266 (transfected on a scale of a 2 x 10⁵ cells/transfection using DMRIE-C (Invitrogen)) After 12 hours, 12-hour treatment of cells with 5 µg/ml DHM2EQ was started and the suppressive effect of DHMEQ on the transcription by NF-κB was examined. The results are shown in FIG. 27C. It was thus revealed that in the DHMEQ-treated MT-1 and TL-Om1, transfer by NF-κB is suppressed by about 50%, as compared with non-treated cells. The suppressive effect of DHMEQ on transcription by NF-κB was therefore indicated.

### EXAMPLE 16

### INHIBITORY EFFECT OF DHMEQ ON PROLIFERATION OF MULTIPLE MYELOMA (MM) CELL LINES

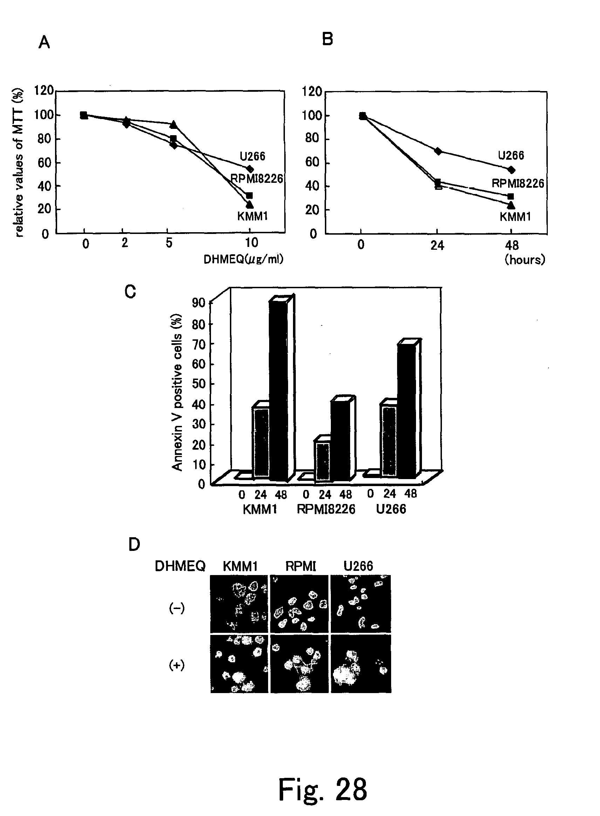

To investigate the influence of the DHMEQ concentrations on inhibition of cell proliferation, the cell viability was judged by using the MM cell lines, KMM1, RPMI8226, and U266 in the same method as in Example 5-(1). The results are shown in FIG. 28A. In the figure, the horizontal axis shows the DHMEQ concentrations and the vertical axis indicates the relative values of the MTT values of treated cells versus untreated cells, i.e., (DHMEQ-treated/untreated) x 100%. As indicated in FIG. 28A, in the MM cell lines, KMM1, RPMI8226, and U266, cell proliferation is suppressed in proportion to the DHMEQ concentrations.

Next, to investigate the time-course influence of DHMEQ on proliferation inhibition, the proliferation-inhibitory effect versus the passage of time was examined by using the MM cell lines, KMM1, RPMI8226, and U266 in the same method as in Example 5- (2). The results are shown in FIG. 28B. In the figure, the horizontal axis indicates the DHMEQ concentrations and the vertical axis indicates the relative values of the MTT values of treated cells versus untreated cells, i.e., (DHMEQ-treated/untreated) x 100%. As indicated in FIG. 28B, in the MM cell lines, KMM1, RPMI8226, and U266, cell proliferation is suppressed in proportion to the DHMEQ treatment time.

Further, to investigate apoptosis induction of MM cell lines by DHMEQ, the MM cell lines, KMM1, RPMI8226, and U266, were incubated for 0, 24, and 48 hours at a concentration of 10 µg/ml and the apoptosis was examined by Annexin-V staining. The results are shown in FIG. 28C. It was thus revealed that in the MM cell lines, KMM1, RPMI8226, and U266, DHMEQ had caused Annexin-V-positive cells to develop, in which apoptosis had been induced.

Further, to confirm apoptosis induction in MM cell lines by DHMEQ, the MM cell lines, KMM1, RPMI8226, and U266, were incubated for 72 hours at a concentration of 10 µg/ml and the apoptosis was examined by observing nuclear condensation or fragmentation with a fluorescence microscope, using Hoechst staining. The results are shown in FIG. 28D. In the MM cell strains, KMM1, RPMI8226, and U266, images of nuclear fragmentation by DHMEQ treatment were observed. In conclusion, it was revealed that DHMEQ induces apoptosis of ATL cells.

### EXAMPLE 17

### INHIBITORY EFFECT OF DHMEQ ON PROLIFERATION OF CELLS OF MULTIPLE MYELOMA (MM) PATIENTS

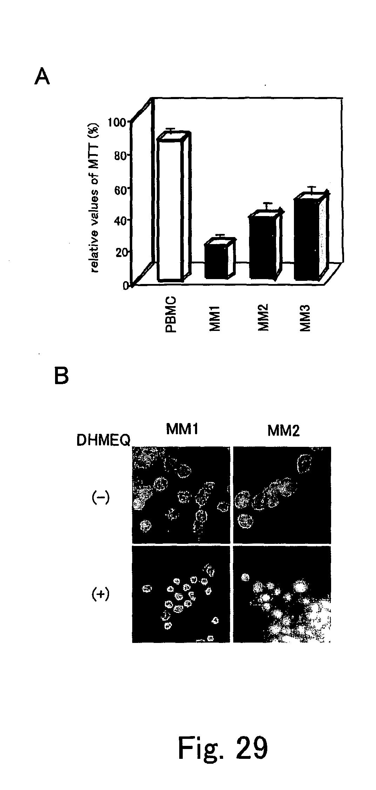

Next, to investigate the inhibitory effect of DHMEQ on proliferation of cells of multiple myeloma (MM) patients, MM cells (MM1, MM2, and MM3) were isolated from patients' bone marrow, incubated for 48 hours at the DHMEQ concentration of 10 µg/ml, and the proliferation-inhibitory effect was examined by the MTT assay. The results are shown in FIG. 29A. Peripheral blood mononuclear cells (PBMC) isolated from normal peripheral blood were used as control cells. In the figure, the vertical axis indicates the relative values of the MTT values of treated cells versus untreated cells, i.e., (DHMEQ-treated/untreated) x 100%. As indicated in FIG. 29A, in the MM cell from multiple myeloma (MM) patients, proliferation was suppressed in proportion to the DHMEQ concentrations, whereas almost no influence on the peripheral blood mononuclear cells was observed.

This revealed that although DHMEQ exhibits inhibition activity on MM cells of MM patients, it hardly acts on normal mononuclear cells, thereby capable of functioning as a pharmaceutical composition having a few side effects.

Further, to investigate apoptosis induction of cells of multiple myeloma (MM) patients by DHMEQ, cells of multiple myeloma (MM) patients were incubated for 72 hours at a concentration of 10 µg/ml and the apoptosis was examined by observing nuclear condensation or fragmentation with a fluorescence microscope, using Hoechst staining. The results are shown in FIG. 29B. As indicated in FIG. 29B, it was confirmed that in MM cells of multiple myeloma (MM) patients DHMEQ treatment induces apoptosis, leading to nuclear fragmentation.

### EXAMPLE 18

### INHIBITORY EFFECT OF DHMEQ ON CONSTITUTIVE NF-κB ACTIVATION IN HODGKIN'S LYMPHOMA (HL) CELL LINES

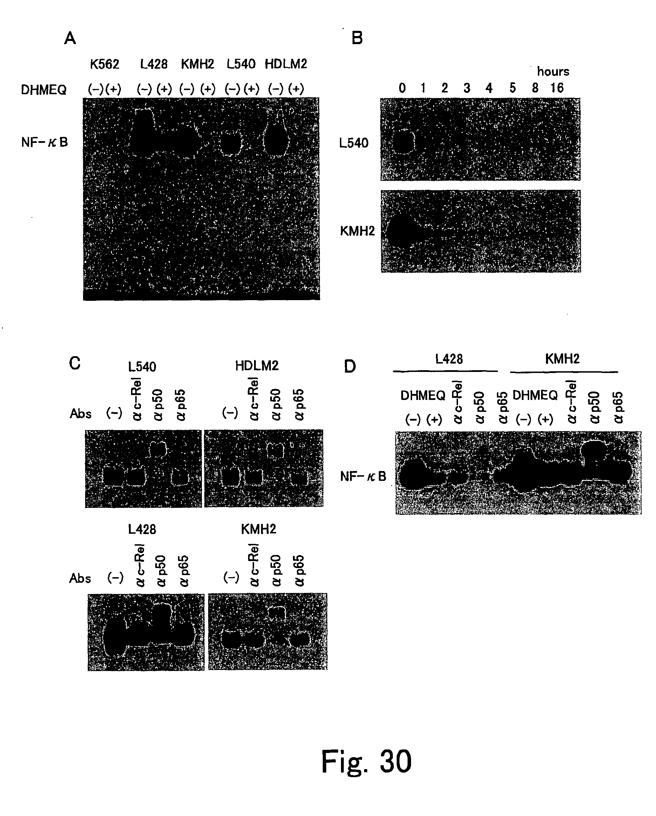

The HL cell lines, KMH2, L428, L540, and HDLM2, and the control cell line K562 (2 x 10⁶ cells each) were treated with 10 µg/ml DHMEQ for 12 hours, and nuclear extract was prepared from each cell line. The gel shift assay was performed in the same method as in Example 4 (1) and the inhibitory effect on NF-κB was examined. As a result, it was found that signals by NF-κB are almost lost by DHMEQ treatment in the HL cell lines (FIG. 30A). Cell line K562 (myelocytic leukemia cell line) is a cell line in which constitutive activation of NF-κB is not observed, and thus used as the negative control. On the other hand, the TNF-treated Jurkat (Jurkat+TNF) was used as the positive control. "Comp" in the figure represents the result of a competitive inhibition experiment using an unlabeled probe, indicating that the signals are specific to the NF-κB binding sequence.

Next, the HL cell lines KMH2 and L540 were treated with 10 µg/ml DHMEQ and nuclear extract was prepared from each cell line. The gel shift assay was performed in the same method as in Example 4 (1) and the time-course inhibitory effect on NF-κB was examined. As a result, it was confirmed that, in 1 hour after the treatment, activation of NF-κB was almost inhibited and that the inhibition of NF-κB activation was sustained even after a lapse of 16 hours (FIG.30B)

NF-κB is a complex consisting of the subunits, p50, p65, and c-Rel. Thus, the NF-κB subunits, constitutively activated in the HL cell lines, KMH2, L428, L-540, and HDLM2, were examined. A supershift assay was performed with antibodies against p50, p65, and c-Rel in the same method as described in Example 1. The results are shown in FIG. 30C. As indicated in FIG. 30C, a supershift was observed especially with p50, and p50 and was confirmed to be present in the complex in all the HL cell lines.

In L428 and KMH2, weak signals remain even after DHMEQ treatment. Thus, investigation was performed to determine whether these signals have NF-κB-specific subunits of DHMEQ resistance. Further, the HL cell lines, KMH2, L428L-540, and HDLM2 were treated with 5 µg/ml DHMEQ for 12 hours, and the remaining component of NF-κB was supershifted with antibodies against p50, p65, and c-Rel according to the same method as described in Example 1. The results are shown in FIG. 30D. As indicated in FIG. 30D, mainly in p50, the remaining component produced the same results as those produced in FIG. 30C, suggesting that there is no distinctive subunit exhibiting resistance to DHMEQ.

### EXAMPLE 19

### ENHANCING EFFECT OF DHMEQ ON THE EFFECTS OF ANTITUMOR AGENTS

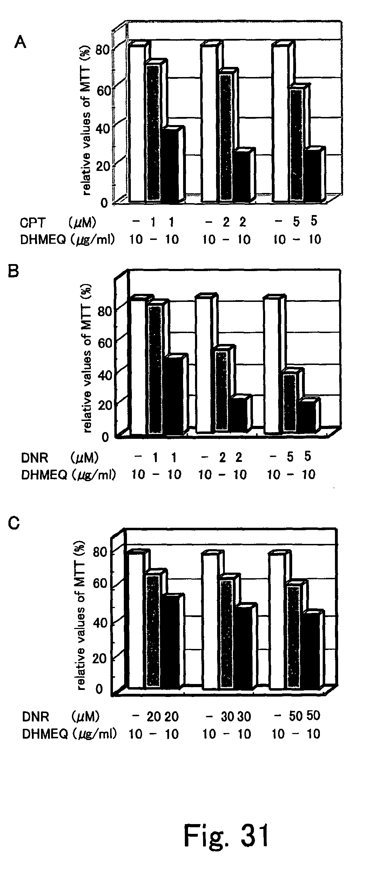

The enhancing effect of DHMEQ on the effect of antitumor agents was examined, using camptothecin (CPT), daunomycin (DNR), and etoposide (ETP) as antitumor agents. The effects of antitumor agents and the enhancing effect thereon were examined by the MTT assay method as described in Example 5(2).
The results are shown in FIG. 31. In this example, the cells used were KMH2. The horizontal axes in FIG. 31 indicate the concentrations of each antitumor agent the DHMEQ concentration. The concentrations of each anticancer agent were set at three levels. The examination was performed on DHMEQ alone, the antitumor agent alone, or both in combination, at each level. DHMEQ was used at a concentration of 10 µg/ml and the treatment time was 48 hours. The vertical axis indicates the relative values of the MTT values of treated cells versus untreated cells, i.e., (treated/untreated) x 100%. As the antitumor agent, camptothecin (CPT), daunomycin (DNR), and etoposide (ETP) were used in FIG31A, B, and C, respectively.
As a result, it was shown that DHMEQ enhances the effect of any antitumor agent used.

### EXAMPLE 20

### THE ENHANCING EFFECT OF DHMEQ ON THE EFFECTS OF ANTITUMOR AGENTS IS DUE TO TREATMENT OF EACH ANTITUMOR AGENT BY INHIBITION OF NF-κB ACTIVATION BY AN ANTITUMOR AGENT.

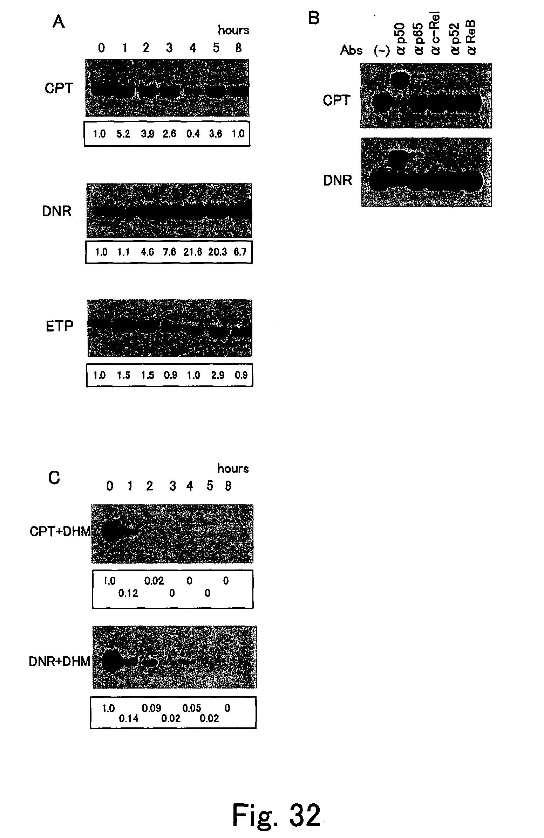

To investigate activation of NF-κB, the activation of NF-κB when tumor cells are treated with each antitumor agent (camptothecin (CPT), daunomycin (DNR), and etoposide (ETP)) was examined by the gel shift assay as described in Example 5(1). The results are shown in FIG. 32A. In this example, the cells used were KMH2. The lower row in each panel in the figure indicates the relative values obtained by quantifying the signals obtained and assuming the value before treatment to be 1. As indicated in FIG. 32A, it was demonstrated that, when treated with any antitumor agent, compared with the cells before treatment, the tumor cells had transiently produced 3 to 20 times the NF-κB activity

To identify the NF-κB subunit(s) induced by camptothecin (CPT) and daunomycin (DNR) in tumor cells, the supershift assay was performed with antibodies against p50, p65, and c-Rel according to the same method described in Example 1.
The results are shown in FIG. 32B. As indicated in FIG. 32B, it was revealed that in tumor cells treated by camptothecin (CPT) or daunomycin (DNR), activated NF- p65 subunit of NF-κB contains p50.

Next, to investigate inhibition of activation of NF-κB by an antitumor agents resulting from DHMEQ, NF-κB induction when treating tumor cells with each antitumor agent (camptothecin (CPT) or daunomycin (DNR)) in combination with DHMEQ was investigated by the gel shift assay as described in Examples 4(1), and the time-course inhibitory effect on NF-κ B was examined. The results are shown in FIG. 32C. In this example, the cells used were KMH2. The lower row in each panel in the figure indicates the relative values obtained by quantifying the signals obtained and assuming the value before treatment to be 1. As indicated in FIG. 32C, when treated with either antitumor agent, NF-κB induction was strongly suppressed by the concurrent use of DHMEQ.

### EXAMPLE 21

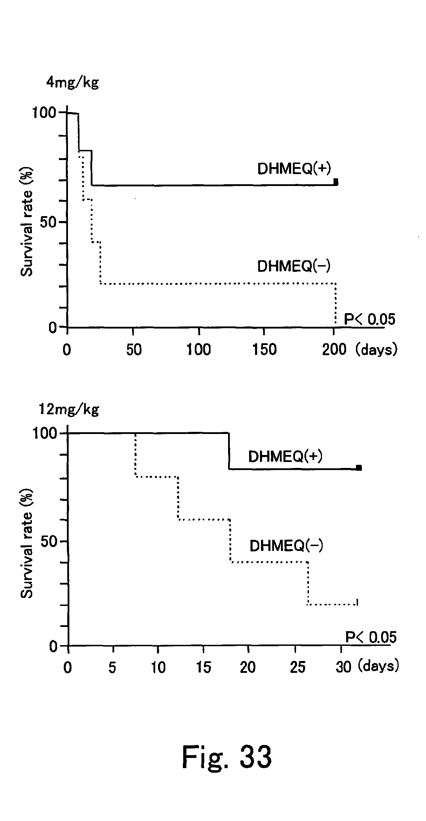

The in vivo effect of DHMEQ was investigated using SCID mice intraperitoneally inoculated with an ATL cell line. First, five-week-old male SCID mice (CB17-scid/scid; SLC Japan, Inc (Shizuoka, Japan)) were treated for 3 to 5 days with 1 mg of IL-2 receptor antibody (TM-β 1; J.Immunol. 147: 2222-2228, 1991), and intraperitoneally inoculated with the ATL cell line MT-2 (3 to 4 x 10⁷ cells). Subsequently, the mice were intraperitoneally given 4 mg/kg bw or 12 mg/kg bw DHMEQ dissolved in 5% carboxymethyl cellulose (CMC; Sigma) solution three times a week for one month, and their survival probability and status were observed. The control group was intraperitoneally given 0.5% CMC solution that did not contain DHMEQ three times a week for one month in the same manner. The results are shown in FIG. 33. The survival curve was calculated by the Kaplan-Mayer method, and the statistically significant difference was determined by the Cox-Mantel test. As indicated in FIG. 33, in the 4 mg/kg DHMEQ-administered group (DHMEQ (+)), 4 out of 6 mice survived about 200 days after the start of the experiment, whereas in the control group (DHMEQ (-)) all the 5 mice were dead, indicating a statistically significant difference (Cox-Mantel test; p<0.05). In the 12 mg/kg DHMEQ-administered group (DHMEQ (+)), 5 out of 6 mice survived about 30 days after the start of the experiment, whereas in the non DHMEQ-administered group (DHMEQ (-)) 4 out of 5 mice were dead, indicating a statistically significant difference (Cox-Mantel test; p<0.05). In the DHMEQ-administered group (DHMEQ (+)), anomalies were not noted in the body weights etc. of the mice and toxicity was not noted even when three volumes of 4 mg/kg DHMEQ3 was administered. These results revealed that DHMEQ can rescue deaths of individual mice resulting from transplanted ATL cells in vivo.

### EXAMPLE 22

### SYNERGISTIC EFFECT WHEN DHMEQ AND IRRADIATION ARE USED IN COMBINATION

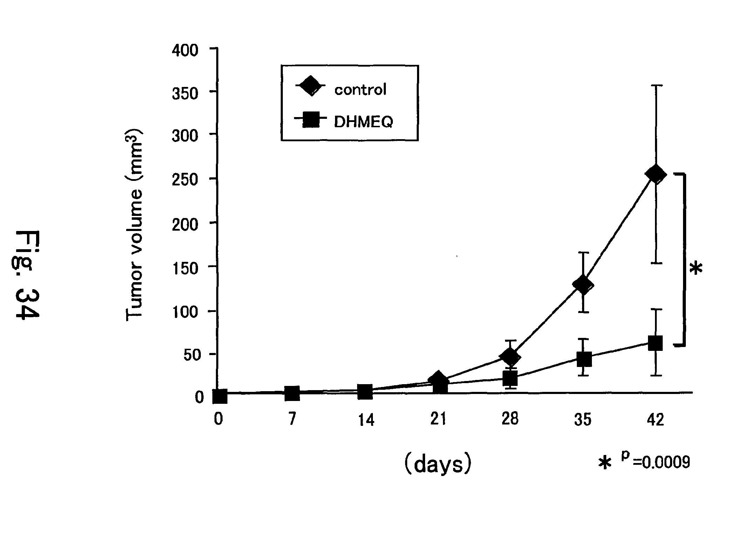

The in vivo suppressive effect of DHMEQ on proliferation of tumor cells in human pancreatic cancer. The human pancreatic cancer cell line PK-2 (2 x 10⁶ cells) was inoculated subcutaneously in the right back of SCID mice (n=7) and then 12 mg/kg DHMEQ was injected intraperitoneally (i. p.) at a dose of 200 µl every other day for five days. Subsequently, the major axis and minor axis of the tumors were measured every seven days. The tumor volume was calculated as follows: (major axis) x (minor axis)² x 0.5. The results are shown in FIG. 34.
As indicated FIG. 34, it was revealed that use of DHMEQ significantly suppresses proliferation of tumor cells.

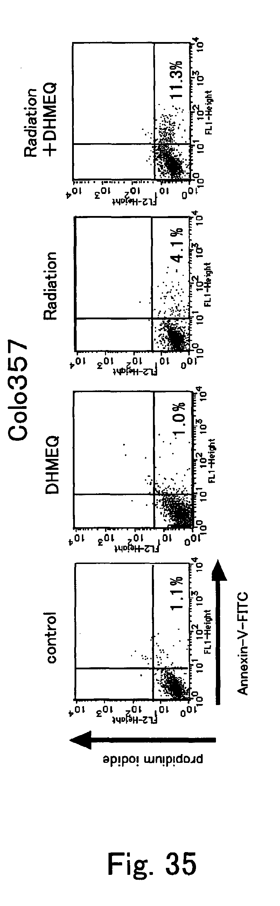

Next, the apoptosis-enhancing effect of DHMEQ on irradiated tumor cells was investigated. The human pancreatic cancer cell line Colo357 given 10 µg/ml DHMEQ for six hours, the human pancreatic cancer cell line Colo357 irradiated with 20 Gy radiation and then incubated for 6 hours, and the human pancreatic cancer cell line Colo357 given 10 µg/ml DHMEQ for six hours immediately after irradiation with 20 Gy radiation were subjected to Annexin-V/propidium iodide (PI) double staining. The apoptotic cells (lower right) were measured by flow cytometry and the ratio was calculated. The results are shown in FIG. 35. As indicated in FIG. 35, apoptosis was not induced in the human pancreatic cancer cell line Colo357 by DHMEQ alone. On the other hand, in the cells to which DHMEQ was administered after irradiation with 20 Gy radiation, the apoptosis-inducing ability is enhanced three-fold, as compared with the cells only irradiated with 20 Gy radiation

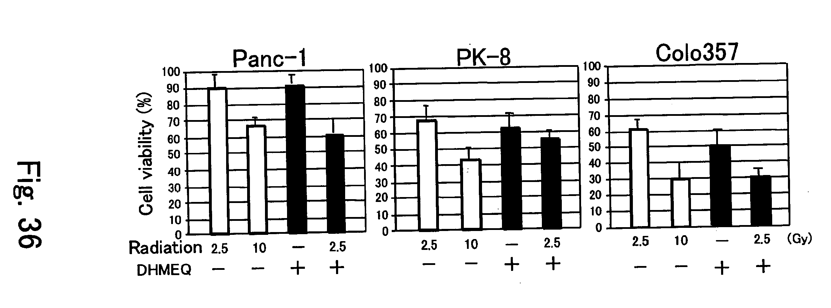

The *in vitro* suppressive effect of DHMEQ on proliferation of human pancreatic cancer cell lines when DHMEQ and irradiation are used in combination was investigated. The human pancreatic cancer cell lines, Panc-1, PK-8, and Colo357 were plated 10 cm dishes (5 x 10⁵ cells each). After two days, cells were irradiated with either 2.5 Gy or 10 Gy radiation, followed by administration of 10 µg/ml DHMEQ for 4 hours in the combinatin group. The number of cells was measured after 24 hours. Either the point of the completion of irradiation or the point of the start of the drug administration was assumed to be 0 hours. The results are shown in FIG. 36. DHMEQ exerted a sufficient suppressive effect on proliferation of the human pancreatic cancer cell lines, PK-8 and Colo357 only after 4-hour contact with the drug, and the effect was equal to that of a radiation of 2.5 Gy. Panc-1, which is radioresistant and on which DHMEQ alone is less effective, the combined use of irradiation and DHMEQ caused therapy resistance to each to disappear. In Colo357 and Panc-1, DHMEQ exhibited a synergistic suppressive effect on their proliferation when used in combination with irradiation, indicating that the irradiation effect of 2.5 Gy is enhanced to 10 Gy or equivalent (i.e., four-fold).

### INDUSTRIAL APPLICABILITY

The present invention can provide pharmaceutical compositions that are capable of improving symptoms accompanied by activation of NF-κB.

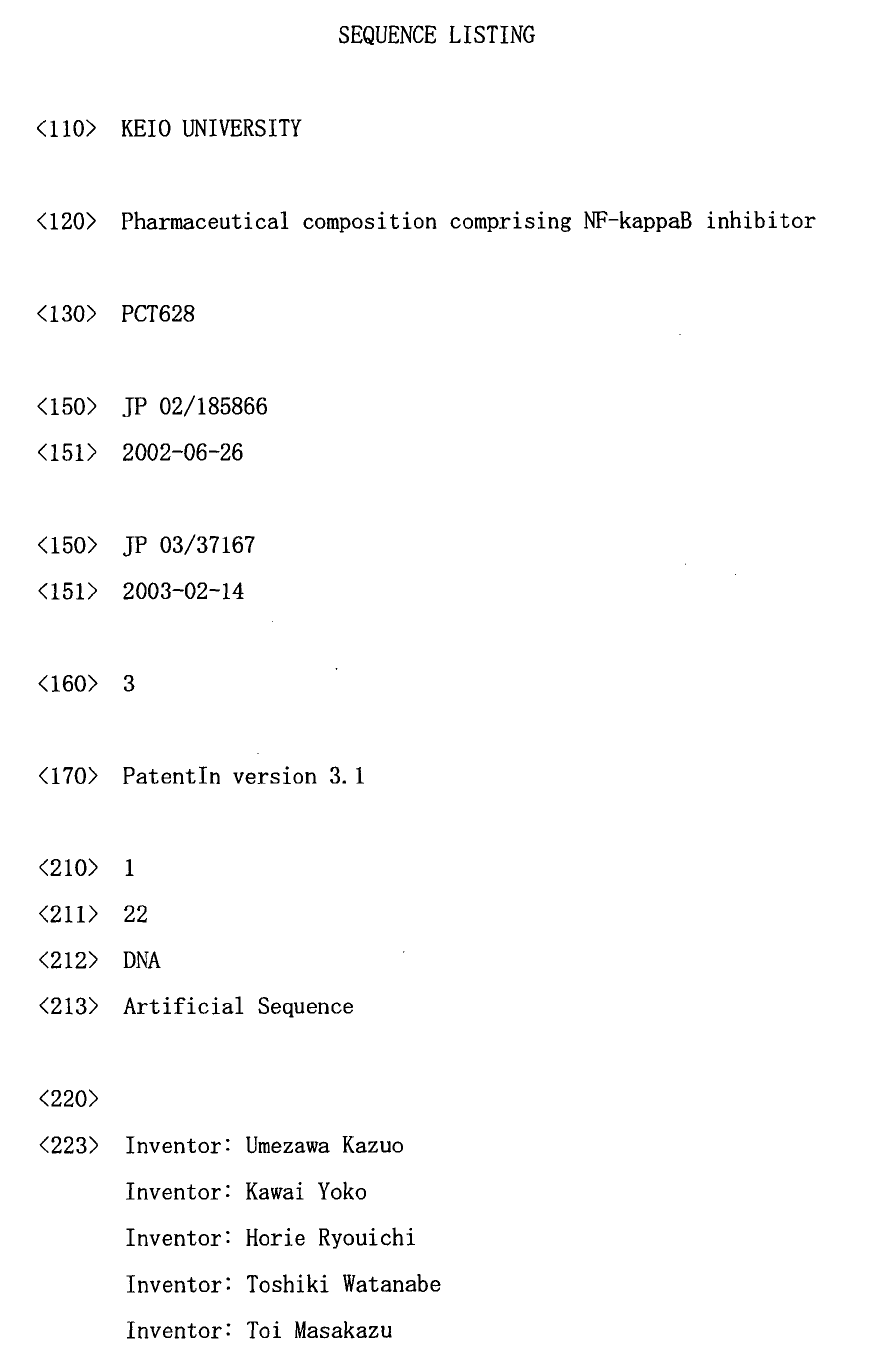

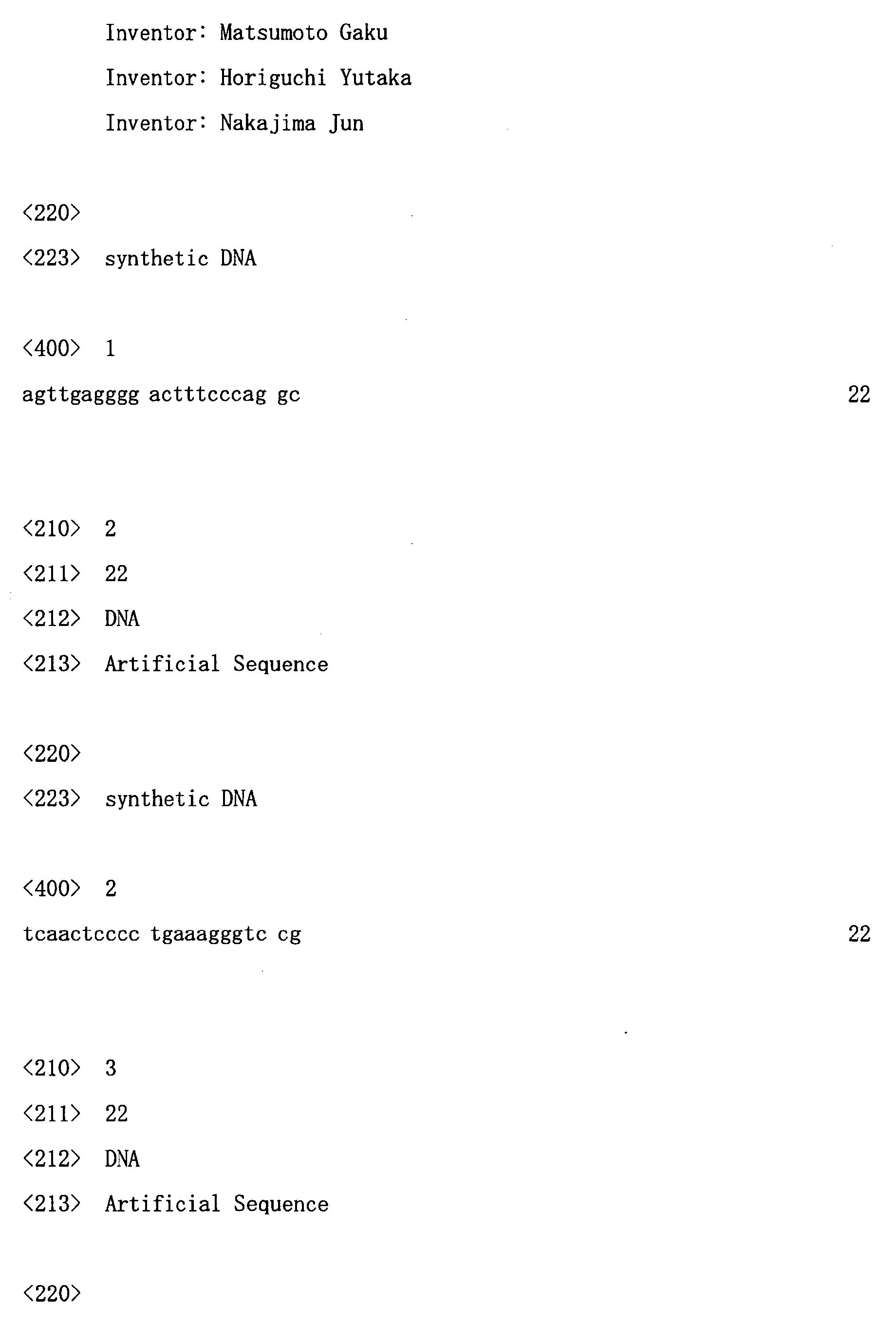

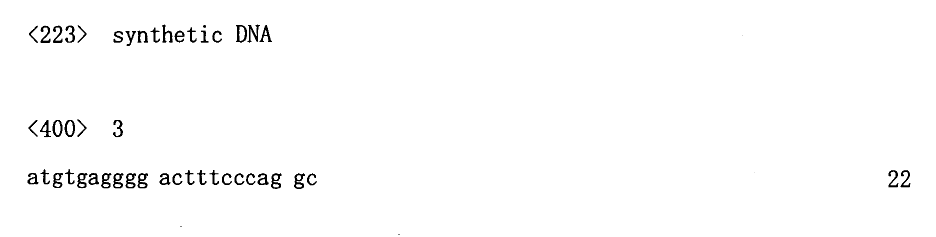

## Claims

1. A pharmaceutical composition for improving at least one symptom resulting from a tumor cell, comprising a compound represented by the following general formula (1) or a pharmacologically acceptable salt thereof as an active ingredient. 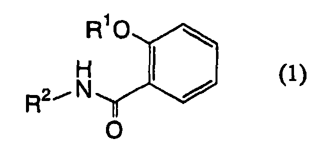 wherein R¹ represents a hydrogen atom or a C2-4 alkanoyl group and R² represents a group represented by the following formulae (A), (B), (C), (D), (E), (F) or (G): 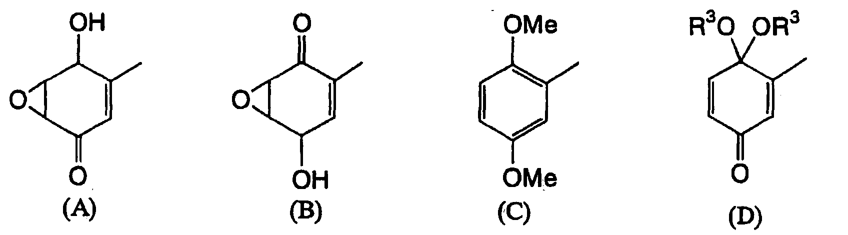 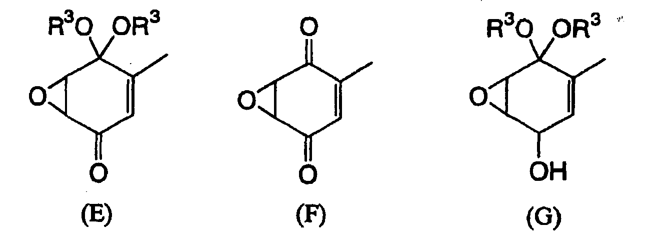 wherein R³ represents a C1-4 alkyl group.

2. The pharmaceutical composition of claim 1, comprising improving at least one symptom by apoptosis of the tumor cell.

3. The pharmaceutical composition of claim 1, comprising improving at least one symptom resulting from the tumor cell without the contribution of apoptosis of the tumor cell.

4. The pharmaceutical composition of claim 3, comprising improving at least one symptom resulting from the tumor cell by inhibiting activation of NF-κB.

5. The pharmaceutical composition of claim 3, wherein the symptom is a tumor metastasis.

6. The pharmaceutical composition of claim 5, comprising improving the tumor metastasis by inhibiting adhesion to a vascular endothelial cell.

7. The pharmaceutical composition of claim 1, comprising improving at least one symptom resulting from the tumor cell by inhibiting proliferation of the tumor cell.

8. The pharmaceutical composition of claim 1, wherein the symptom is one selected from the group consisting of Hodgkin's disease, cancer cachexia, and leukemia.

9. The pharmaceutical composition of claim 3, wherein the tumor cell is a breast cancer cell.

10. The pharmaceutical composition of claim 3, wherein the composition is the following formula (1a) or (1b).

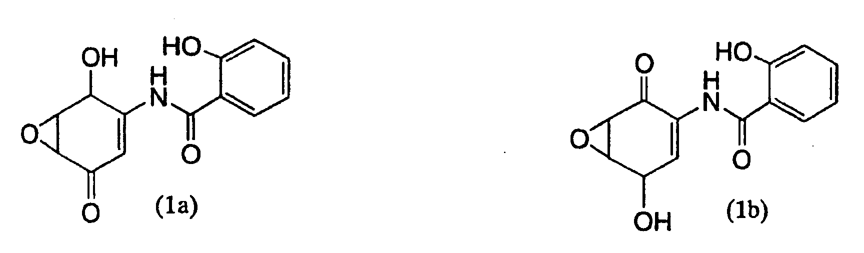

11. The pharmaceutical composition of claim 8, comprising improving at least one symptom among loss of body weight, a decrease in hematocrit, a decrease in fat, and a decrease in muscle, which are the symptoms of cancer cachexia.

12. The pharmaceutical composition of claim 3, comprising improving at least one symptom resulting from the tumor cell by inhibiting intratumoral angiogenesis formed by the tumor cell.

13. A pharmaceutical composition comprising as an active ingredient a compound, represented by the following general formula (1), which is capable of enhancing the effect of a therapy by inhibiting activation of NF-κB caused by the therapy that causes the activation of NF-κB, or a pharmacologically acceptable salt thereof. 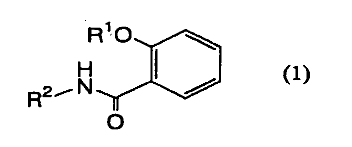 wherein R¹ represents a hydrogen atom or a C2-4 alkanoyl group and R² represents a group represented by the following formulae (A), (B), (C), (D), (E), (F) or (G): 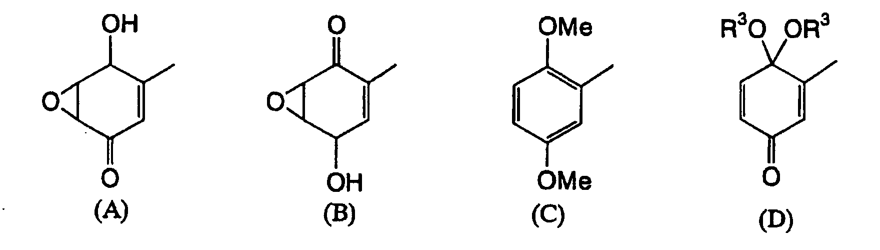 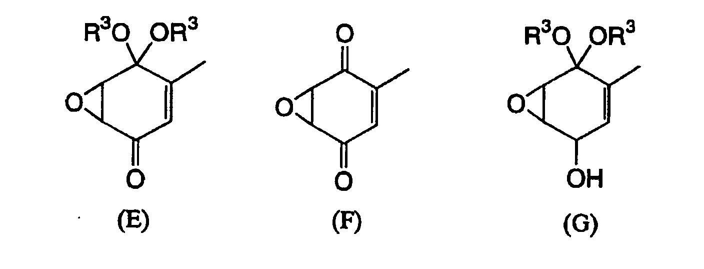 wherein R³ represents a C1-4 alkyl group.

14. The pharmaceutical composition of claim 13, wherein the therapy that activates NF-κB is a therapy using an antitumor agent.

15. The pharmaceutical composition of claim 13, wherein the therapy that activates NF-κB is radiotherapy for a tumor cell.

16. The pharmaceutical composition of claim 14, comprising the antitumor agent as an active ingredient.

17. The pharmaceutical composition of claim 14, wherein the antitumor agent is camptothecin or daunorubicin.

18. The pharmaceutical composition of claim 13, wherein the compound is the following formula (1a) or (1b).

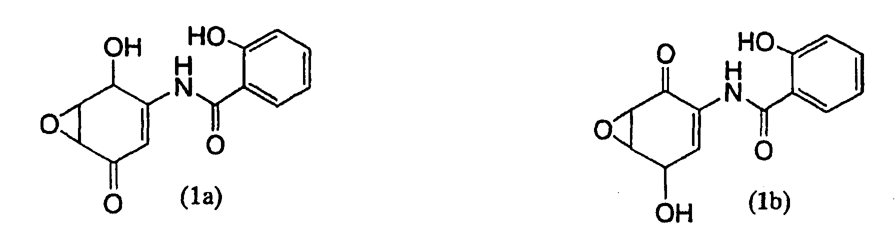

19. A tumor cell proliferation inhibitor for inhibiting proliferation of a tumor cell comprising a compound represented by the following general formula (1) or a pharmacologically acceptable salt thereof as an active ingredient. 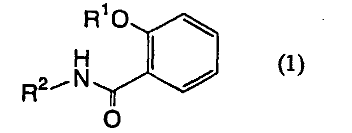 wherein R¹ represents a hydrogen atom or a C2-4 alkanoyl group and R² represents a group represented by the following formulae (A), (B), (C), (D), (E), (F) or (G): 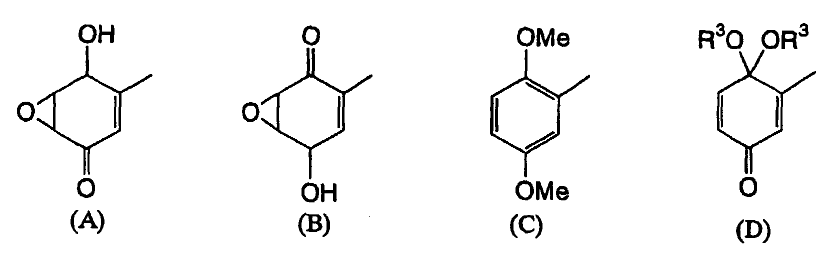 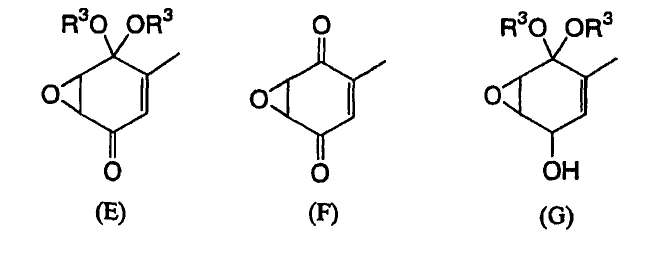 wherein R³ represents a C1-4 alkyl group.

20. The tumor cell proliferation inhibitor of claim 3, wherein the composition is the following formula (1a) or (1b).

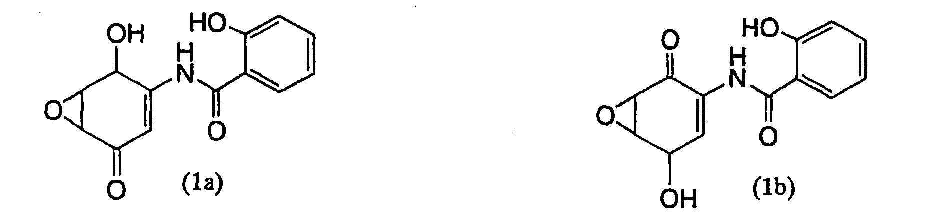

21. An adhesion molecule expression inhibitor for suppressing the expression of an adhesion molecule in a vascular endothelial cell, comprising a compound represented by the following general formula (1) or a pharmacologically acceptable salt thereof as an active ingredient. 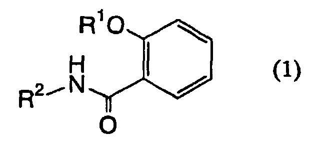 wherein R¹ represents a hydrogen atom or a C2-4 alkanoyl group and R² represents a group represented by the following formulae (A), (B), (C), (D), (E), (F) or (G): 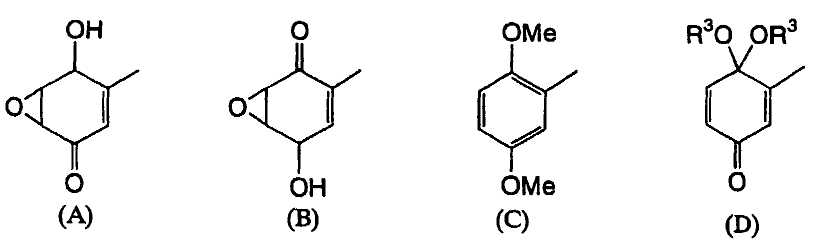 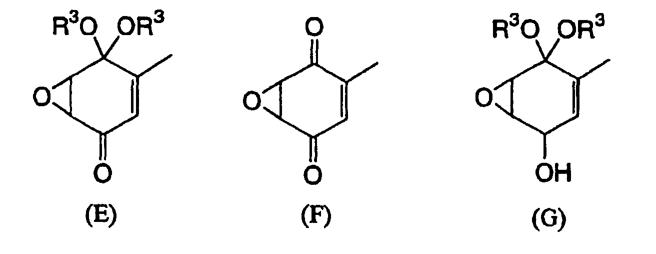 wherein R³ represents a C1-4 alkyl group.

22. The adhesion molecule expression inhibitor derived from a vascular endothelial cell, wherein the composition is the following formula (1a) or (1b).

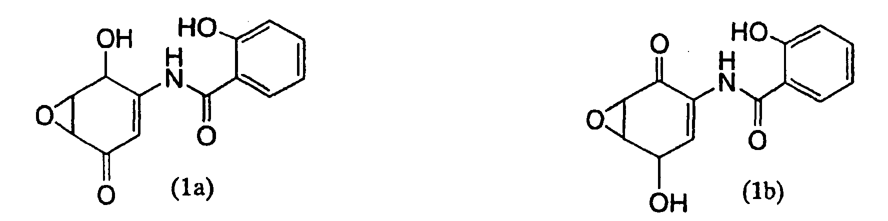

23. An apoptosis inducer for inducing apoptosis of a tumor cell, comprising a compound represented by the following general formula (1) or a pharmacologically acceptable salt thereof as an active ingredient. 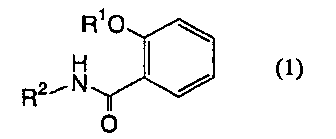 wherein R¹ represents a hydrogen atom or a C2-4 alkanoyl group and R² represents a group represented by the following formulae (A), (B), (C), (D), (E), (F) or (G): 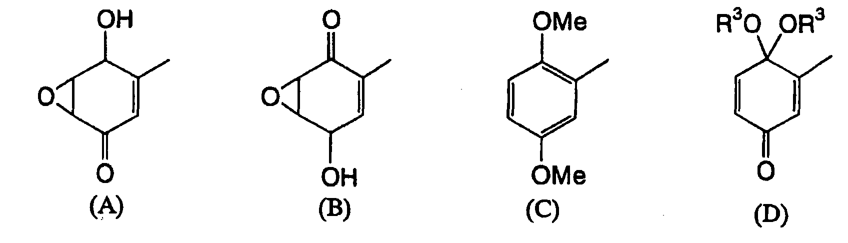 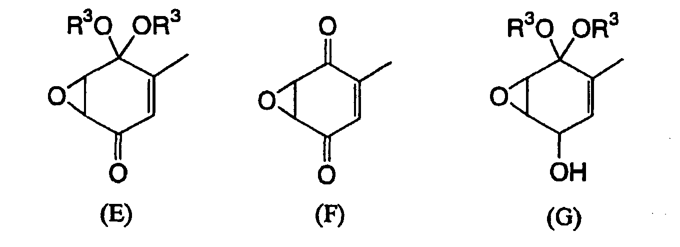 wherein R³ represents a C1-4 alkyl group.

24. An apoptosis inducer, wherein the composition is the following formula (1a) or (1b).

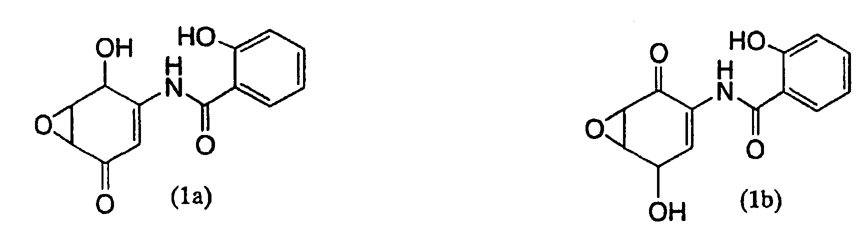

25. Preventive and therapeutic agents for arteriosclerosis, comprising a compound having NF-κB-inhibitory effect as an active ingredient.

26. The preventive and therapeutic agents of claim 25, wherein the compound having NF-κB inhibitory effect is represented by the following general formula (1) or a pharmacologically acceptable salt thereof. 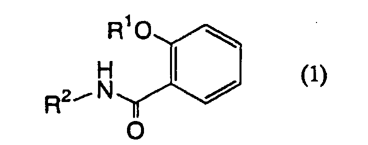 wherein R¹ represents a hydrogen atom or a C2-4 alkanoyl group and R² represents a group represented by the following formulae (A), (B), (C), (D), (E), (F) or (G): 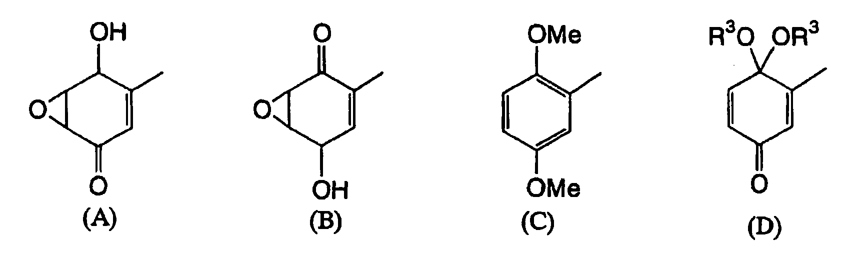 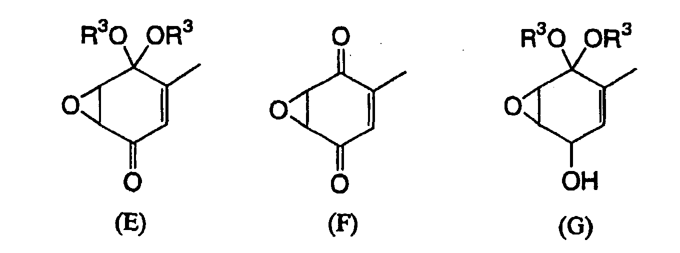 wherein R³ represents a C1-4 alkyl group.

27. Preventive and therapeutic agents for cancer, comprising a compound having NF-κB-inhibitory effect as an active ingredient.

28. The preventive and therapeutic agents of claim 27, wherein the compound having NF-κB-inhibitory effect is represented by the following general formula (1) or a pharmacologically acceptable salt thereof. 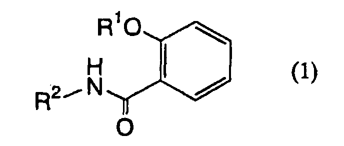 wherein R¹ represents a hydrogen atom or a C2-4 alkanoyl group and R2 represents a group represented by the following formulae (A), (B), (C), (D), (E), (F) or (G): 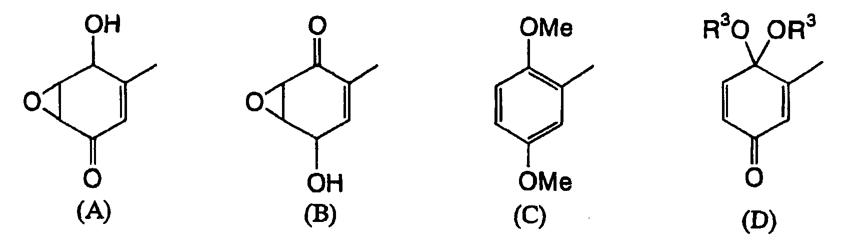 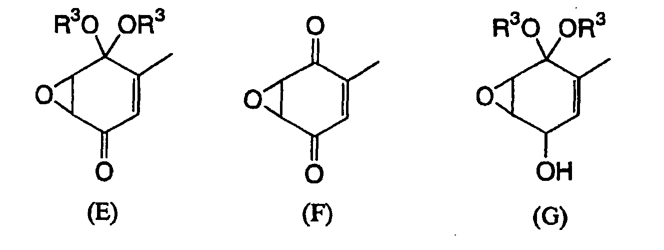 wherein R3 represents a C1-4 alkyl group.

29. The preventive and therapeutic agents of claim 27, which are used for repressing cancer metastasis.

30. A therapeutic agent for cachexia, comprising a compound represented by the following general formula (1) or a pharmacologically acceptable salt thereof as an active ingredient. 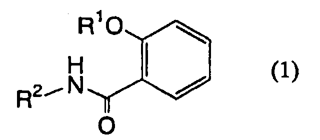 wherein R¹ represents a hydrogen atom or a C2-4 alkanoyl group and R² represents a group represented by the following formulae (A), (B), (C), (D), (E), (F) or (G): 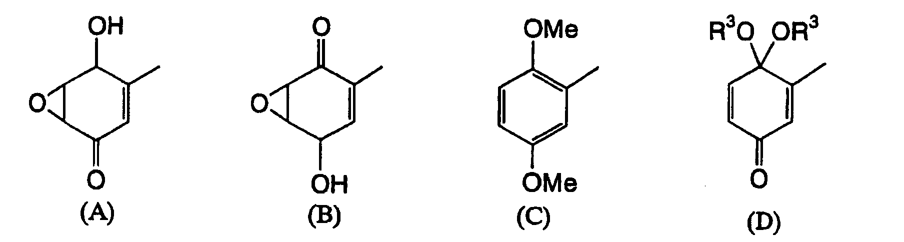 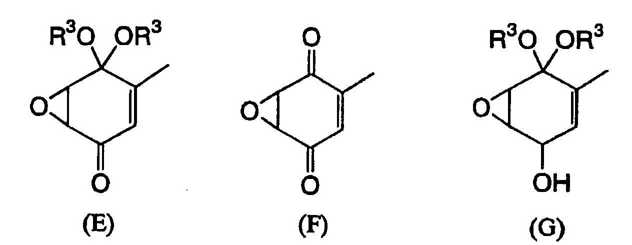 wherein R³ represents a C1-4 alkyl group.

31. The therapeutic agent for cachexia of claim 30, wherein the composition is the following formula (1a) or (1b).

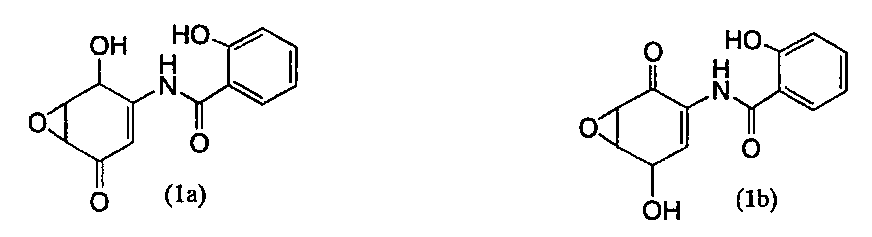

32. The therapeutic agent for cachexia of claim 30, which is a therapeutic agent for cancer cachexia in a tumor patient.

33. The pharmaceutical composition of claim 30, comprising improving at least one symptom among loss of body weight, a decrease in hematocrit, a decrease in fat, and a decrease in muscle, which are the symptoms of the cancer cachexia.

34. A therapeutic agent for cachexia, comprising a compound having NF-κB-inhibitory effect as an active ingredient.

35. A therapeutic method, wherein a compound for improving at least one symptom resulting from a tumor cell, represented by the following general formula (1) or a pharmacologically acceptable salt thereof is used. 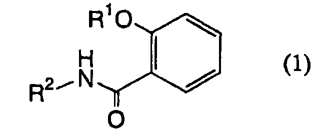 wherein R¹ represents a hydrogen atom or a C2-4 alkanoyl group and R² represents a group represented by the following formulae (A), (B), (C), (D), (E), (F) or (G) : 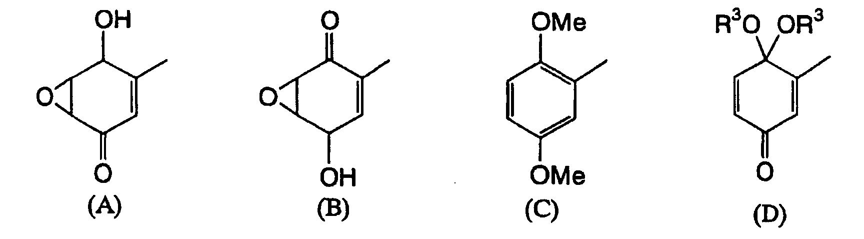 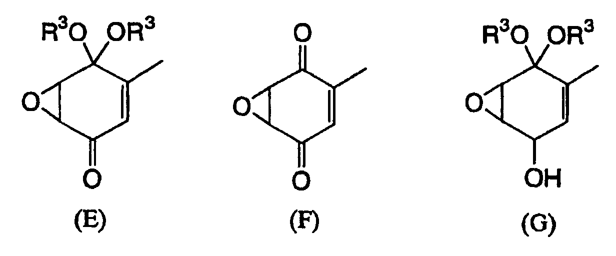 wherein R³ represents a C1-4 alkyl group.

36. The therapeutic method of claim 35, comprising improving at least one symptom by apoptosis of the tumor cell.

37. The therapeutic method of claim 35, comprising improving at least one symptom resulting from the tumor cell without the contribution of apoptosis of the tumor cell.

38. The therapeutic method of claim 35, comprising improving at least one symptom resulting from the tumor cell by inhibiting activation of NF-κB.

39. The therapeutic method of claim 35, wherein the symptom is one selected from the group consisting of tumor metastasis, a symptom resulting from the proliferation of the tumor cell, Hodgkin's disease, and cancer cachexia.

40. The therapeutic method of claim 35, wherein the composition is the following formula (1a) or (1b).

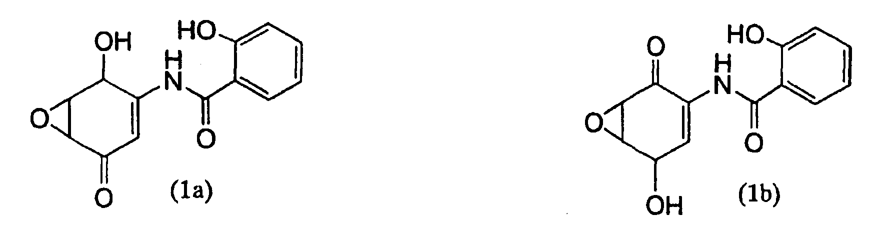

41. A therapeutic method, wherein a compound, for improving arteriosclerosis by inhibiting adhesion of a vascular endothelial cell to a leukocyte, represented by the following general formula (1) or a pharmacologically acceptable salt thereof is used. 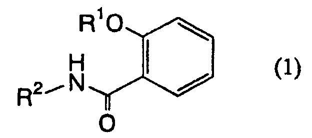 wherein R¹ represents a hydrogen atom or a C2-4 alkanoyl group and R² represents a group represented by the following formulae (A), (B), (C), (D), (E), (F) or (G): 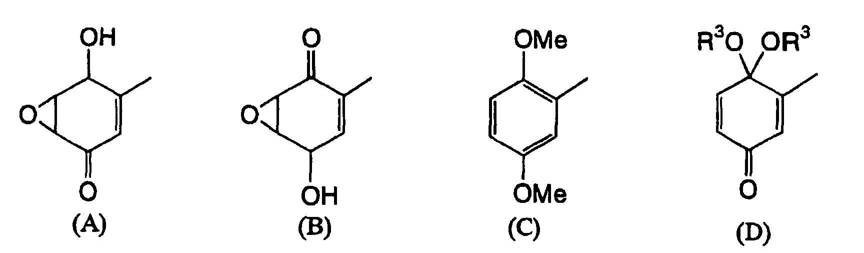 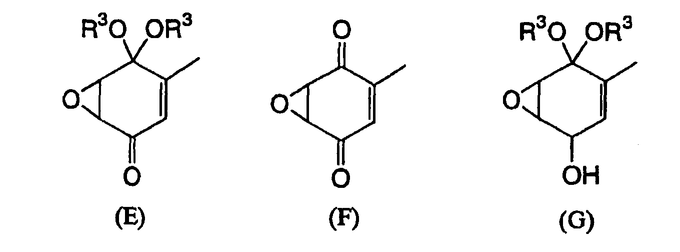 wherein R³ represents a C2-4 alkyl group.

42. The therapeutic method of claim 41, wherein the composition is the following formula (1a) or (1b).

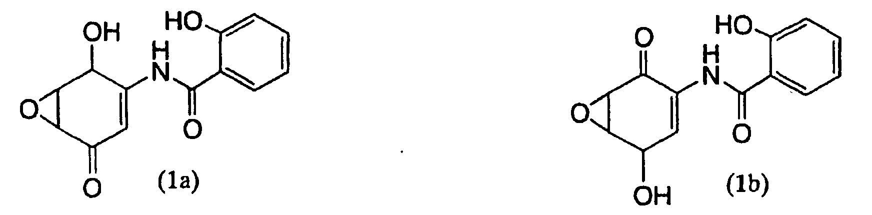

43. A therapeutic method, comprising the steps of performing a therapy for activating NF-κB and administering a pharmaceutical composition containing a compound represented by the following general formula (1) or a pharmacologically acceptable salt thereof as an active ingredient. 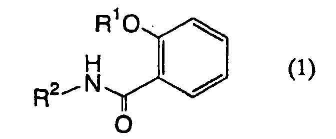 wherein R¹ represents a hydrogen atom or a C2-4 alkanoyl group and R² represents a group represented by the following formulae (A), (B), (C), (D), (E), (F) or (G): 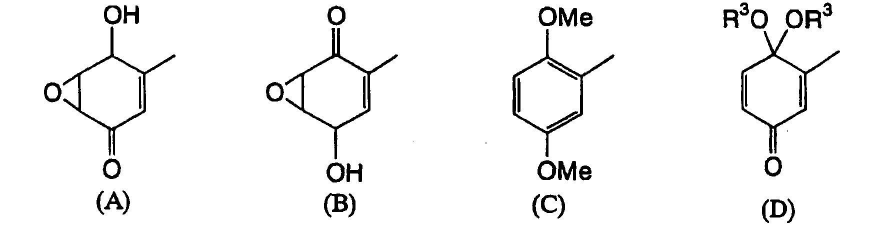 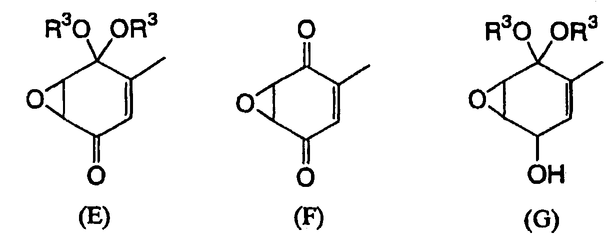 wherein R³ represents a C1-4 alkyl group.

44. The therapeutic method of claim 43, wherein the therapy that activates NF-κB is administration of an antitumor agent.

45. The therapeutic agent of claim 43, wherein the therapy that activates NF-κB is radiotherapy for a tumor cell.

46. The therapeutic method of claim 43, wherein the composition is the following formula (1a) or (1b).
